(19) Europäisches Patentamt / European Patent Office / Office européen des brevets

(11) EP 1 480 979 B1

(12) **FASCICULE DE BREVET EUROPEEN**

(45) Date de publication et mention de la délivrance du brevet:
**02.05.2007 Bulletin 2007/18**

(21) Numéro de dépôt: **03727569.0**

(22) Date de dépôt: **05.03.2003**

(51) Int Cl.:
*C07D 471/08* (2006.01) *A61K 49/06* (2006.01)
*C07D 257/02* (2006.01) *C07D 403/14* (2006.01)
*C07D 519/00* (2006.01) *C07F 9/6593* (2006.01)

(86) Numéro de dépôt international:
**PCT/FR2003/000712**

(87) Numéro de publication internationale:
**WO 2003/074523 (12.09.2003 Gazette 2003/37)**

(54) **OLIGOMERES DE CHELATES DE GADOLINIUM LEUR, APPLICATION COMME PRODUITS DE CONTRASTE EN IMAGERIE PAR RESONANCE MAGENTIQUE ET LEURS INTERMEDIAIRES DE SYNTHESE**

OLIGOMERE DER GADOLINIUM CHELATE, IHRE VERWENDUNG ALS NMR-KONTRASTMITTEL UND ZWISCHENVERBINDUNGEN

GADOLINIUM CHELATE OLIGOMERS, THEIR USE AS CONTRAST PRODUCTS IN MAGNETIC RESONANCE IMAGING AND THEIR SYNTHESIS INTERMEDIATES

(84) Etats contractants désignés:
**AT BE BG CH CY CZ DE DK EE ES FI FR GB GR HU IE IT LI LU MC NL PT RO SE SI SK TR**

(30) Priorité: **05.03.2002 FR 0202791**

(43) Date de publication de la demande:
**01.12.2004 Bulletin 2004/49**

(73) Titulaire: **GUERBET**
**93420 Villepinte (FR)**

(72) Inventeurs:
- **NACHMAN, Isabelle**
  **F-92400 COURBEVOIE (FR)**
- **PORT, Marc**
  **F-95170 DEUIL LA BARRE (FR)**
- **RAYNAL, Isabelle**
  **F-94100 SAINT-MAUR DES FOSSES (FR)**
- **ROUSSEAUX, Olivier**
  **F-60300 SENLIS (FR)**

(74) Mandataire: **Colombet, Alain André et al**
**Cabinet Lavoix,**
**2, Place d'Estienne d'Orves**
**75441 Paris Cedex 09 (FR)**

(56) Documents cités:
**EP-A- 0 922 700**
**WO-A-00/75141**
**WO-A-97/01359**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 480 979 B1

Printed by Jouve, 75001 PARIS (FR)

**Description**

La présente invention concerne des oligomères de chélates paramagnétiques, leur application comme produits de contraste à rémanence vasculaire pour l'imagerie par résonance magnétique (IRM) ainsi que leurs procédés de préparation et intermédiaires de synthèse.

L'administration de produits de contraste aux patients contribue à améliorer la résolution des images obtenues et la précision du diagnostic.

La relaxivité longitudinale $r_1$ d'un produit de contraste paramagnétique donne la mesure de son efficacité magnétique et permet d'apprécier son influence sur le signal enregistré ; l'efficacité massique, définie comme étant le rapport de $r_1$ à la masse moléculaire du composé donne, quant à elle, la mesure de l'efficacité de l'unité pondérale du produit de contraste et permet de comparer les produits en terme de poids de dose diagnostique administrée et notamment de tolérance et de coût.

Les chélates de gadolinium, utilisés en clinique humaine, tels que Magnevist®, Dotarem® ou Omniscan®, sont de faible masse moléculaire, ont des relaxivités $r_1$ inférieures à 5 $mM^{-1}s^{-1}$ et se répartissent rapidement dans l'espace extravasculaire après leur injection.

D'autres produits à plus forte relaxivité, désignés RR (à Rotation Restreinte) ont été décrits par la demanderesse dans les demandes de brevets WO 97/01359, EP-A-922700 et WO 00/75141. Ces produits sont des complexes du gadolinium paramagnétique avec des macrocycles azotés porteurs sur les atomes d'azote de groupes acétiques caractérisés par la présence sur l'atome de carbone en alpha du carboxyle de groupes hydrophiles.

Certains chélates de gadolinium, volumineux, sont des produits à rémanence vasculaire (Blood Pool Agents ou BPA) : la rémanence vasculaire limite l'élimination du produit de contraste par les reins et par le transfert vers les compartiments extravasculaires, Les BPA visent à améliorer ainsi la caractérisation de lésions (par exemple la vascularisation de lésions ou la détection d'hémorragies) et la perfusion d'organes, notamment la perfusion myocardique.

On connaît en particulier des produits désignés RC BPA (Rapid Clearance Blood Pool Agents). Le niveau de rémanence vasculaire de ces produits RC BPA les rend avantageux dans des applications préférées telles que la perfusion myocardique ou l'angiographie des coronaires où ils sont utilisables en plusieurs injections successivement. Parmi les RC PBA on connaît notamment les produits décrits dans le document EP 922 700, et des produits de type dendrimère : plusieurs chélates de gadolinium sont greffés à la périphérie d'une structure arborescente "pleine et dense" à fort encombrement formant une sorte de sphère pleine (Gadomer17® par exemple).

Ceci étant il reste souhaitable de trouver des produits encore plus efficaces sur le plan magnétique, dont la perméabilité vasculaire soit très faible, et dont l'élimination soit encore plus faible que celle des RCBPA déjà connus. Différentes tentatives ont visé à obtenir des composés désignés SC BPA (Slow Clearance Blood Pool Agent). Parmi les SC BPA on connaît notamment des produits comprenant un chélate de gadolinium greffé sur une molécule biologique de gros poids moléculaire, telle que l'albumine.

Ceci étant le besoin demeure d'autres produits permettant une optimisation de la dose de gadolinium, une amélioration de la qualité de l'image grâce à la distribution sélective dans le compartiment vasculaire et à un contraste amélioré entre les vaisseaux et les tissus environnants.

En particulier, il est souhaitable d'obtenir des produits d'une efficacité analogue, voire meilleure à celle des dendrimères ou des produits qui se lient à l'albumine, mais dont la synthèse chimique est moins complexe industriellement, et dont l'efficacité massique et la tolérance sont améliorées.

De nouveaux BPA, dont la rémanence vasculaire est plus forte que celle des RC BPA connus, visent des améliorations significatives notamment, dans la détermination du volume sanguin dans les différents tissus, la différenciation des tumeurs cancéreuses, l'identification des zones inflammatoires ou la lymphographie.

La demanderesse a maintenant trouvé que des oligomères polymétalliques, en étoile autour d'un coeur plurifonctionnel appelé noyau central (d'où une structure de type sphère creuse au lieu de sphère pleine), dérivés de ses produits RR précédemment décrits, présentaient des propriétés significativement améliorées de BPA, pour des doses pondérales acceptables pour le patient et un coût compatible avec l'économie de la santé. Les produits sont éliminés majoritairement par voie rénale lentement, mais dans un délai raisonnable pour éviter l'apparition d'effets secondaires dûs à une rétention prolongée dans le corps et cela même si les complexes sont stables.

Ces nouveaux oligomères comportent plusieurs chélates de gadolinium fonctionnalisés qui ont été greffés sur une molécule, le noyau central, portant des groupes susceptibles de réagir avec ces fonctions de couplage. Selon une réalisation avantageuse, les fonctions de couplage sont des fonctions amine. Selon un mode de réalisation ces oligomères sont obtenus à partir de monomères de chélates, ces polymétalliques portent 3 à 6 chélates de gadolinium.

Selon un autre mode de réalisation, ces oligomères sont obtenus à partir de dimères de chélates ; ces polymétalliques portent typiquement de 4 à 8 chélates de gadolinium.

Ce sont des produits représentant une solution technique à plusieurs problèmes techniques :

- La masse moléculaire est supérieure à 10000 daltons et monodisperse, pour limiter la diffusion extravasculaire non

souhaitée, ce qui permet une meilleure imagerie vasculaire et tissulaire (imagerie de caractérisation) ;

- La proportion pondérale d'ions de gadolinium (taux de Gd = nombre de Gd x 157,25 / masse moléculaire) est de l'ordre de 4 à 6%, ce qui permet de limiter la dose de gadolinium injectée (toxicité réduite) et d'obtenir une efficacité massique améliorée (efficacité massique par Gd = r1 par Gd x 1000 / 157,25 (masse moléculaire du Gd) ), supérieure à 100 $g^{-1}Gd\ s^{-1}$;

On rappelle que l'efficacité massique molaire (em) en $g^{-1}s^{-1}$ est le rapport : (nombre de Gd x r1 x 1000) / masse moléculaire de la molécule).

En particulier, la structure en étoile peu dense décrite plus loin permet, pour un même encombrement moléculaire recherché (permettant de réduire la diffusion extravasculaire), de limiter significativement le poids moléculaire du composé (et donc son coût et sa difficulté de synthèse) par rapport à une structure de type dendrimères (proportion pondérale d'ions de gadolinium de l'ordre de 18%, voire nettement davantage).

- Le nombre de chélates greffés sur le coeur choisi est parfaitement contrôlé, contrairement au cas des dérivés connus qui résultent du greffage de polymères. Leur relaxivité $r_1$ par Gd est équivalente ou supérieure à celle des monomères dont ils dérivent et le signal obtenu est satisfaisant dans la gamme de champ magnétique appliqué par les appareils actuels d'imagerie médicale, c'est-à-dire typiquement de 20, voire 40 MHz ou 60 MHz.

Par rapport à des BPA de l'art antérieur, les nouveaux produits obtenus ont des relaxivités par Gd supérieures, une efficacité massique par Gd supérieure (l'efficacité massique de l'oligomère polymétallique est supérieure à celle du monomère monométallique correspondant), une monodispersité et une pureté contrôlées.

Les SC BPA connus de l'art antérieur suggéraient :

- un greffage sur de grosses molécules biologiques telles que des protéines, ou sur des structures chimiques porteuses pleines, au détriment d'une efficacité massique performante
- des structures de type polymères mais beaucoup trop polydisperses pour une utilisation fiable, avec une pureté et une homogénéité insuffisantes des produits.

Pour obtenir ces nouveaux oligomères, la structure des chélates RR antérieurement décrits a dû être modifiée pour introduire un groupe susceptible de réagir sur la molécule précurseur du noyau central, sans que cette modification n'entraîne une perte de stabilité ou d'efficacité magnétique.

Selon un premier aspect, l'invention concerne des molécules polymétalliques, de formule générale suivante :

$$\text{NOYAU CENTRAL} - [\ (\text{lien 2} - \text{Div})_o - (\ \text{lien 1} - \text{cœur Gd} - \ (\text{branche})_n\ )_s\ ]_m$$

Dans laquelle :

n est compris entre 1 et 3
s est compris entre 1 et 3, de préférence s=1 ou 2
o est 0 ou 1
m est compris entre 2 et 6 quand o=0, et m est compris entre 1 et 4 quand o=1.

Les coeurs Gd portant des branches hydrophiles sont des chélates de gadolinium de type RR, c'est à dire à Rotation Restreinte, notion décrite dans le brevet délivré EP B 661 279 et EP B 922 700 et rappelée plus loin. En effet, les inventeurs ont effectué des essais avec des dérivés non RR mais les résultats n'ont pas été satisfaisants. Les inventeurs ont ainsi réussi à construire des produits efficaces en combinant :

- l'utilisation de dérivés Gd à rotation restreinte, tels que décrits dans les brevets EP B 661 279, EP B 922 700, EP B 1 183 255, pour obtenir une relaxivité satisfaisante (pour un signal suffisant) ;
- la construction optimisée d'une architecture de type polymétallique permettant une multiplication du signal par Gd tout en ayant notamment un volume moléculaire, une efficacité massique, et une hydrophilie suffisants pour une utilisation performante comme BPA, avec une molécule finale possédant une relaxivité élevée pour un nombre de Gd limité.

Ces nouveaux composés polymétalliques diffèrent clairement des composés de type dendrimère (o > 1) de l'art antérieur, dont les couples (Div - lien 2) peuvent être différents à chaque génération.

Avantageusement, les coeurs Gd ont été choisis parmi :

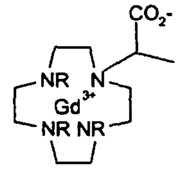
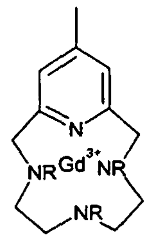
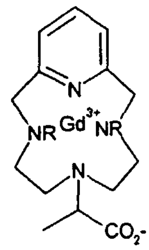

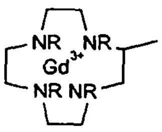
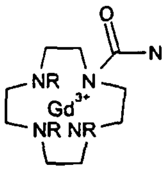
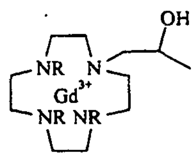
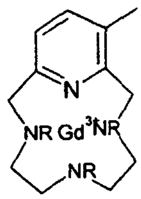
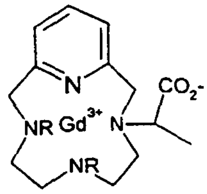

R = 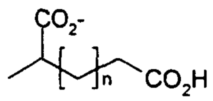 n = 0,1,2

Les inventeurs ont également étudié des composés utilisant des coeurs Gd à rotation restreinte de type DTPA décrits notamment dans le document EP 661 279, de formule générale :

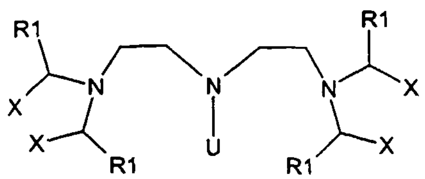
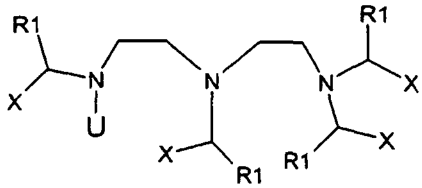

avec R1,U tels que décrits plus loin.

Avantageusement les branches ont été choisies parmi :

- les branches désignées AAG1 AA28Br, AAG1 AA29Br décrites plus loin ;
- les branches décrites dans les documents EP 661 279, EP 922 700, EP 1 183 255 ;
- les branches dites flash décrites plus loin ;
- les branches dites à linker rigide CO - NH - ϕ - CO - NH, dites P792, mentionnées plus loin.

De telles branches hydrophiles formant des bras latéraux sur les groupes acides peuvent être de nature différente et sont destinées à diminuer la liberté de mouvement du complexe paramagnétique et de l'ion paramagnétique qui lui y est attaché, dont la rotation dans le champ magnétique (fonction inverse de r1 ) est ainsi restreinte.

De plus les inventeurs ont dû, de manière non évidente, adapter la construction [ (coeur Gd - lien 1 )s - (Div - lien $2)_o$], sans que cette construction n'entraîne une perte de stabilité thermodynamique ou cinétique, ou d'efficacité magnétique.

Dans le cas où un diviseur est utilisé, différents diviseurs sont possibles, tant qu'ils permettent de faire le lien (en préservant la structure en étoile) entre d'une part au moins deux chélates, et d'autre part un noyau central polyfonctionnel. Différents squelettes polyfonctionnels peuvent être utilisés par l'homme du métier comme diviseur, décrits dans Chemical Reviews, 2001, 101 (12), 3819-386 et Topics in Current Chemistry, vol 217,212,210,197. On préfère comme

diviseur Div des squelettes aromatiques polyfonctionnalisés par des groupements carboxylates et/ou aminés.

La demanderesse a préféré utiliser des diviseurs Div de type 1,3,5 triazine :

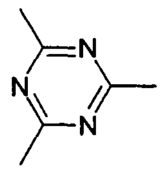

Le lien 1 et le lien 2 sont de préférence choisis parmi :

a) $(CH_2)_2$- ϕ- $NH_2$, $(CH_2)_3$ - $NH_2$, NH-$(CH_2)_2$-NH, NH-$(CH_2)_3$-NH, $NH_2$-$(CH_2)_2$-O$(CH_2)_2$-O$(CH_2)_2$-$NH_2$,
b) P1-I-P2, identiques ou différents, P1 et P2 étant choisis parmi OH, SH, $NH_2$, H, $CO_2H$, NCS, NCO, $SO_3H$,
Avec I = Alkylène (de préférence C1 à C8), cycloalkylène, alkoxyalkylène, polyalkoxyalkylène, alkylène interrompu par phénylène, alkylidène, acylidène.
c) P1-I-P2, avec P1 étant tel qu'en a) ou b) et P2 étant choisi parmi hydroxamate, cathécolate, phénantroline, guanine, quand le noyau est un métal M tel que décrit dans le brevet US 6 056 939 et Topics in current chemistry 2002, 221, 123-164.

Le NOYAU central a été choisi parmi deux types de groupements (le composé entre parenthèses est un exemple de composé correspondant exemplifié dans la description détaillée) :

a) mélamine (P799), cyanurile monochloré (MC606 et MC617), téréphtalique dithiourée (MC607), phényltriisothiourée (MC616), P730 Gd c'est à dire DOTA Gd avec présence sur l'atome de carbone en alpha du carboxyle de groupes hydrophiles (MC635), tétrakis (MC636 et MC645), hexakis phosphazène (MC647).
b) coeur polyacide éventuellement halogène, coeur gadoliné polyacide ou polyamine, coeur isothiocyanate, isocyanate, coeur polyamine, coeur polysulfate, polymère polycarboxylique ou polyaminé.
c) coeur (M), choisi parmi les lanthanides tels que le gadolinium Gd, et les métaux de transition comme Fe, Co, Zn, Ni, Ca2+ lorsque P2 est choisi parmi hydroxamate, cathécolate, phénantroline, guanine, tel que décrit dans le brevet US 6 056 939 et Topics in current chemistry 2002, 221, 123-164..

Parmi l'ensemble des composés ci-dessus, les composés en étoile répondant à la formule générale suivante (E) ont été préférés :

$$W - (A)_m \qquad (E)$$

dans laquelle :

- W est un NOYAU central
- A représente [ ( D )$_q$ - ( $I_{a,b,c,d,e,f}$)$_r$ ];
  (E) s'écrivant: W-[(D)$_q$-($I_{a,b,c,d,e,f}$)$_r$]$_m$ Avec :
  - q = 0 (E est un composé polymétallique de monomères tel que décrit plus loin) ou q=1 (E est un composé polymétallique de dimères tel que décrit plus loin)
  - r=1 lorsque q=0, ou r est 2 ou 3 lorsque q=1
  - m est compris entre 3 et 6 lorsque q=0 et m est compris entre 2 et 4 lorsque q=1
  - D étant une molécule polyfonctionnelle capable de relier le NOYAU central à

au moins deux chélates métalliques,
D s'écrivant (Div - lien 2), Div étant un groupement ayant un nombre de valences libres au moins égal à r, Div étant aussi désigné comme diviseur,
D étant lié d'une part à au moins deux chélates métalliques par l'intermédiaires de liens 1 (deux liens 1 pour les dimères), et d'autre part au NOYAU central par l'intermédiaire d'un lien 2, le lien 1 et le lien 2 étant tels que définis ci-dessus ; de la manière suivante pour r=2

$$(\text{lien } 1)_2 - \text{Div} - (\text{lien } 2)\ ;$$

(E) s'écrivant :

$$\text{NOYAU} \ - \ [\ (\text{Lien } 2 - \text{Div}\ )_q - (\ I_{a,b,c,d,e,f})_r\ ]_m \qquad (E)$$

Lien 1 étant inclus, comme décrit plus loin, dans $I_{a,b,c,d,e,f}$

D étant de préférence un squelette aromatique polyfonctionnalisé par des groupements carboxylates et/ou aminés, de préférence Div étant de type 1,3,5 triazine, de formule :

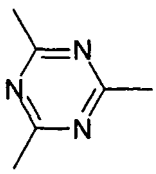

avec $(\text{lien } 1)_2$ - Div - (lien 2) s'écrivant :

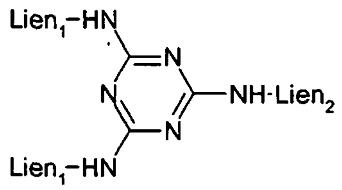

- $I_{a,b,c,d,e,f}$ signifie $I_a$ ou $I_b$ ou $I_c$ ou $I_d$ ou $I_e$ ou $I_f$ , représentant les dérivés à Rotation restreinte $I_a$ , $I_b$ , $I_c$ ayant les significations :

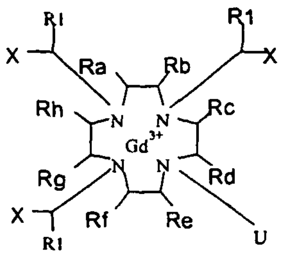

Ia

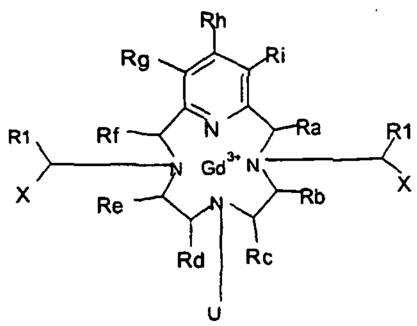

Ib

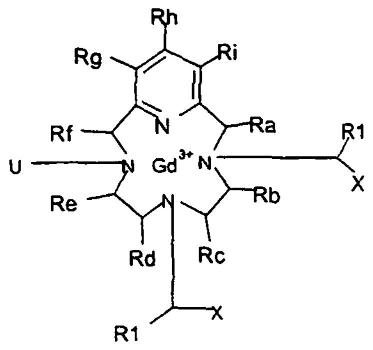

Ic

où :

- les X, identiques ou différents, sont choisis parmi $CO_2R'a$, CONR'bR'c ou $P(R'd)O_2H$, avec :

  R'a, R'b, R'c identiques ou différents représentant H ou (C1-C8) alkyle, éventuellement hydroxylé ;
  P est l'atome de phosphore, R'd représente OH, (C1-C8) alkyle ou (C1-C8) alkoxy, (C1-C8) arylalkyle ou (C1-C8) alkoxyalkyle ;

- R1 représente un groupe hydrophile, de taille appropriée pour obtenir à la fois un niveau de relaxivité suffisant et une efficacité massique performante, typiquement de masse moléculaire supérieure à 300 et inférieure à

3000, comprenant au moins trois atomes d'oxygène, sélectionné parmi des groupes :

- polyoxy (C2-C3) alkylène (i.e polyoxyéthylènes et polyoxypropylènes), notamment le polyéthylène glycol et ses monoéthers et monoesters en C1 à C3, de masse moléculaire de préférence de 1000 à 2000
- Polyhydroxyalkyle
- Polyol (dont oligosaccharides fonctionnalisés [ce type de fonctionnalisation étant décrit notamment dans J. Polymer. Sc. Part A Polymer chemistry 23 1395-1405 (1985) et 29, 1271-1279 (1991) et dans Bioconjugate chem. 3, 154-159 (1992)])
- $(R_2g)_e\ [(R_2g)_iR_3]_h$ où:

    - h = 1 ou 2 ; i = 0, 1 ou 2; e = 1 à 5
    - $R_2$ représente (les $R_2$ étant identiques ou différents) :

        - rien, un alkylène, un alkoxyalkylène, un polyalkoxyalkylène ;
        - un phénylène, ou un reste hétérocyclique saturé ou non, éventuellement substitué par OH, Cl, Br, I, (C1-C8)alkyle, (C1-C8)alkyloxy, NO2, $NR_XR_Y$, $NR_XCOR_Y$, $CONR_XR_Y$, $COOR_X$, $R_X$ et $R_Y$ étant H ou (C1-C8)alkyle, et les groupes alkyle, alkylène, alkoxy, en C1 à C14 linéaires, ramifiés ou cycliques pouvant être hydroxylés ;

- g représente (les g étant identiques on différents) : rien ou une fonction O, CO, OCO, COO, SO3, OSO2, CONR', NR'CO, NR'COO, OCONR',NR', NR'CS, CSNR', SO2NR', NR'SO2, NR'CSO, OCSNR',NR'CSNR', P(O)(OH)NR', NR'P(O)-(OH), dans laquelle R' est H (C1-C8)alkyle ou $R_3$;
- $R_3$ représente alkyle, phényle, alkyle substitué ou interrompu par un ou des groupes phényles, alkylèneoxy; amino ou amido substitués ou non par alkyle éventuellement substitué ou interrompu par l'un des groupes précédents ; les groupes phényles, phénylènes et hétérocycliques pouvant être substitués par OH, Cl, Br, I, (C1-C8)alkyle, (C1-C8)alkyloxy, NO2, $NR_XR_Y$, $NR_XCOR_Y$, $CONR_XR_Y$, $COOR_X$, $R_X$ et $R_Y$ étant H ou (C1-C8)alkyle, et les groupes alkyle, alkylène, alkoxy, en C1 à C14 linéaires, ramifiés ou cycliques pouvant être hydroxylés ;

- $R_a$ à $R_i$ (c'est à dire Ra, Rb, Rc, Rd, Re, Rf, Rg, Rh, Ri) représentent indépendamment H, alkyle, hydroxyalkyle, alkylphényle, cycloalkyle.
- U est un groupe -CX R4-lien 1, CHR4CON-lien1, CHR4-CHR5OH-lien1
- R4, R5 représentant indépendamment H, alkyle, hydroxyalkyle.
- X et lien 1 ayant la signification ci-dessus.
- $I_d$, $I_e$, $I_f$ ayant les significations :

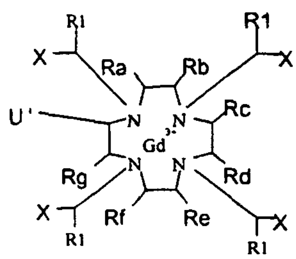

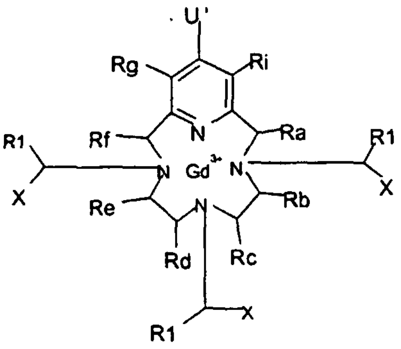

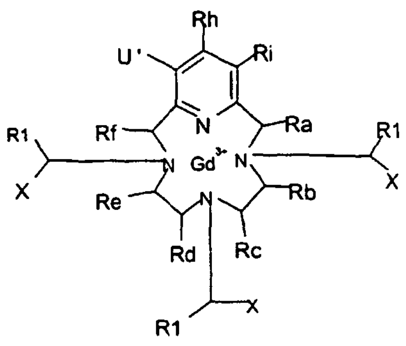

Id

If

$I_e$

- X, R1, Ra à Ri ayant la même signification que ci-dessus.

- U' est lien 1.

Le noyau central est choisi parmi :

a) mélamine (P799), cyanurile monochloré (MC606 et MC617), 1-4 phénylène dithiourée (MC607), phényltriisothiourée (MC616), P730 Gd c'est à dire DOTA Gd avec présence sur l'atome de carbone en alpha du carboxyle de groupes hydrophiles (MC635), tétrakis phénylméthane (MC636 et MC645), hexakis phosphazène (MC647).
b) coeur polyacide éventuellement halogène, coeur gadoliné polyacide ou polyamine, coeur isothiocyanate, isocyanate, coeur polyamine, coeur polysulfate, polymère polycarboxylique ou polyaminé.
c) coeur (M), choisi parmi les lanthanides tels que le gadolinium Gd, et les métaux de transition comme Fe, Co, Zn, Ni, Ca2+ lorsque P2 est choisi parmi hydroxamate, cathécolate, phénantroline, guanine, tel que décrit dans le brevet US 6 056 939 et Topics in current chemistry 2002, 221, 123-164.

L'invention concerne aussi les sels des composés de formule (E) avec des acides ou des bases minérales ou organiques, notamment les chlorhydrates des groupes amino et les sels de sodium, de potassium et de N-méthylglucamine des groupes acides carboxyliques présents sur les chélates.

Par ailleurs, la demanderesse fait les commentaires suivants :

- Pour U=CX$R_4$-CHR5OH-lien 1 ou U=CHR4CON-lien1 , on facilite la synthèse chimique ; toutefois les résultats de relaxivité sont en général moins bons avec ces derniers.
- De manière préférée, X représente CO2$R'_a$; toutefois l'utilisation de CONR'b R'c permet d'obtenir des composés non ioniques intéressants pour diminuer l'osmolalité du produit, et l'utilisation de $P(R'_d)O_2H$ peut permettre d'obtenir des produits à plus forte relaxivité.
- De manière préférée, Ra, Rb, Rc représentent H, mais on peut également utiliser des groupes alkyles ou cycloaklyles pour stabiliser la structure et améliorer la relaxivité, à condition de ne pas interférer avec les propriétés recherchées du produit (la rigidification par greffage de groupes alkyles est connue de l'homme du métier dans Inorganic Chemistry, vol 41, n°25,p6846-6855, 2002). Les groupes hydroxyalkyle sont connus de l'homme du métier pour diminuer la toxicité des structures, comme décrit dans Inorganic Chemical Acta 317, 2001, 218-229, et Coordination Chemistry Reviews, 185-186, 1999, 451-470.

On préfère les composés (E) où R1 = (CH2)xCONHR avec x=1, 2 ou 3 et R est un groupe hydrophile de poids moléculaire supérieur à 200, choisi parmi :

1 ) un groupement :

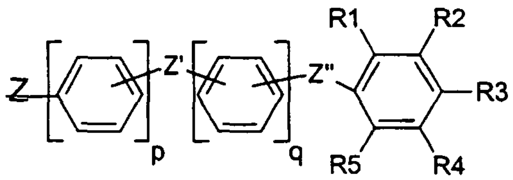

IV1

et Z est une liaison, $CH_2$, $CH_2CONH$ ou $(CH_2)_2NHCO$
Z' est une liaison, O, S, NQ, $CH_2$, CO, CONQ, NQCO, NQ-CONQ ou $CONQCH_2CONQ$,
Z" est une liaison, CONQ, NQCO ou $CONQCH_2CONQ$
p et q sont des nombres entiers dont la somme vaut 0 à 3 ;
$R_1$, $R_2$, $R_3$, $R_4$ ou $R_5$ représentent :

- soit indépendamment l'un de l'autre H, Br, Cl, I, $CONQ_1Q_2$ ou $NQ_1COQ_2$ avec $Q_1$ et $Q_2$ identiques ou différents sont H ou un groupe ($C_1$-$C_8$)alkyle mono- ou polyhydroxylé ou éventuellement interrompu par un ou des atomes d'oxygène, et au moins l'un et au plus deux des $R_1$ à $R_5$ sont $CONQ_1Q_2$ ou $NQ_1COQ_2$;
- soit $R_2$ et $R_4$ représentent

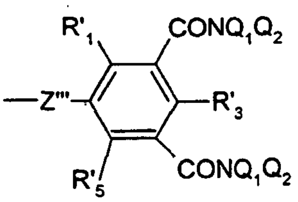

et $R_1$, $R'_1$, $R_3$, $R'_3$, $R_5$ et $R'_5$, identiques ou différents, représentent H, Br, Cl ou I, $Q_1$ et $Q_2$ ont la même signification que précédemment et Z''' est un groupe choisi parmi CONQ, $CONQCH_2CONQ$, $CONQCH_2$, NQ-CONQ, $CONQ(CH_2)_2NQCO$ et Q est H ou $(C_1\text{-}C_4)$alkyle, éventuellement hydroxylé, les groupes alkyle pouvant être linéaires ou ramifiés ;

2) une branche dite "flash

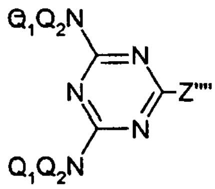

avec Z '''' étant $NQ(CH_2)_j(CH_2OCH_2)_i(CH_2)_jNH_2$, avec i = 2 à 6 et j = 1 à 6

Parmi l'ensemble des composés (E) ci-dessus, deux types de composés sont particulièrement avantageux.

Le premier type est représenté par les composés appelés " Polymétalliques de Monomères de dérivés à Rotation Restreinte », en abrégé Poly M RR . Ces oligomères en étoile de chélates de forte relaxivité ont pour formule:

$W1\text{-}(A1)_{m1}$ (I1) Poly M RR

dans laquelle :

- m1 est 3, 4, 5 ou 6 ;
- W1 est le radical d'une molécule organique portant m1 groupes carbonyle qui forment des groupes carboxamido avec A1 ;
  ou W1 est un groupe:

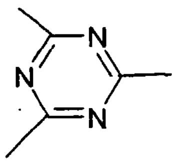

- A1 représente le groupe :

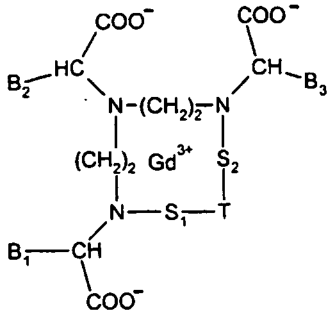

II1

dans lequel :

- $S_1$-T-$S_2$- est
  - soit

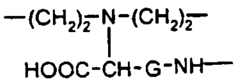

où $S_1 = S_2 = (CH_2)_2$
avec $B_1$, $B_2$, $B_3$ représentant tous les trois $(CH_2)_xCONHR$ avec x = 1, 2 ou 3
  - soit

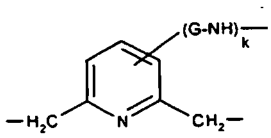

III1

avec k = 0 et $S_1 = S_2 = CH_2$
l'un des B1,B2,B3 représentant G-NH, et les autres représentant $(CH_2)_xCONHR$
  - soit

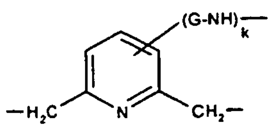

III1

avec k=1
$B_1$, $B_2$, $B_3$ représentant tous les trois $(CH_2)_xCONHR$ avec x = 1, 2 ou 3 et GNH choisi parmi :
les groupes $-(CH_2)_n$-NH- avec n = 1 à 4,
ou

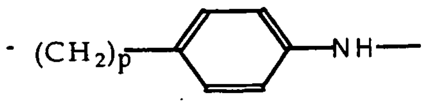 avec p = 0 à 3 ;

étant précisé que :

- R représente un groupe hydrophile de poids moléculaire supérieur à 200, R représentant selon une variante un groupement :

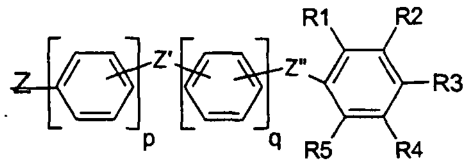

IV1

et Z est une liaison, $CH_2$, $CH_2CONH$ ou $(CH_2)_2NHCO$
Z' est une liaison, O, S, NQ, $CH_2$, CO, CONQ, NQCO, NQ-CONQ ou $CONQCH_2CONQ$,
Z" est une liaison, CONQ, NQCO ou $CONQCH_2CONQ$
p et q sont des nombres entiers dont la somme vaut 0 à 3 ;
$R_1$, $R_2$, $R_3$, $R_4$ ou $R_5$ représentent :

- soit indépendamment l'un de l'autre H, Br, Cl, I, $CONQ_1Q_2$ ou $NQ_1COQ_2$ avec $Q_1$ et $Q_2$ identiques ou différents sont H ou un groupe $(C_1\text{-}C_8)$alkyle mono- ou polyhydroxylé ou éventuellement interrompu par un ou des atomes d'oxygène, et au moins l'un et au plus deux des $R_1$ à $R_5$ sont $CONQ_1Q_2$ ou $NQ_1COQ_2$ ;
- soit $R_2$ et $R_4$ représentent

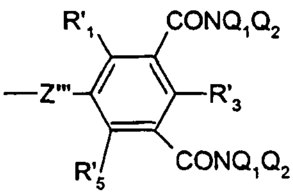

et $R_1$, $R'_1$, $R_3$, $R'_3$, $R_5$ et $R'_5$, identiques ou différents, représentent H, Br, Cl ou I, $Q_1$ et $Q_2$ ont la même signification que précédemment et Z"' est un groupe choisi parmi CONQ, $CONQCH_2CONQ$, $CONQCH_2$, NQ-CONQ, $CONQ(CH_2)_2NQCO$ et Q est H ou $(C_1\text{-}C_4)$alkyle, éventuellement hydroxylé, les groupes alkyle pouvant être linéaires ou ramifiés ;

- R représentant selon une autre variante (branches dites "flash) :

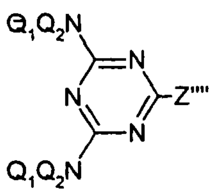

avec Z"" étant $NQ(CH_2)_j(CH_2OCH_2)_i(CH_2)_jNH_2$, avec i = 2 à 6 et j = 1 à 6, de préférence

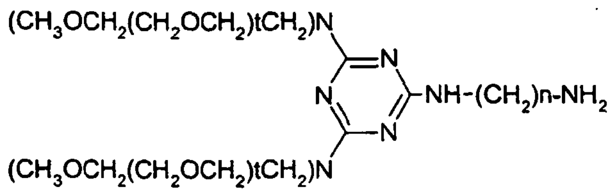

ou

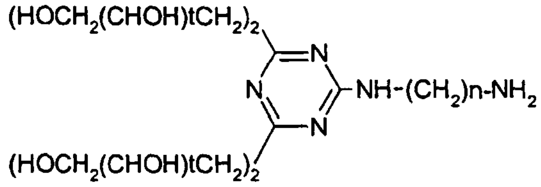

avec t =1, 2, 3 ou 4 et n=2 à 6.

L'invention concerne aussi les sels des composés de formule 11 avec des acides ou des bases minérales ou organiques, notamment les chlorhydrates des groupes amino et les sels de sodium, de potassium et de N-méthylglucamine des groupes acides carboxyliques présents sur les chélates.

Le deuxième type est représenté par les composés appelés "Polymétalliques de Dimères de dérivés à rotation restreinte », en abrégé Poly D RR, de formule générale :

W2 - $(A2)_{m2}$ (I2) Poly D RR

dans laquelle :

- m2 est 2, 3 ou 4 ;
- W2 est le radical d'une molécule organique portant m2 groupes carbonyle qui forment des groupes carboxamido avec A2 ;
  ou W2 est un groupe :

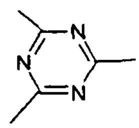

- A2 représente 1) le groupe :

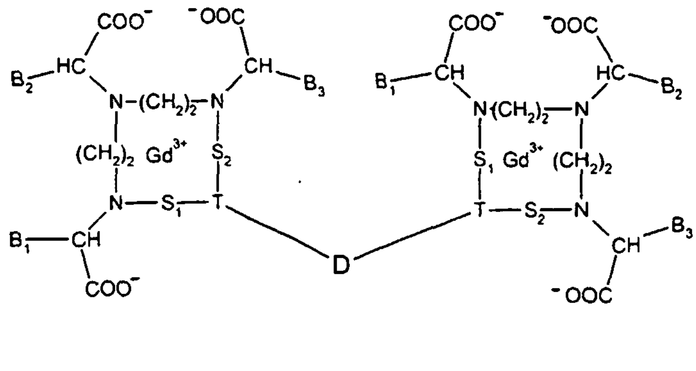

II2

dans lequel
$-S_1-T-S_2-$ est

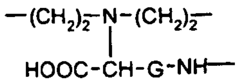

où $S_1 = S_2 = (CH_2)_2$
$B_1$, $B_2$, $B_3$ représentant tous les trois $(CH_2)_xCONHR$ avec x = 1, 2 ou 3

- ou A2 représente 2)

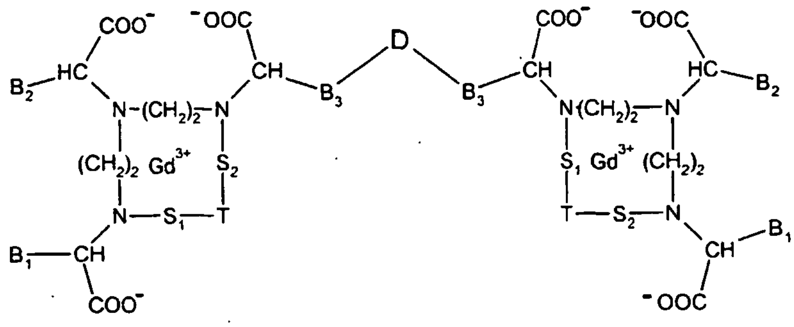

IIa2 (composé dit PCTA N fonctionnalisé)
Ou IIb2 (composé dit PCTA N fonctionnalisé et isomère de position du IIb2) de formule

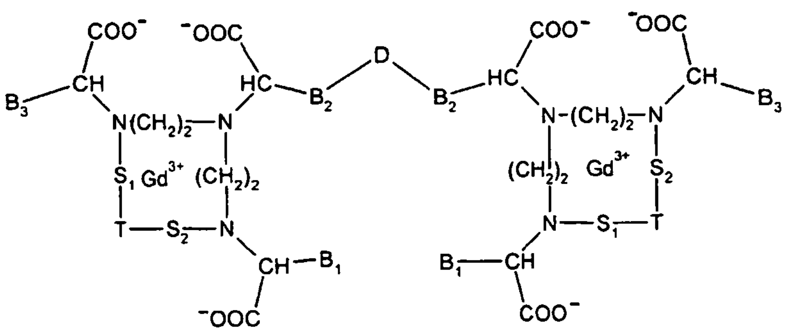

dans lesquels $S_1-T-S_2-$ est :

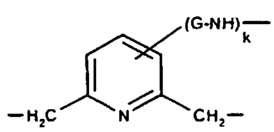

III2

avec k = 0 et $S_1 = S_2 = CH_2$;
$B_3$ représentant G-NH, et B1 et B2 représentant $(CH_2)_xCONHR$ pour IIa2 avec x=1,2 ou 3
$B_2$ représentant G-NH, et B1 et B3 représentant $(CH_2)_xCONHR$ pour II b2

- ou A2 représente 3)

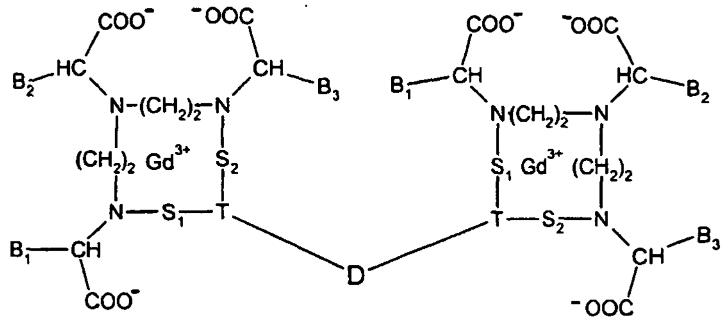

IIc2 (composé dit PCTA C fonctionnalisé) lorsque $S_1$-T-$S_2$- est :

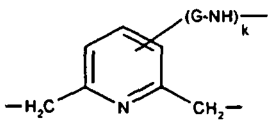

III2

avec k = 1 et $S_1 = S_2 = CH_2$ ;
$B_1$, $B_2$, $B_3$ représentant tous les trois $(CH_2)_xCONHR$ avec x = 1, 2 ou 3 pour II c 2 Sachant que, pour II2, IIa2, IIb2 et IIc2, GNH est choisi parmi les liens 1 et de préférence parmi les groupes $-(CH_2)_n-$
ou

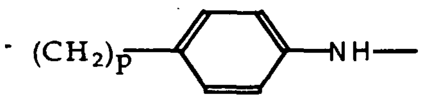 avec p = 0 à 3 ;

NH- avec n = 1 à 4,

- D étant (Div - lien 2), avec Div étant de préférence de type 1,3,5 triazine, D étant de préférence :

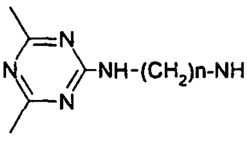

ou

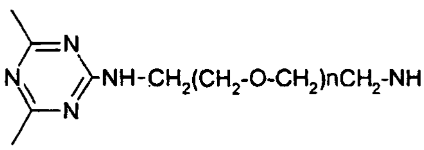

avec n= 2 à 6

- R est tel que mentionné ci-dessus.

Dans les composés I1 et I2 ci-dessus, les restes W1 et W2 dérivent d'acides polycarboxyliques aliphatiques, linéaires ou cycliques ou comportant un ou plusieurs noyaux phényle. On préfère ces derniers notamment lorsque les acides sont fixés sur les noyaux aromatiques.
On peut citer parmi les acides aliphatiques, les acides ($C_4$-$C_{18}$) alcane polycarboxyliques dont la chaîne est éventuellement interrompue par un hétéroatome comme dans $C(CH_2O(CH_2)_2COOH)_4$ décrit dans OPPI Briefs 28(2), 1996, 242-244 ou dans les dérivés d'éthylènediamine dont l'acide diéthylènetriaminopentaacétique, les acides ($C_5$-$C_6$)cycloalcane- polycarboxyliques, l'acide citrique, l'acide 1,4,7,10-tétraazacyclododécane-1,4,7,10-tétraacétique ou l'acide adamantane tétracarboxylique décrit dans J. Org. Chem. 57(1), 1992, 358-362.
Parmi les acides aromatiques, on peut citer les acides phényl-, naphtyl- ou biphényl- polycarboxylique, les acides alkyl- tri ou tétra(phénylcarboxylique), les acides phényl- tri ou tétra(phényl carboxylique) comme

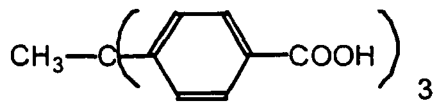

décrit dans Chem. Ber. 123(2), 1990, 375-379,
ou encore les dérivés du phosphazène comme l'hexaacide :

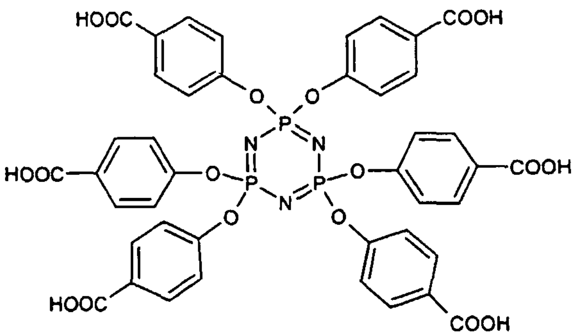

décrit dans Macromolécules 22(1), 1989, 75-79 et 29, 1996, 3694-3700, les dérivés du phloroglucinol comme :

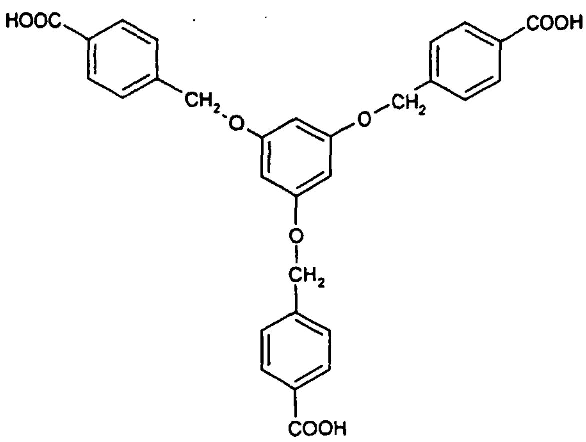

décrit dans J. Org. Chem. 60(5), 1995, 1303-1308.

Enfin, on peut citer parmi les acides aliphatiques ceux comportant des noyaux aromatiques tels que :

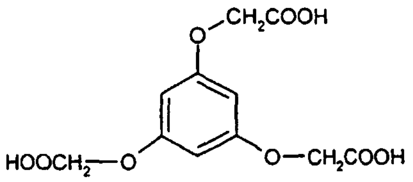

;

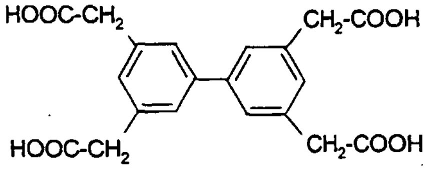

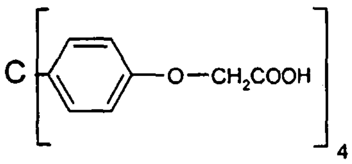

ou l'éther phénoxyacétique de l'hexahydroxyphosphazène.

Parmi les polyacides, on choisira nécessairement ceux qui donneront des produits biocompatibles, étant donné l'application en diagnostic des produits de l'invention. On préfère aussi les acides donnant des restes W1 et W2 rigides et/ou dont la position des groupes carboxyliques permet une disposition régulière dans l'espace des groupes A1 et A2 autour de W1 et W2.

Enfin, un critère de choix peut être l'accessibilité chimique au polyacide de départ et/ou sa réactivité avec les dérivés des chélates porteurs d'une fonction amine.

Les polyacides sont des produits connus ou ils peuvent être préparés par des procédés classiques d'éthérification, carboxylation, amidification à partir de produits connus.

Au lieu d'un noyau central W1 ou W2 portant des groupes carbonyle qui forment des groupes carboxamido avec A1 ou A2, on peut à l'inverse utiliser un noyau W1 ou W2 polyaminé, la structure des liens 2 reliant le noyau central et le ou les chélates étant adaptée en conséquence pour former des liaisons amides, isothiocyanate, isocyanate (connus de l'homme du métier dans Topics in Current Chemistry, New Class of MRI Central Agents, 221,Springer).

La demanderesse a par ailleurs étudié des composés de type polymétalliques de multimères : au lieu d'avoir des diviseurs D reliés à deux chélates de Gd (r=2, donc plusieurs dimères reliés au noyau central), on a des diviseurs D reliés à au moins trois Gd (plusieurs trimères reliés au noyau central lorsque r=3).

Parmi les restes W 1 ou W2, on préfère ceux dérivés des acides :

- 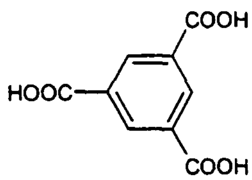

- 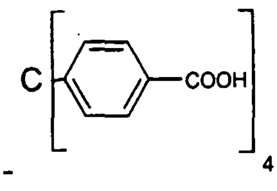

décrit dans Angew. Chem. 98(12), 1986, 1095-1099 ;

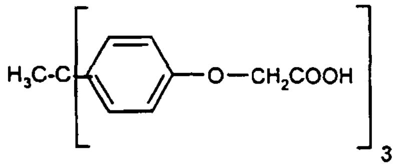

préparé selon US 4,709,008 ;

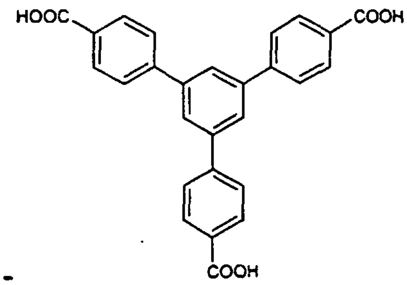

décrit dans Chem. Ber., 123, 1990, 859-867 ;

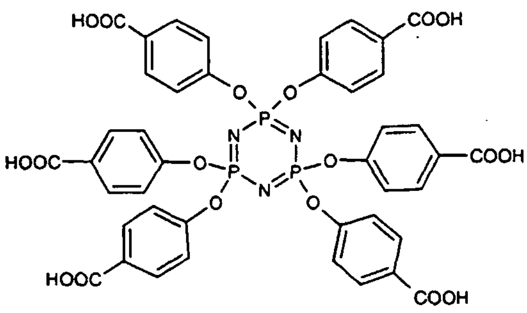

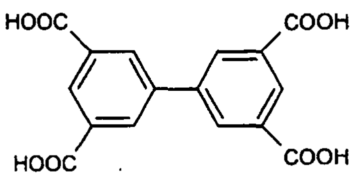

décrit dans J. Org. Chem., 64 (7), 1999, 2422-2427,
et le reste de la 1,3,5-triazine ; dans ce cas, le composé de formule I1 peut être obtenu par action de l'amine AH sur la 2,4,6-trichlorotriazine commerciale.

On peut citer parmi les restes A1 ceux des trois groupes suivants dans lesquels x et R ont les significations précédentes :

- ceux dans lesquels le macrocycle est le cyclen, dans lesquels, dans la formule II1,

$$-S_1-T-S_2-$$

représente :

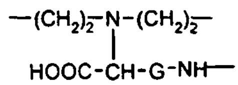

qui ont pour formule :

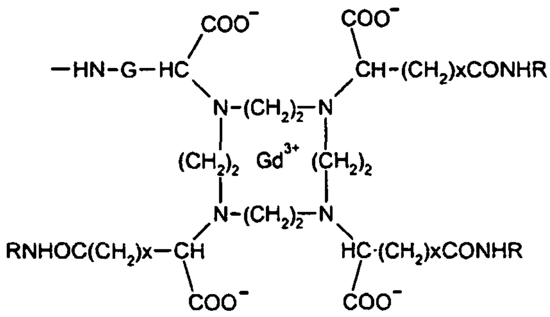

II'1

avec -G-NH étant $-(CH_2)_3$-NH- ou

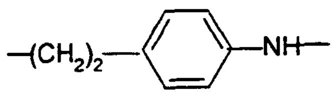

- ceux dans lesquels le macrocycle est le 3,6,9,15-tétraazabicyclo[9.3.1]pentadéca-1(15),11,13-triène, fonctionnalisé sur l'un des atomes d'azote aliphatiques de formule II1' dans laquelle
  $-S_1$ - T - $S_2$- représente :

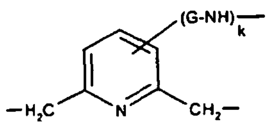

avec k = 0
de formule :

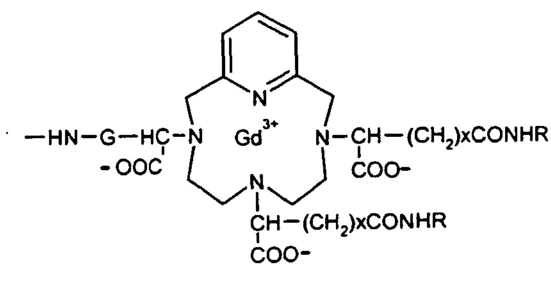

II"a1

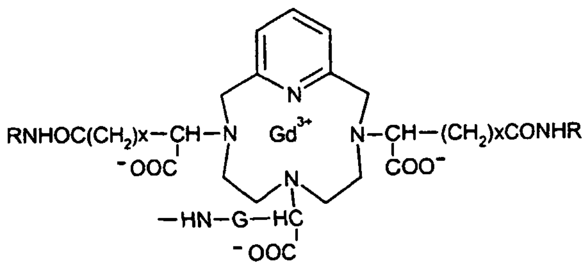

II " b 1

ou :

avec -G-NH = $-(CH_2)_3$-NH- ou

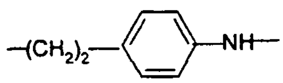

- et ceux fonctionnalisés sur le noyau pyridyle de formule II1 dans laquelle $-S_1 - T - S_2-$ représente :

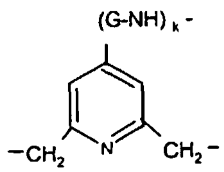

avec k = 1 et G = $(CH_2)_3$;

de formule :

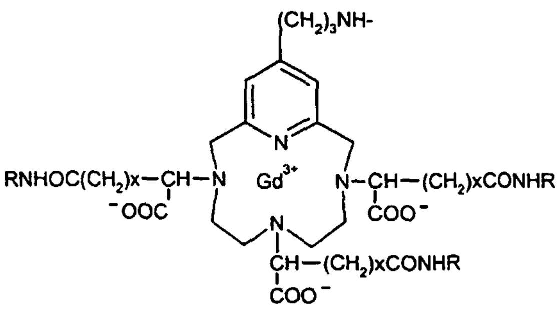

II "' 1

et spécialement les restes A1 dans lesquels x = 2.

De manière analogue, on peut citer parmi les composés A2 des composés analogues des trois composés précédents II ' 1, II " a1, II " b1, II"' 1, en remplaçant le monomère de Gd par un dimère de Gd, respectivement II ' 2, II " a2, II"b2, II"' 2.

1)

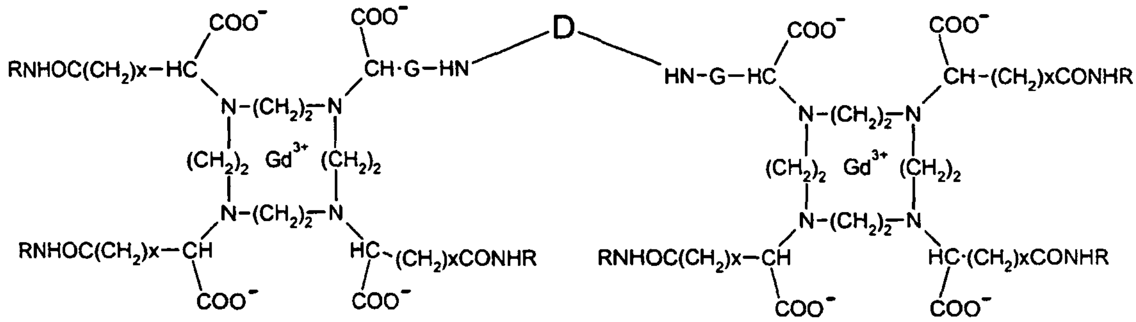

II ' 2

avec -G-NH étant $-(CH_2)_3$-NH- ou

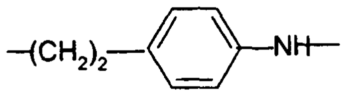

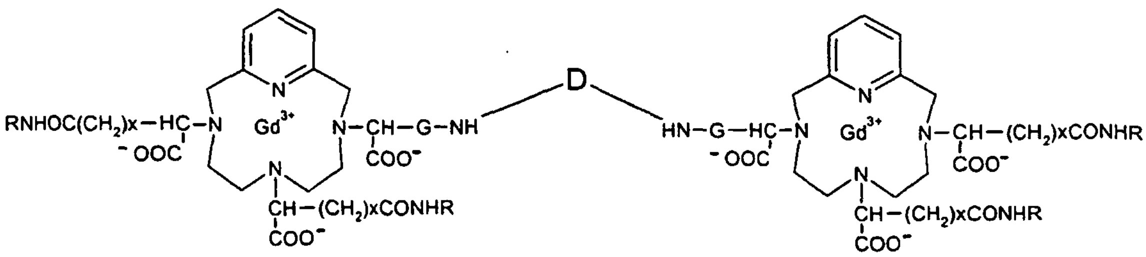

2)

II" a2

Ou II " b2 (isomère de position de II"a2)

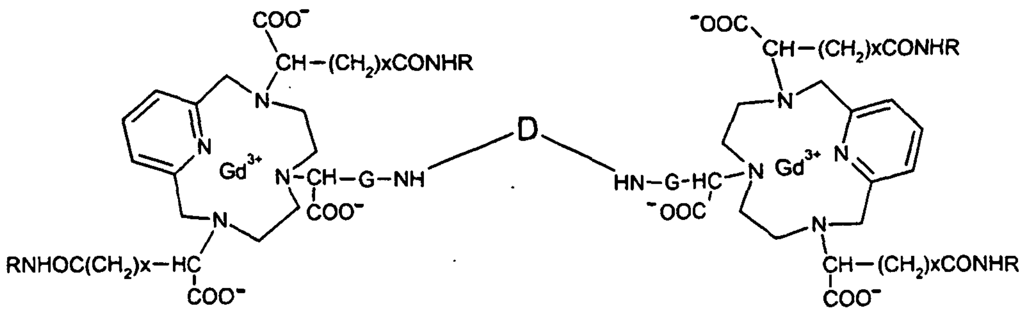

avec -G-NH étant $-(CH_2)_3$-NH- ou

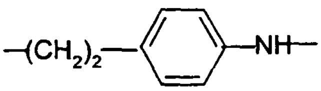

3) 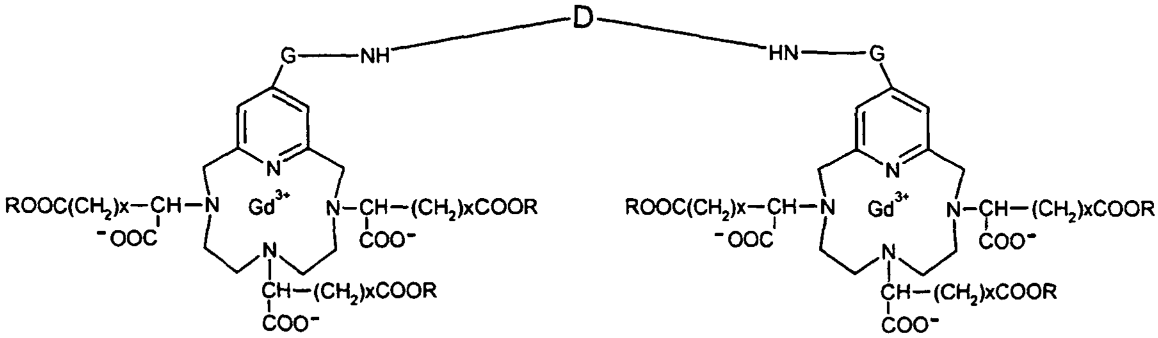

II'''2

Avec G-NH étant -(-$CH_2$)$_3$-NH.
et spécialement les restes A2 dans lesquels x = 2.

Les composés de formules II'$_2$, II"$_{a2}$, II"$_{b2}$ sont obtenus au départ de deux équivalents des composés de structure $V_1$ tels que définis dans la demande, par double réaction de substitution sur le 2,4,6-trichloro-1,3,5-triazine en milieu aqueux ou dans un mélange composé d'eau et d'un solvant polaire miscible à l'eau, en contrôlant le pH et la température.

Les résidus de formule R- sont introduits par couplage peptidique selon les méthodes connues de l'homme du métier, des amines correspondantes de formule R-$NH_2$ dont la structure a été définie précédemment, par exemple en milieu aqueux en présence d'un agent de couplage compatible comme l'EDCI et éventuellement d'un catalyseur.

Le troisième atome de chlore est finalement déplacé par un large excès de diamine par exemple de formule $H_2N$-($CH_2$)$_a$-$NH_2$ ou $H_2N$-$CH_2$-($CH_2$-O-$CH_2$)$_b$$CH_2$-$NH_2$ avec a=2 à 5 et b=1 à 4.

Les composés de formule II'''$_2$ sont préparés au départ des précurseurs aminés dérivés des résidus de formule II'''$_1$ et de structure :

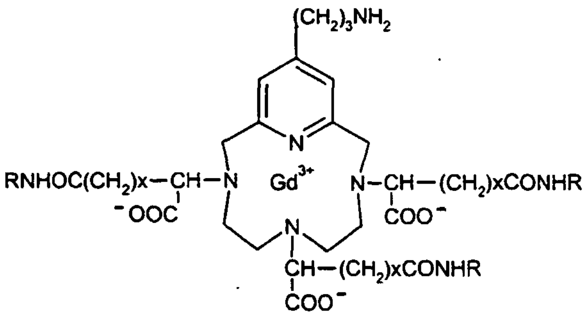

selon un protocole analogue par double substitution du cycle triazine et déplacement de l'atome de chlore résiduel par un large excès de diamine tel que définie précédemment.

Parmi les composés de formule I1 et I2, on préfère ceux dans lesquels $B_1$, $B_2$, $B_3$, lorsqu'ils ne représentent pas G-NH, représentent ($CH_2$)$_2$CONHR avec dans R, p = q = 0 et Z = $CH_2$CONH et notamment :

- soit R représente R' :

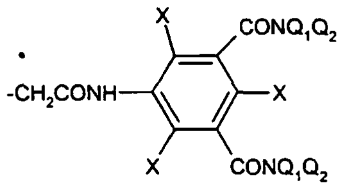

- soit R représente R" :

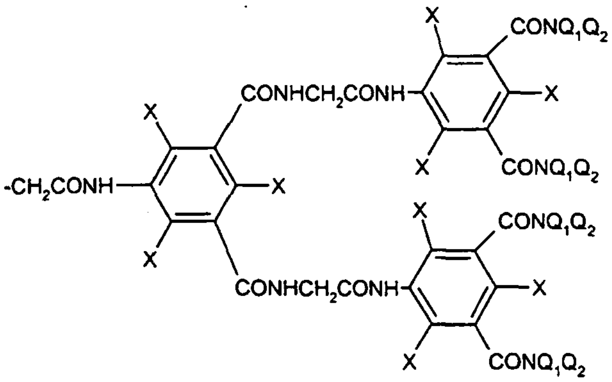

et dans R' et R" les X sont identiques et représentent Br ou I tandis que $Q_1$ et $Q_2$ identiques ou différents, sont des groupes $(C_1\text{-}C_8)$alkyle, mono- ou polyhydroxylés, de telle sorte que chaque $CONQ_1Q_2$ comporte de 4 à 10 hydroxyles au total.

En outre, les composés de formule I1 dans lesquels R comprend 2 ou 3 noyaux phényle, présentent des avantages pour certaines applications spécifiques, du fait de leur masse et/ou volume moléculaire élevé. Parmi ceux-ci, on peut citer plus particulièrement ceux dans lesquels R représente R"' :

- soit

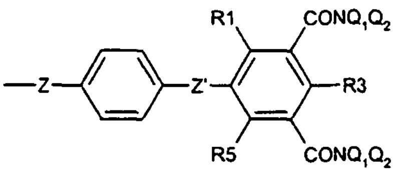

et Z est $CH_2$ ou $CH_2CONH$ et Z' est CONH ou $CONHCH_2CONH$ ;

- soit R"" :

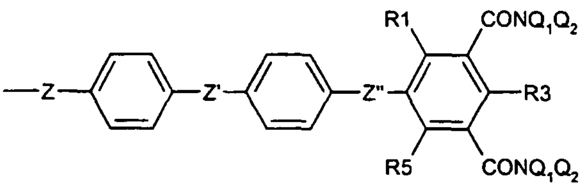

et Z est $CH_2CONH$, Z' est CONH, Z" est $CONHCH_2CONH$ ;
et dans R"' et R"", $R_1$, $R_3$ et $R_5$, identiques, sont Br ou I et $Q_1$ et $Q_2$, identiques

ou différents, sont des groupes $(C_1\text{-}C_8)$alkyle, monohydroxylés ou polyhydroxylés, de telle sorte que chaque groupe $CONQ_1Q_2$ comporte 4 à 10 hydroxyles au total.

On préfère aussi des composés (branches désignées flash) dans lesquels R représente :

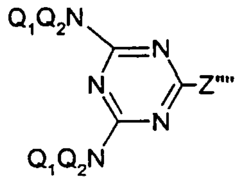

avec Z "" étant $NQCH_2(CH_2OCH_2)_i(CH_2)_jNH_2$, avec i = 2 à 6 et j = 1 à 6

Enfin, les composés de formule I1 ou I2 dans lesquels $Q_1$ représente $CH_2CHOHCH_2OH$ ou $CH_2(CHOH)_4CH_2OH$ et $Q_2$ représente $CH_2(CHOH)_4CH_2OH$ notamment dans les groupes $CONQ_1Q_2$, sont généralement préférés pour former des molécules suffisamment hydrophiles en terme de solubilité aqueuse et de biocompatibilité.

Les produits de formule I1 peuvent être préparés soit à partir des amines A1H, soit à partir de l'un de leurs précurseurs A'1NH, notamment les composés dans lesquels il y a $(CH_2)_xCOOH$ au lieu de $(CH_2)_xCONHR$ dans $B_1$, $B_2$ ou $B_3$. A1H et A'1NH, dérivés fonctionnalisés de chélates de gadolinium macrocycliques sont des intermédiaires de synthèse de tous les composés de formule I1 nouveaux, dont il a été nécessaire de mettre au point un procédé de préparation adapté à la nature d'aminoacide du groupe réactif greffé sur le macrocycle permettant de fixer le chélate sur le reste W1 sans perte de stabilité du chélate macrocyclique.

L'invention concerne donc aussi les précurseurs des composés polymétalliques selon l'invention, nécessaires à leur synthèse.

Selon une réalisation l'invention concerne les précurseurs A'1NH de formule :

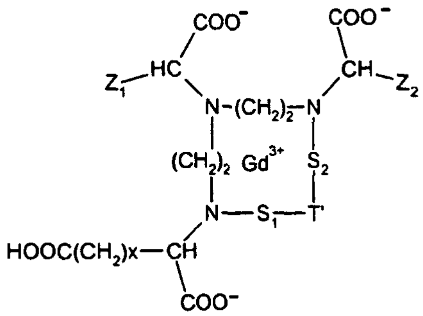

V1

dans laquelle x = 1, 2 ou 3 et $-S_1-T'-S_2-$ est :

1)

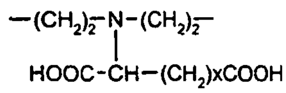

avec $S_1 = S_2 = (CH_2)_2$

ou

2)

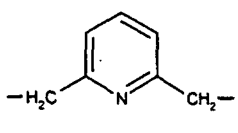

avec $S_1 = S_2 = CH_2$

et l'un des groupes $Z_1$ ou $Z_2$ est choisi parmi les groupes $-(-CH_2)_3NH_2$ ou

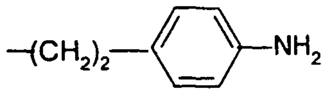

dans lequel le groupe $NH_2$ peut être éventuellement protégé de façon classique, sous forme de carbamate, de phtalimide ou de benzylamine comme décrit de façon générale dans Protective Groups in Organic Synthesis, 3rd Ed., Ed. T.W. Greene, Pig. M. Wuts (J. Wiley) p. 494-653, et l'autre des $Z_1$ ou $Z_2$ est $(CH_2)_xCOOH$, sous forme de sels avec une base alcaline.

Les composés V1 du 1) sont désignés de type DOTA RR, les composés du 2) sont désignés de type PCTA RR N fonctionnalisé.

L'invention concerne également les précurseurs A'1NH de formule :

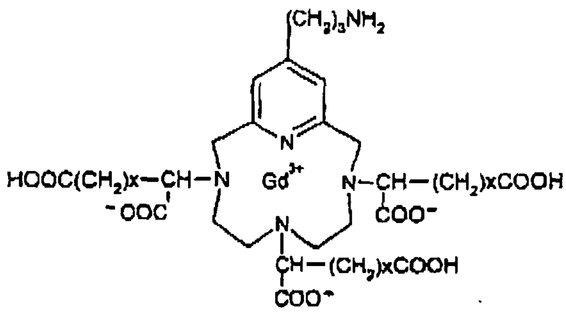

VI 1

avec x = 1, 2, 3
dans lequel le groupe $NH_2$ est éventuellement protégé ou salifié,
et particulièrement les composés V1 et VI1 dans lesquels x = 2,
sons forme de sels avec une base alcaline, telle que NaOH ou KOH,

Ces composés VI1 sont désignés PCTA RR du type C fonctionnalisé, la fonction amine étant située sur le cycle externe.

Dans le cas où W1 est le reste 1,3,5-triazino, on fait réagir de préférence le précurseur V1, VI1 ou VI'1 sur la 2,4,6-trichloro-1,3,5-triazine dans les conditions habituelles d'une substitution nucléophile en présence d'une base dans un solvant polaire aprotique, éventuellement en mélange avec de l'eau, notamment comme décrit dans Comprehensive Organic Chemistry, D. Bostow, W. Ollis, vol. 4, p. 150-152 (Pergamon Press) ou dans Tetrahedron Letters, 41(11), 2000, 1837-1840. On peut effectuer la réaction en présence d'une base minérale telle que NaOH ou $Na_2CO_3$ ou d'une amine tertiaire, telle que la triéthylamine, par exemple dans l'eau en présence de 5 à 60% en volume de dioxanne-1,6, de tétrahydrofuranne ou de diméthylformamide.

Les chélates V1 ou VI1 ou éventuellement le produit de condensation sur W1 sont porteurs des groupes acide :

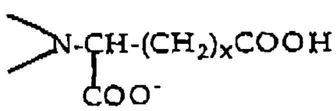

et peuvent ensuite être mis à réagir avec l'amine $RNH_2$ en milieu aqueux, éventuellement en présence d'un tiers solvant tel que dioxanne ou tétrahydrofuranne, avec activation des groupes carboxyliques par addition d'un carbodiimide soluble, qui est porteur d'un groupe amine comme décrit dans J. Org. Chem., 21 (1956), 439-441 et 26(1961), 2525-2528, ou qui est porteur d'un groupe ammonium quaternaire comme le 1-éthyl-3-(3-diméthylamino)carbodiimide (EDCI) ou le 1-cyclohexyl-3-(2-morpholinoéthyl)carbodiimide métho p-tolylsulfonate d'Org. Synthesis vol. V, 555-558.

Parmi les autres activateurs des acides carboxyliques dans les réactions d'amidification, on peut mentionner aussi le N-hydroxysulfosuccinimide (NHS), Bioconjugate Chem. 5, 1994, 565-576 ou le 2-succinimido-1,1,3,3-tétraméthyluronium tétrafluoroborate de Tetrahedron Letters, 30, 1989, 1927-1930. On peut aussi utiliser un mélange d'EDCI et NHS.

Un certain nombre des amines $RNH_2$ nécessaires à la préparation des composés de formule I sont connues, décrites notamment dans les demandes de brevet précédemment citées WO 97/01359, WO 00/75241 et EP-A-922700. Les autres pourront être préparées par des procédés analogues, relevant des connaissances générales de l'homme du métier.

Dans le cas où W1 est un dérivé de polyacide, on préfère préparer préalablement l'amine A1H à partir des chélates V1 ou VI1 porteurs des fonctions acides et éventuellement protégés sur les fonctions amines sur lesquels on fait réagir l'amine $RNH_2$ appropriée par mise en oeuvre de l'une des méthodes classiques d'amidification peptidique, citées précédemment.

Pour préparer les composés intermédiaires de formule V1 dans lesquels $Z_1$ ou $Z_2$ représente $(CH_2)_3NH_2$, on peut faire réagir dans une première étape sur le composé macrocyclique correspondant dans lequel l'atome d'azote porteur dudit groupe $Z_1$ ou $Z_2$ est libre et les autres atomes d'azote ont été éventuellement préalablement protégés, de façon connue en soi, le composé Y'1-Br de formule :

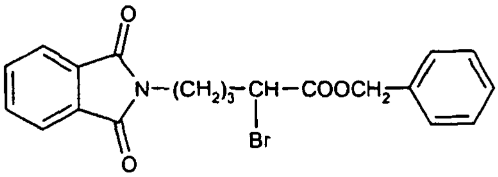

préparé selon Tetrahedron Letters 38(47), 1997, 8253-8256 et J. Org. Chem., 50, 1985, 560-565, tandis que pour ceux dans lesquels $Z_1$ ou $Z_2$ représente :

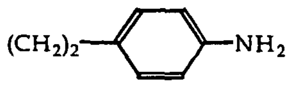

on fait réagir le composé Y"1-Br de formule :

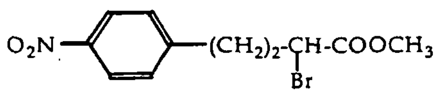

décrit dans J. Org. Chem. 58, 1993, 3869-3876.

On fait ensuite réagir sur les autres atomes d'azote macrocyclique après leur éventuelle déprotection, le diacide bromé, protégé sous forme d'ester Y"'1Br:

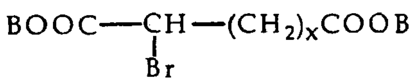

lequel peut être par exemple préparé :

- pour x = 1, B = diphénylméthyle,
  selon J.B.I.C. 4, 1999, 341-347 ;
- pour x = 2, B = $(C_1-C_3)$alkyle ou benzyle,
  selon WO 00/75241 ;
- et pour x = 3, B = $CH_3$,
  selon EP-A-614 899,
  avant de libérer la fonction amine du groupe phtalimido ou de réduire le groupe nitro introduits précédemment. Les fonctions acides sont déprotégées par action d'une base ou d'un acide en milieu aqueux ou hydroalcoolique, avant ou après la formation du groupe amino.

Après libération des fonctions acides carboxyliques, le complexe de gadolinium est ensuite préparé selon l'une

des méthodes connues notamment de US 5,554,748 ou Helv. Chim. Acta, 69, 1986, 2067-2074, par action de $Gd_2O_3$ on $GdCl_3$ en milieu aqueux à pH compris entre 5 et 7.

Lors de la préparation du produit de formule V dans lequel T' représente le pyridyle, lorsque, dans la première étape, Y'Br ou Y"Br sont mis à réagir sur le macrocycle dont aucun des atomes d'azote n'est bloqué, on obtient, après réaction avec Y"'Br et formation du groupe amino, les dérivés dissymétriques du type :

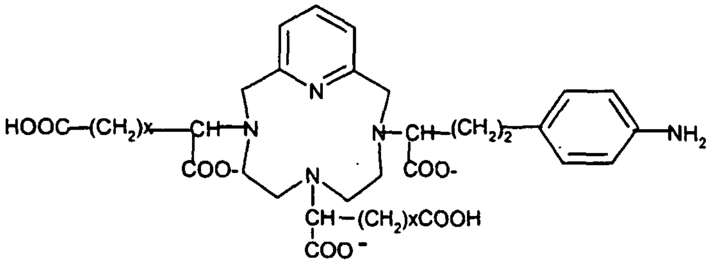

VII 1

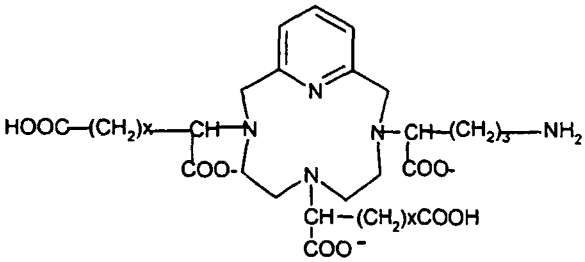

VII ' 1

Ces composés VII'1 sont désignés PCTA RR du type N fonctionnalisé, la fonction amine étant située sur un bras latéral.

Pour obtenir le dérivé substitué symétriquement, on peut utiliser le procédé selon le schéma réactionnel du tableau 1 suivant dans lequel x et B ont les significations précédentes, à partir de la triamine protégée (a) décrite dans Tetrahedron Letters, 41(39), 2000, 7443-7446, suivi des étapes analogues à celles citées pour le composé dissymétrique :

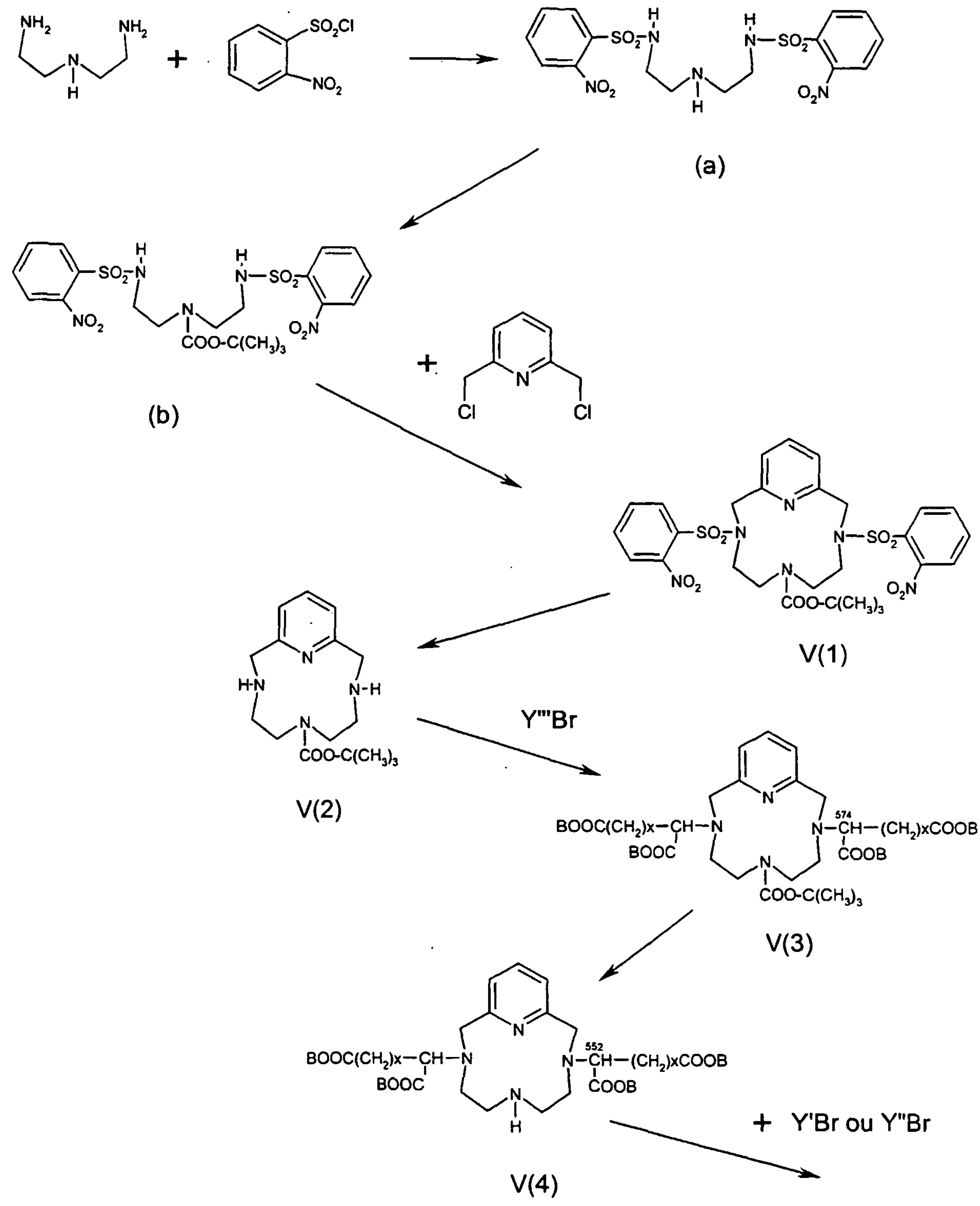


TABLEAU 1

(Les composés V(1) à V(4) sont des intermédiaires de V1)

Pour préparer les composés de formule VI 1, on effectue une réaction de Heck sur le macrocycle bicyclique, bromé sur le noyau pyridyle, de formule :

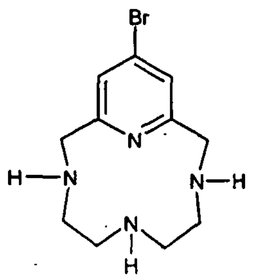

décrit dans J. Heterocyclic Chem. 27, 1990, 167-169, suivi d'une réduction. La réaction de Heck peut être réalisée dans les conditions décrites dans Metal Catalyzed cross-coupling reactions, Ed. F. Diederich, P.J. Stang, Wiley, VCH, chap. 3, p. 99-166. Le schéma réactionnel des premières étapes du procédé de la préparation de VI est représenté tableau 2 ; on effectue ensuite l'hydrolyse des groupes esters et la complexation du gadolinium, avant ou après la déprotection du groupe amino par action de l'acide trifluoroacétique.

Pour préparer les amides résultant de la réaction du composé VI avec une amine $RNH_2$, on préfère effectuer l'amidification avant la déprotection de l'amine aliphatique.

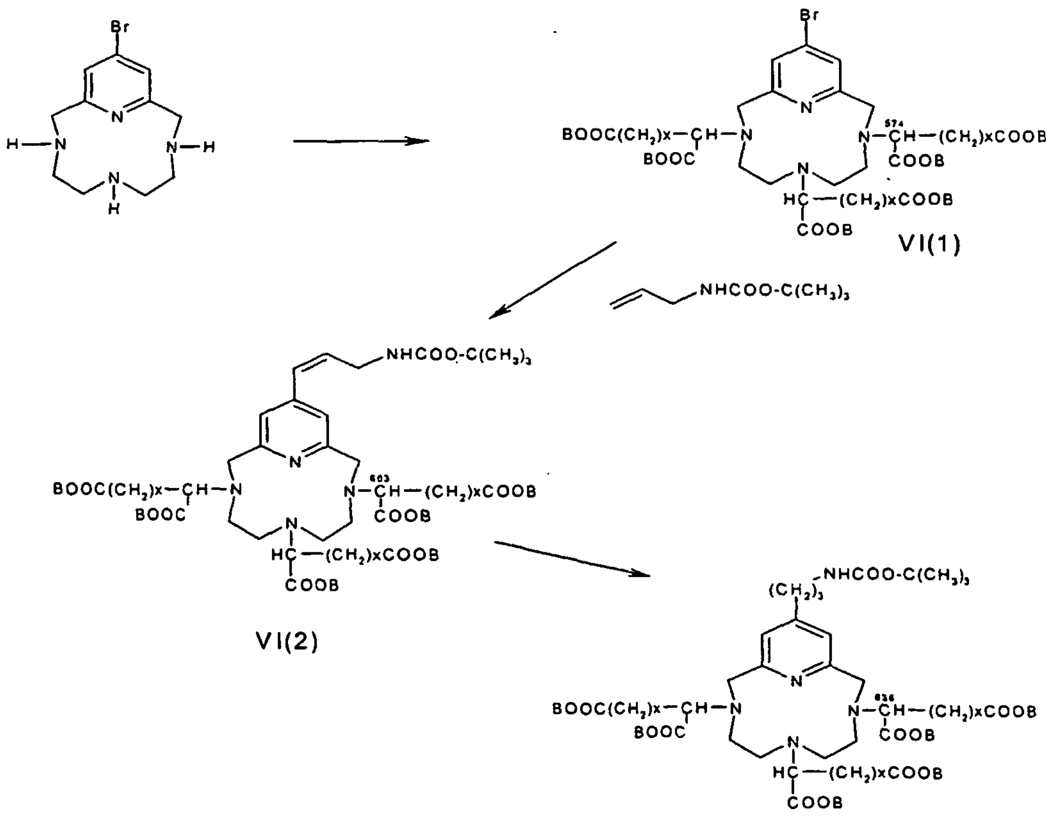

VI(1)

VI(2)

VI(3)

TABLEAU 2

Les composés VI (1) à VI (4) sont des intermédiaires de VI 1.

De manière analogue, l'invention concerne aussi les précurseurs $A'_2NH$, dimères de formule :

1)

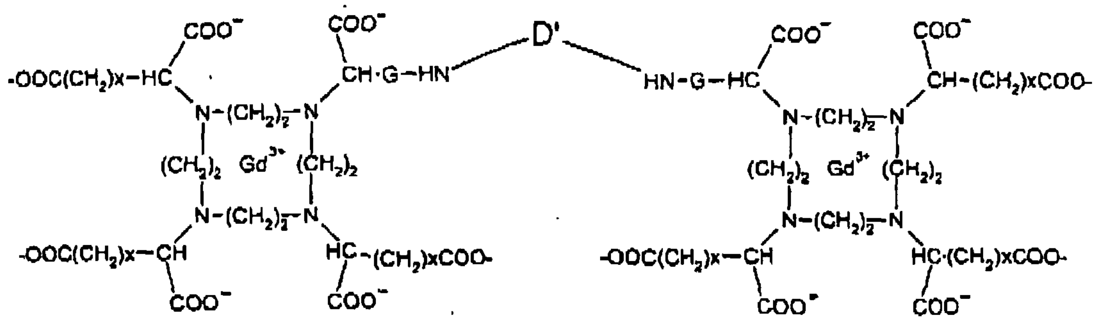

V2 précurseur de II'2
dans laquelle x = 1, 2 ou 3 de préférence x=2
D'=D-H, D étant

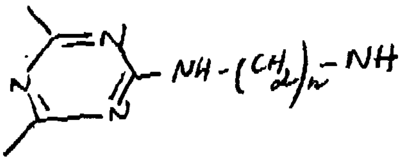

ou

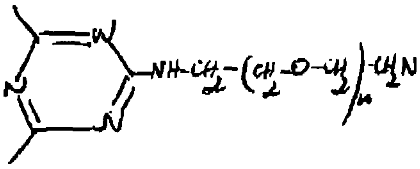

avec n = 2 à 6
G-NH étant $-(CH_2)_3$-NH- ou

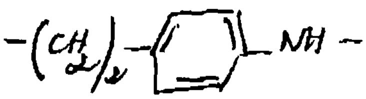

sous forme de sels avec une base alcaline.

2)

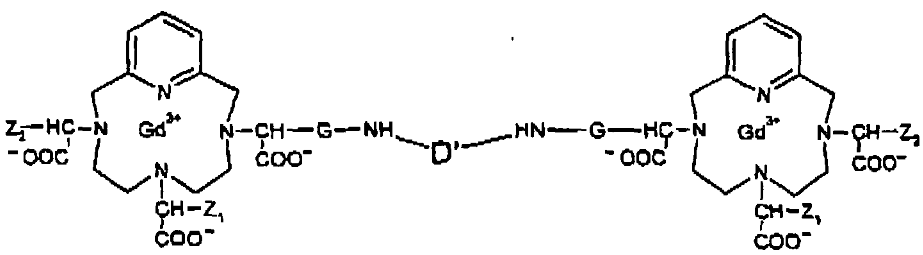

V " a2 précurseur de II " a2
avec pour 1) et 2);

- G-NH est choisi parmi les groupes $-(-CH_2)_3NH$ ou

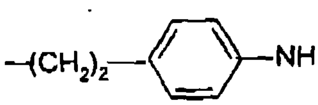

avec pour 2) :
$Z_1$ et $Z_2$ sont $(CH_2)_xCOOH$. dans lesquelles x = 1, 2 ou 3 de préférence x = 2

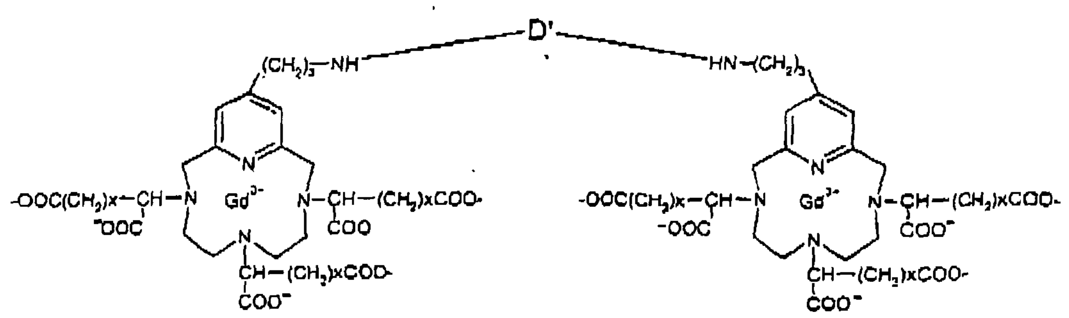

3)

VI 2 précurseur de II "' 2
avec x=1, 2 ou 3, de préférence x=2
D'=D-H, D étant

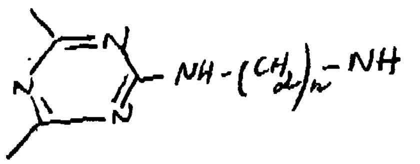

ou

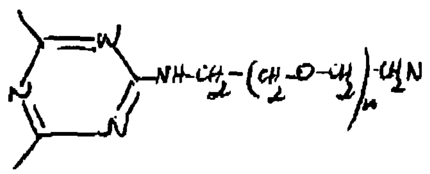

avec n = 2 à 6
G-NH étant $-(CH_2)_3$-NH- ou

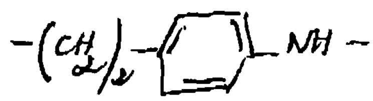

sous forme de sels avec une base alcaline.

L'invention concerne aussi les produits de contraste pour l'imagerie médicale par résonance magnétique qui comprennent au moins un composé de formule (E), de préférence de formule Poly M RR et Poly D RR, éventuellement associé à un véhicule et à des additifs pharmaceutiquement acceptables pour une administration par voie orale ou par injection intravasculaire sous-cutanée, ou percutanée. Les compositions de diagnostic pour voie orale seront présentées sous forme de comprimés ou de gélules, ou suspensions et solutions buvables. La solubilité aqueuse et la faible osmolalité des composés de l'invention permettent de préparer des solutions aqueuses isotoniques, de forte concentration et de viscosité acceptable pour l'injection.

Selon un autre aspect, l'invention concerne les complexes paramagnétiques formés entre les ligands de l'invention et les ions métalliques paramagnétiques convenables, autres que le gadolinium, tels que ceux du dysprosium ou du manganèse, ainsi que les compositions d'agents de contraste pour l'imagerie médicale par résonance magnétique nucléaire qui comprennent ces complexes, associés aux véhicules et additifs usuels. Les ligands selon l'invention peuvent aussi former des complexes avec des radioéléments comme Tc, In, ou Yb, qui peuvent être utilisés pour établir un diagnostic ou effectuer un traitement thérapeutique. Ces complexes se présentent, en général, sous forme de sel interne, résultat de la neutralisation par le cation métallique central de groupes acides du ligand; lorsque le complexe comprend d'autres groupes acides ceux-ci peuvent être salifiés par une base minérale ou organique pharmaceutiquement acceptable y compris les aminoacides, par exemple NaOH, lysine, N-méthylglucamine, arginine, ornithine ou diéthanolamine. Les doses auxquelles les agents de contraste selon l'invention peuvent être administrés dépendent de la nature du complexe, de la relaxivité qu'il induit, de la voie d'administration et de l'organe visé. Par exemple par voie orale, notamment pour la sphère gastro-intestinale, on pourra administrer de l'ordre de 0.01 à 3 mmol/kg d'animal, et par voie

parentérale de l'ordre de 0,001 à 0,02 mmol/kg.

Les additifs classiques peuvent être introduits dans les compositions de diagnostic de l'invention tels que tampons, anti-oxydants, électrolytes, tensio-actifs, polyols, ainsi que d'autres chélates des cations biologiques ou d'agents complexants en faible quantité.

Les solutions peuvent être préparées extemporanément à partir de la poudre lyophilisée contenant le composé de formule (E) et éventuellement des additifs et un solvant stérile ou, du fait de la grande stabilité des complexes en solution in vitro, comme *in vivo,* les solutions peuvent être fournies au radiologue en flacons ou en seringues.

Les doses unitaires seront fonction de la structure du composé de formule E, de la voie d'administration, du type de diagnostic à établir, ainsi que du patient. Les doses unitaires seront en général de 0,1 μmole à 150 μmole de gadolinium par kg, de préférence de 1 à 100 μmole de gadolinium par kg pour un homme de taille moyenne.

Du fait de leur confinement vasculaire, de leur élimination ralentie et de leur forte relaxivité, les compositions de diagnostic de l'invention sont utiles pour l'imagerie des vaisseaux sanguins et tissus lymphoïdes. Ils permettent la détermination de la perfusion et du volume sanguin dans les territoires pathologiques, l'étude de la perméabilité microvasculaire et l'identification des états d'ischémie ou la caractérisation des tumeurs et des états inflammatoires.

L'invention concerne donc en particulier l'utilisation des composés de diagnostic de formule (E) et notamment de formule I1 ou 12, pour le diagnostic par imagerie, et leur utilisation pour la préparation d'une composition de diagnostic de ces indications. L'invention concerne aussi une méthode de diagnostic d'imagerie médicale utilisant ces composés.

L'invention concerne aussi un procédé de criblage, et les composés obtenus par ce procédé de criblage, consistant à sélectionner les composés de formule (E), efficaces sur le plan diagnostic (propriétés pharmacocinétiques et de biodistribution), ledit procédé comprenant :

- la préparation d'un composé candidat polymétallique de formule (E)
- l'utilisation du composé candidat dans un protocole de test approprié d'une indication diagnostique
- la sélection des candidats présentant une efficacité massique d'au moins 30%, de préférence d'au moins 50% supérieure à celle du chélate non polymétallique correspondant.

Comme exemple de test de criblage on utilisera par exemple les tests *in vitro* et/ou *in vivo* décrits en détail par la demanderesse dans le document Physical and biological evaluation of P792, a rapid clearance blood pool agent for Magnetic Resonance Imaging, Investigative radiology, vol 36, n°8, 445-454,2001.

On décrit maintenant plusieurs exemples de composés polymétalliques selon l'invention, à titre d'illustration.

Les exemples 1 à 13 et 39 concernent des polymétalliques de monomères.

Les exemples 25 à 36, 38, 41 et 43 concernent des polymétalliques de dimères ; les exemples 14 à 24, 37, 40 et 42 des précurseurs dimériques de ces polymétalliques.

## EXEMPLE 1

Composé de formule VI 1 avec x = 2 selon méthode du tableau 2

a) Composé de formule VI(1) avec B = éthyle

22 g de 13-bromo-3,6,9,15-tétraazabicyclo[9.3.1.]pentadéca-1(15),11,13-triène sont introduits dans 440 ml de $CH_3CN$ en présence de 48 g de $K_2CO_3$ calciné et le mélange est maintenu à 80° C pendant 1 h avant l'addition d'une solution de 93 g de 2-bromoglutarate d'éthyle dans 100 ml de $CH_3CN$ ; le milieu réactionnel est alors agité 20 h à 80° C puis refroidi à température ambiante, filtré et le solvant est évaporé.

Le résidu est repris par 500 ml d'une solution aqueuse de HCl 1 N en présence d'un volume d'éther diéthylique. Après séparation de la phase organique, la phase aqueuse est neutralisée par $NaHCO_3$ puis extraite par $CH_2Cl_2$. Après lavage à l'eau puis séchage sur sulfate de magnésium, la phase organique est concentrée et le résidu est purifié sur colonne de silice (Merck® 500 g, d = 10 cm) en éluant par $CH_3COOC_2H_5$.

m = 37 g ;
HPLC : colonne n° 1 : Symmetry® C18 ; 100 Å ; 5 μm ; L = 25 cm ;
d = 4,6 mm (Waters) ;
éluant n° 1 : $CF_3COOH$ dans l'eau (pH 3)/$CH_3CN$, gradient linéaire de 90/10 à 20/80 (v/v) en 40 min, débit 1 ml/min : $t_r$ = 26 min (2 pics).

b) Composé de formule VI (2)

On ajoute 23,5 g de 3-(tert-butyloxycarbonylamino)-propène, 25,3 ml de triéthylamine, puis 3,4 g de triphénylphosphine et enfin 1,8 g d'acétate de palladium à une solution de 28 g du composé obtenu à l'étape a) dissous dans 400 ml de toluène.

Après chauffage à 80° C pendant une nuit sous atmosphère inerte, le milieu est évaporé et le résidu est repris par une solution aqueuse d'acide chlorhydrique (pH = 1).

La phase aqueuse est lavée avec 1 volume d'éther diéthylique puis de toluène avant d'être amenée à pH 6 par addition de NaOH (1 N).

Après extraction de la solution aqueuse par $CH_2Cl_2$, la phase organique séchée sur sulfate de magnésium est évaporée.

On obtient une huile marron.

m = 17 g,
HPLC : colonne n° 1, éluant n° 1, gradient linéaire de : 60/40 à 20/80 (v/v) en 50 min, débit 1 ml/min : tr = 14 min à 19 min (3 pics des isomères).

c) Composé de formule VI (3)

A 17 g du composé obtenu à l'étape b) dissous dans 350 ml de $CH_3OH$, on ajoute 3 g de catalyseur charbon palladié à 10% puis le milieu réactionnel est agité durant 2 h 30 à 20° C sous $4.10^5$ Pa d'hydrogène.

Après filtration sur Clarcel®, le solvant est évaporé et on obtient 16,8 g d'huile brute.

HPLC colonne n° 1, éluant n° 1, gradient linéaire de : 60/40 à 20/80 (v/v) en 50 min, débit 1 ml/min : tr = 14-15-21 min (3 pics isomères).

d) Hydrolyse des groupes esters éthyliques

20 g du composé obtenu à l'étape c) dissous dans 50 ml d'une solution aqueuse de NaOH 5N et 80 ml de $CH_3OH$ sont chauffés à 70° C pendans 18 h.

Après concentration du milieu réactionnel, le résidu est repris dans l'eau et la solution, amenée à pH 5,5-6 par quelques gouttes d'acide acétique, est concentrée avant d'être purifiée par chromatographie sur une colonne (d = 15 cm) contenant 1 kg de silice silanisée (Merck® 0,063 - 0,200 μm) en éluant à l'eau.

Après concentration à sec on obtient 9,3 g de cristaux blancs.

HPLC colonne n° 1, éluant n° 2 : $H_2SO_4$ dans l'eau (0,037 N)/$CH_3CN$ gradient linéaire de :98/2 à 20/80 (v/v) en 50 min :
tr = 16,7 - 17,5 - 17,9 min (3 pics).

e) Complexation du gadolinium

8,7 g du composé obtenu à l'étape d) sont dissous dans 70 ml d'eau puis on ajoute en une fois 2,1 g de $Gd_2O_3$ et l'ensemble est chauffé à 60° C durant 3 h 45 min en maintenant le pH entre 5,5 et 6 par addition d'une solution aqueuse de NaOH 1 N.

Après filtration, le milieu réactionnel est évaporé et le résidu est cristallisé dans l'éthanol.

On obtient 9,6 g de cristaux blancs.

HPLC : colonne n° 1, éluant n° 2, gradient linéaire de : 98/2 à 20/80 (v/v) en 50 min, débit 1 ml/min : tr = 31 à 33 min (4 pics).

f) Libération de l'amine

On maintient 3 h sous agitation une solution de 9 g du complexe obtenu à l'étape e) dans 180 ml de $CF_3COOH$ à 25° C avant d'éliminer le liquide sous pression réduite.

Le résidu est repris dans l'éther diéthylique et la suspension filtrée. Après élimination du solvant, le résidu est introduit par portions dans une suspension d'au moins 5 ml de résine anionique faible ($OH^-$) dans 50 ml d'eau ; en fin d'addition le pH, stable, doit être de 8 à 8,5.

La résine est alors séparée par filtration, le solvant éliminé et le résidu précipité par addition d'éther éthylique.

**<u>EXEMPLE 2</u>**

Composé de formule V1 dans laquelle x = 2, et

$-S_1$-T'-$S_2$- est, avec $S_1 = S_2 = CH_2$,

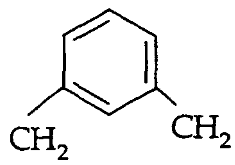

et $Z_1$ est

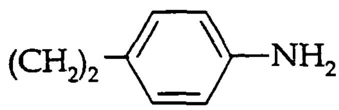

et $Z_2$ est $(CH_2)_2$-COOH préparé selon la méthode du tableau 1.

a) N,N"-bis(o-nitrophénylsulfonyl)diéthylènetriamine

On dissout 8,4 g de cristaux de NaOH dans 100 ml d'$H_2O$ refroidie à 0° C et on ajoute 9,2 g de diéthylènetriamine, puis goutte à goutte à 0° C une solution de 41,5 g du chlorure de l'acide 2-nitrophénylsulfonique dissous dans 100 ml de tétrahydrofuranne.
Une fois concentré, le milieu réactionnel est repris dans $CH_2Cl_2$ et la phase organique est lavée par $H_2O$ puis séchée sur sulfate de magnésium. Après évaporation du solvant, on obtient 37,5 g de cristaux qui sont engagés dans l'étape suivante.
HPLC : colonne n° 1, éluant n° 3 : $CF_3COOH$ dans $H_2O$ , pH 3,2 : tr = 16 min (32 min pour le dérivé trisubstitué).

b) N,N"-bis(2-nitrophénylsulfonyl)N'-t-butoxycarbonyl diéthylène triamine

18 g de carbonate de di-tert-butyle sont ajoutés par fraction à une solution contenant 32,4 g de composé obtenu à l'étape a) dans un mélange de 97 ml de solution aqueuse de NaOH 2N et 225 ml de $CH_3CN$.
Après 3 h d'agitation à 25° C, le milieu réactionnel est évaporé à sec et le résidu est repris par 400 ml de $CH_2Cl_2$. La phase organique est lavée 2 fois avec 100 ml d'$H_2O$.
Après séchage sur sulfate de magnésium puis concentration de la phase organique, le résidu obtenu est purifié par chromatographie sur colonne (d = 15 cm) contenant 1 kg de silice (Merck® 40-63 $\mu$m) en éluant avec un mélange $CH_2Cl_2/CH_3COCH_3$ gradient de 99/1 à 90/10 (v/v).
Après évaporation du solvant, on obtient 28,5 g de produit.

HPLC : colonne n° 1, éluant n° 1, débit 1 ml/min, gradient linéaire 90/10 à 10/90 (v/v) en 40 min : tr = 29 mn.

c) Composé de formule V(1)

Une solution contenant 28 g du composé obtenu à l'étape b) dans 210 ml de $CH_3CN$ en présence de 41,4 g de $K_2CO_3$ calciné est portée à reflux pendant 1 h 30.
Après addition de 11 g de 2,6-bis-(chlorométhyl)-pyridine, le mélange est chauffé à reflux pendant une nuit.
Le précipité formé est filtré, lavé avec 1 l d'eau, puis séché sous vide ; m = 26,8 g.
HPLC : colonne n° 1, éluant n° 1, débit 1 ml/min, gradient linéaire de : 90/10 à 10/90 en 40 min puis 0/100 (v/v) en 10 min : tr = 32 min.

d) Composé de formule V(2)

A 20 g du composé obtenu à l'étape c) en suspension dans 310 ml de diméthylformamide, on ajoute 16 g de LiOH puis goutte à goutte, en ½ h, 18 g d'acide thioglycolique.
Le milieu réactionnel coloré en rouge est agité pendant 6 h à 25° C puis 400 ml d'$H_2O$ et 400 ml de $CH_2Cl_2$ y sont ajoutés. Après agitation et décantation, la phase aqueuse est séparée et extraite par 400 ml de $CH_2Cl_2$. Cette

phase organique, après lavage deux fois à l'eau, est réunie à la phase précédente et l'ensemble est concentré. Le résidu huileux est purifié par chromatographie flash sur silice (Merck®, 40-63 $\mu$m) en éluant avec un mélange $CH_3OH/NH_4OH$ (50/1) après élimination des impuretés par élution avec $CH_3OH$.

Après évaporation des fractions conformes, on obtient 5,5 g du produit.

HPLC : colonne n° 1, éluant n° 4 : PIC B8 (Waters)/$CH_3CN$, débit 1 ml/min, gradient linéaire de : 90/10 à 10/90 (v/v) en 40 min : tr = 14 min (tr = 32 min pour le produit de départ).

e) Composé de formule V(3) avec x = 2, B = éthyle

A une solution de 8 g de composé obtenu à l'étape d) dans 60 ml de $CH_3CN$ et 26 ml d'éther diisopropylique, on ajoute 22 g de 2-bromoglutarate d'éthyle et 11 g de $K_2CO_3$ calciné puis le mélange est porté à sa température de reflux pendant 24 h.

Après filtration puis évaporation sous vide, l'huile obtenue est purifiée par flash-chromatographie sur silice (Merck® , 40-63 $\mu$m) en éluant avec un mélange heptane/acétate d'éthyle (60/40 v/v).

Après évaporation, on obtient 7,7 g d'huile.

HPLC : colonne n° 1, éluant n° 1, débit 1 ml/min, gradient linéaire de : 98/2 à 10/90 (v/v) en 50 min : tr = 31 et 32 min (2 pics) (produit de départ tr = 4 min).

f) Composé de formule V(4)

5,3 g du composé obtenu à l'étape e) sont dissous dans 32 ml d'acide trifluoroacétique et le mélange est agité durant 1 h 30 à 25° C.

Après concentration sous vide du milieu réactionnel, l'huile obtenue est purifiée par flash-chromatographie sur silice (Merck® , 40-60 $\mu$m) en éluant avec un mélange $CH_2Cl_2/CH_3OH$ (97/3, v/v).

Après élimination du solvant, on obtient 3,8 g du produit solide.

HPLC : colonne n° 1, éluant n° 1, débit 1 ml/min, gradient linéaire de : 98/2 à 10/90 (v/v) en 50 min : tr = 23 min.
RMN $^{13}C$ (125 MHz - DMSOd6 - 30° C)
$\delta$ (ppm) 160,4 ($C_{1,11}$); 119,8 ($C_{12,14}$); 138 ($C_{13}$); 52,9 ($C_{2,10}$); 51,6 ($C_{4,8}$); 45,0-45,3 ($C_{5,7}$); 172,2 (C=O) ; 14,3 ($\underline{C}H_3$-$CH_2$); 59,7-60,3 ($CH_3$-$\underline{C}H_2$-O); 65,4 ($\underline{C}$-N) ; 25,4-25,5 ; 30,4 - 30,5 (C-$\underline{C}H_2$-$\underline{C}H_2$-CO).

g) Réaction avec Y"Br =

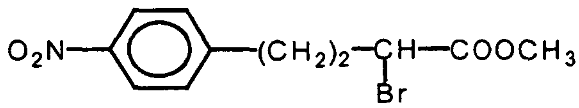

A une suspension de 9,4 g du composé obtenu à l'étape f) dans 30 ml d'éther diisopropylique et 65 ml de $CH_3CN$ en présence de 4,5 g de $K_2CO_3$ calciné, on ajoute 17 g du composé Y"Br.

Après agitation 48 h à 85° C, le milieu réactionnel est filtré et concentré sous vide, l'huile résiduelle est purifiée par chromatographie sur colonne (d = 15 cm)contenant 1 kg de silice (Merck® 40-60 $\mu$m) en éluant avec un mélange $CH_2Cl_2$/acétone 70/30 v/v.

Après concentration à sec, on obtient 5 g de produit.

HPLC : colonne n° 1, éluant n° 1, débit 1 ml/min, gradient linéaire de : 98/20 à 10/90 (v/v) en 50 min : tr = 26 min.

h) Hydrolyse des groupes esters éthyliques

4 g du composé obtenu à l'étape g) sont ajoutés à une solution de 10 ml d'HCl 12N, puis le mélange est agité 48 h à sa température de reflux.

Après filtration, concentration, le résidu est purifié par chromatographie sur gel de silice silanisée (Merck® 0,063-0,20 $\mu$m) en éluant avec un mélange $H_2O/CH_3OH$ pour donner 2,2 g de produit.

HPLC : colonne n° 1, éluant n° 1, débit 1 ml/min, gradient linéaire de : 98/2 à 10/90 (v/v) en 50 min : tr = 16 min (2 pics).

i) Complexation du gadolinium

On introduit dans 40 ml d'$H_2O$, 2,2 g du composé obtenu à l'étape h).

Le pH de la suspension est amené à 5 par ajout d'une solution de NaOH aqueuse 2N puis le milieu est chauffé à 50° C jusqu'à solubilisation complète.

Après addition de 0,6 g de $Gd_2O_3$, la solution toujours maintenue à pH 5 par ajout d'une solution aqueuse de NaOH 2N, est chauffée à 80° C pendant 6 h.

Après filtration des sels par évaporation, le résidu est cristallisé dans $C_2H_5OH$ ; m = 2 g.

HPLC : colonne n° 1, éluant n° 1, débit 1 ml/min, gradient linéaire de : 96/2 à 10/90 (v/v) en 50 min : tr = 17 et 21 min (2 pics).

j) Réduction du groupe nitro

A 2 g du composé obtenu à l'étape i) dissous dans 50 ml d'$H_2O$, on ajoute 0,4 g de catalyseur charbon palladié à 10%, puis le milieu réactionnel est agité à 25° C sous une pression d'hydrogène de $3.10^5$ Pa pendant 6 h. Après élimination du catalyseur par filtration sur filtre Millipore® (0,45 μm et 0,22 μm), la solution est évaporée pour donner 1,8 g de produit.

HPLC : colonne n° 1, éluant n° 1, débit 1 ml/min ; gradient linéaire de : 98/2 à 10/90 (v/v) en 50 min : tr = 10 min.

<u>EXEMPLE 3</u>

Composé de formule VII1 dans laquelle x = 2

a) A une suspension de 70 g de 3,6,9-15-tétraazabicyclo[9.3.1.]pentadéca-1(15),11,13-triène dans 800 ml de $CH_3CN$ en présence de 910 ml de résine échangeuse d'anions sous forme de base forte (Amberlite® IRA458), on ajoute une solution de 102 g de l'ester 2-bromo-4-nitrophényl butyrate de méthyle dans 100 ml de $CH_3CN$.

Après agitation 3 jours à 25° C, filtration de la résine et évaporation, l'huile obtenue est purifiée par chromatographie sur colonne de 5 kg de silice (Merck®, 40-60 μm) en éluant avec un mélange $CH_2Cl_2/CH_3OH$ (70/30 v/v). On obtient 38 g de produit.

HPLC : colonne n° 1, éluant n° 1 mais pH 3, débit 1 ml/min, gradient linéaire de : 98/2 à 10/90 en 50 min : tr = 15 min.

RMN $^{13}C$ (125 MHz, DMSOd6, 30° C)

δ (ppm) : 160,1 ($C_1$) ; 53,8 ($C_{2,4}$) ; 45-45,4-45,7 ($C_{5,7,8}$) ; 51,3 ($C_{10}$) ; 161,6 ($C_{11}$); 119,3 ($C_{12}$); 119,6 ($C_{14}$) ; 137,6 ($C_{13}$); 51,7 (O-<u>C</u>$H_3$); 172,8 (<u>C</u>=O); 65,8 (<u>C</u>-N) ; 31,06-31,45 (<u>C</u>$H_2$-<u>C</u>$H_2$); 149,6-129,6-122 (<u>A</u>r); 145,6 (<u>A</u>r-$NO_2$).

b) Réaction avec Y'''Br =

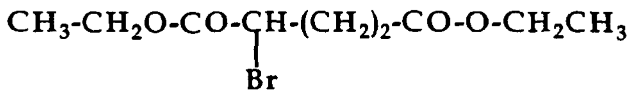

A une solution de 7 g du composé obtenu à l'étape a) dans 70 ml de $CH_3CN$ et 35 ml d'éther diisopropylique, on ajoute 6,8 g de $K_2CO_3$ et 13 g de 2-bromoglutarate d'éthyle puis on laisse sous agitation 24 h à reflux.

Après élimination des sels par filtration, concentration de la solution, l'huile obtenue est purifiée par chromatographie sur silice (Merck® 40-63 μm) en éluant avec un mélange $CH_2Cl_2$/acétone (70/30 v/v).

On obtient 6 g de produit solide.

HPLC : colonne n° 1, éluant n° 1, débit 1 ml/min, gradient linéaire de : 98/2 à 10/90 (v/v) en 50 min : tr = 33 min.

c) Hydrolyse des groupes esters éthyliques

En appliquant le même mode opératoire que pour l'étape h) de l'exemple 2, on obtient 2,8 g à partir de 6 g du composé obtenu à l'étape b).

HPLC : colonne n° 1, éluant n° 1, gradient de : 98/2 à 10/90 (v/v) en 50 min ; débit 1 ml/min : tr = 17 à 19 min (3 pics).

d) Chélate de gadolinium du composé précédent

Dans 35 ml d'une solution à pH 5 de 3,9 g du composé obtenu selon l'étape c), on introduit 2 g de $GdCl_3$, $6H_2O$ et on maintient le mélange à 50° C pendant 5 h au cours desquelles on ajuste le pH si nécessaire en ajoutant une solution de NaOH aqueux (2N).
Le milieu est ensuite filtré puis évaporé ; 4 g de résine échangeuse de cations faiblement acide Chelex® 100 (Bio-Rad) sont ajoutés à l'huile obtenue dissoute dans 40 ml d'eau.
Après agitation 2 h à 25° C, la résine est éliminée par filtration, la solution est évaporée pour donner 4,5 g de produit.
HPLC : colonne n° 1, éluant n° 1, gradient de : 98/2 à 10/90 (v/v) en 50 min, débit 1 ml/min : tr = 15,6 à 18,7 min (plusieurs pics).

e) Réduction du groupe nitro

En appliquant le même mode opératoire que pour l'étape j) de l'exemple 2, on obtient 4 g de produit à partir de 4,5 g du composé obtenu à l'étape d).
HPLC : colonne n° 1, éluant n° 1, gradient linéaire de : 98/2 à 10/90 (v/v) en 50 min, débit 1 ml/min : tr = 8,6 à 9,5 min (plusieurs pics).

**<u>EXEMPLE 4</u>**

Composés de formule V1 où x = 2,
et $-S_1-T'-S_2-$ est, avec $S_1 = S_2 = (CH_2)_2$,

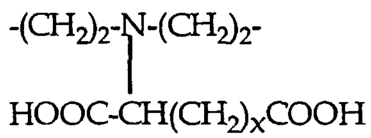

tandis que $Z_1$ est

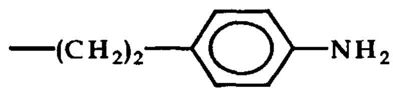

et $Z_2$ est $-(-CH_2)_2-COOH$
a) A une suspension de 20 g de 1,4,7,10-tétraazacyclododécane dans 140 ml de $CH_3CN$, on ajoute goutte à goutte 40,4 g de 2-bromo-4-(4-nitrophényl)butyrate de méthyle en solution dans 50 ml de $CH_3CN$.
Après agitation 24 h à 25° C, la solution est filtrée, lavée par $CH_3CN$, puis par 200 ml d'éther diéthylique.
Après filtration, le produit sous forme de bromhydrate est recristallisé dans 200 ml de $CH_3CN$.
m = 42 g ; F = 170° C.
HPLC : colonne n° 2 : Lichrospher® Merck® $C^{18}$, 100 Å, 5 µm, L = 25 cm, d = 4,6 mm, éluant n° 5 : $KH_2PO_4$ dans $H_2O$ (0,01 M)/$CH_3CN$ (45/55 v/v),débit 1 ml/min: tr = 2,5 min.
b) Réaction avec Y'''Br =

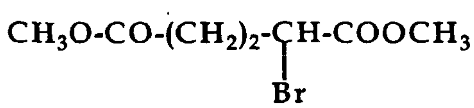

Une suspension contenant 20 g du composé obtenu à l'étape a) et 20 g de $Na_2CO_3$ dans 400 ml de $CH_3CN$ est portée à température de reflux durant 15 min avant de rajouter goutte à goutte 40 g de 2-bromoglutarate de méthyle.
Après agitation 24 h à reflux puis une nuit à 25° C, le milieu est filtré puis le solvant est évaporé et le résidu est dissous dans 100 ml de $CH_2Cl_2$. La phase organique est lavée à l'eau puis séchée sur sulfate de sodium avant l'élimination du solvant par évaporation sous pression réduite. Le résidu est dissous dans un volume minimum de solution

aqueuse d'HCl 1 M. Cette solution est lavée par un même volume d'éther diéthylique puis amenée à pH 4 par $NaHCO_3$ avant d'être extraite par de l'éther diéthylique. Après évaporation de la phase organique, le résidu est purifié par chromatographie sur silice (Merck® Si 60) en éluant avec un mélange heptane/$CH_3COOC_2H_5$ (40/60 v/v puis 30/70 v/v) ; m = 8 g.

HPLC : colonne n° 1 : éluant n° 1 (pH = 2,8), débit 1 ml/min, gradient linéaire de : 90/10 à 60/40 (v/v) en 20 min puis 20/80 en 10 min : tr = 22 à 29 min (3 pics isomères).

c) Hydrolyse des groupes esters de méthyle

10 g du composé obtenu selon l'étape b) sont dissous dans 20 ml d'une solution aqueuse d'HCl 12N et le mélange est porté à reflux pendant 24 h. Après refroidissement, la solution est évaporée et le résidu dissous dans l'eau. Après concentration sous vide, on obtient 7,7 g de produit brut.

d) Complexation du gadolinium avec le composé précédent

La solution de 5 g du produit brut précédent dans 30 ml d'$H_2O$ est amenée à pH 5,2 par addition de NaOH 5M avant d'ajouter 1,2 g de $Gd_2O_3$.

Le milieu est chauffé à 80° C durant 2 h 30 pendant lesquelles le pH est maintenu entre 5,2 et 5,5 par addition d'une solution aqueuse d'HCl 6M.

Après refroidissement à 25° C, le milieu est versé sur 250 ml de $C_2H_5OH$ à 10° C. Le précipité obtenu après lavage à $C_2H_5OH$ est séché ; m = 5 g.

HPLC : colonne n° 1, éluant n° 1 (pH = 2,8), débit = 1 ml/min, gradient linéaire de : 90/10 à 85/15 (v/v) en 15 min, puis 70/30 en 15 min, puis 40/60 en 10 min : tr = 31 à 34 min (3 pics).

e) Réduction du groupe nitro

5 g du complexe de gadolinium sous forme de sel de sodium sont dissous dans 70 ml d'eau et hydrogénés sous pression comme à l'étape j) de l'exemple 2. On obtient 5 g de produit sous forme de sel de sodium.

HPLC : colonne n° 1, éluant n° 1 (pH = 2,8), débit 1 ml/min, gradient de : 98/2 à 85/15 (v/v) en 20 min, puis 70/30 en 20 min : tr = 17 à 21 min (3 pics).

<u>**EXEMPLE 5**</u>

Composés de formule V1 où x = 2, $-S_1$-T'-$S_2$- est, avec $S_1 = S_2 = (CH_2)_2$,

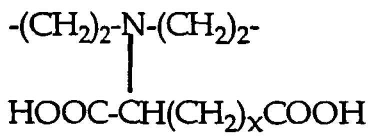

$Z_1$ est $-(-CH_2)_3-NH_2$
$Z_2$ est $-(-CH_2)_2COOH$

a) A une suspension de 20 g de 1,4,7,10-tétraazacyclododécane dans 235 ml de $CH_3CN$ agitée, on ajoute goutte à goutte en 30 min, 49 g de 2-bromo-5-phtalimido pentanoate de benzyle (composé Y'Br) en solution dans 235 ml de $CH_3CN$.

Après une nuit à 25° C, le précipité obtenu est filtré puis lavé avec un minimum de $CH_3CO_2C_2H_5$.
m = 45 g (mélange amine et bromhydrate).
HPLC : colonne n° 1, éluant n° 1 (pH = 2,8) pendant 5 min puis gradient linéaire de : 97/3 à 60/40 v/v en 35 min ; débit 1 ml/min : tr = 26 min.

b) Réaction avec Y'''Br =

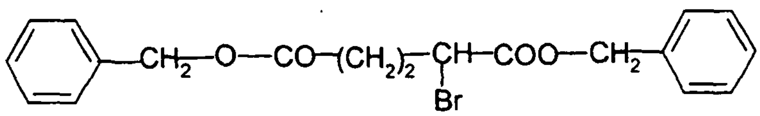

Une solution constituée de 109 g de 2-bromo glutarate de benzyle dans 200 ml de $CH_3CN$ sec est ajoutée rapidement à une suspension de 45 g du composé obtenu à l'étape a) dans 500 ml de $CH_3CN$, préalablement chauffée à 80° C.

35 g de $Na_2CO_3$ sont ajoutés à la fin de l'addition puis le milieu est porté à reflux 48 h.

Après refroidissement, le milieu réactionnel est filtré, le solvant est évaporé sous pression réduite et l'huile obtenue est purifiée par chromatographie sur silice en éluant avec $CH_2Cl_2$ pur puis avec un mélange $CH_2Cl_2/CH_3CO_2C_2H_5$ : (95/5 v/v) ; m = 41 g.

c) Hydrolyse des groupes ester de benzyle et libération de l'amine

Une solution de 5 g du produit brut obtenu à l'étape b) dans 25 ml de solution aqueuse d'HCl 2N est portée à reflux 24 h. Après refroidissement, on ajoute 100 ml d'eau avant d'extraire la phase aqueuse par trois fois 50 ml d'éther diéthylique.

Après évaporation de la phase aqueuse, on obtient 2,5 g de produit qui sont purifiés par filtration de la solution aqueuse sur silice silanisée (Merck® Si60); m = 2,2 g.

HPLC : colonne n° 3 : Hyper Carb® (hypersil) 5 µm 250 Å ; L = 25 cm ; d = 4,6 mm (Waters) ; éluant n° 6 : $H_2SO_4$ dans $H_2O$ (0,1% m/v)/$CH_3CN$, gradient linéaire de : 98/2 à 85/15 (v/v) en 20 min puis 50/50 en 30 min : tr = 21 à 28 mn (plusieurs pics).

d) Complexation du gadolinium

En appliquant le même mode opératoire que pour l'étape d) dans l'exemple 4, on obtient 1,4 g de produit attendu en partant de 2,2 g du composé obtenu à l'étape c) précédente.

HPLC : colonne n° 1, éluant n° 6, 100/0 (v/v) pendant 5 min puis gradient de : 100/0 à 85/5 (v/v) en 25 min puis 50/50 en 20 min ; débit 1 ml/min : tr = 22 à 26 min (plusieurs pics).

**<u>EXEMPLE 6</u>**

Composé de formule A1H dans laquelle A1 est la formule II"' 1, avec x = 2 et R =

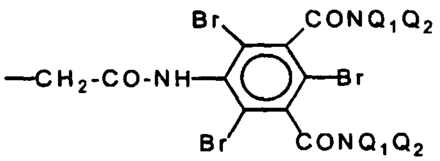

avec $Q_1 = CH_2CHOHCH_2OH$ et $Q_2 = CH_2(CHOH)_4CH_2OH$

a) 16 g du composé obtenu à l'étape e) de l'exemple 1 et 61 g de l'amine $RNH_2$ sont dissous dans 600 ml d'$H_2O$. La solution est amenée à pH 6 par ajout d'une solution aqueuse de NaOH 1N puis on ajoute 20 g de 1-(3-diméthylaminopropyl)-3-éthyl-carbodiimide, chlorhydrate (EDCI) ainsi que 1,1g de l'acide N-hydroxysulfosuccinimide (NHS).

Après 18 h à 25° C durant lesquelles le pH est maintenu à 6 par ajout d'une solution aqueuse de NaOH 1 N ou de HCl 1 N, le solvant est évaporé puis le résidu est précipité dans $C_2H_5OH$.

Le produit obtenu par filtration est ensuite purifié par chromatographie sur colonne de silice silanisée en éluant par $H_2O$ puis par des mélanges $H_2O/CH_3OH$.

Après évaporation des fractions conformes, le residu dissous dans l'eau est traité par 30 ml de résine anionique ($OH^-$). La solution une fois filtrée puis évaporée donne 22 g de cristaux blancs.

HPLC : colonne n° 1, éluant n° 3, gradient linéaire de : 98/2 à 60/40 (v/v) en 50 min ; tr = 18 min.

b) Déprotection du groupe amino

21 g du composé précédent dissous dans 400 ml de $CF_3COOH$ sont agités 3 h à 25° C.

Après évaporation du solvant, le résidu est cristallisé dans l'éther diéthylique. Après filtration, le produit obtenu est ajouté par portions à 10 ml de résine anionique $OH^-$ (Amberlite® IRA67) dans 100 ml d'eau en maintenant toujours le pH de la solution au-dessus de 5 durant l'addition par l'ajout éventuel de résine. A la fin de l'addition, la solution dont le pH est stabilisé autour de 8, est filtrée pour éliminer la résine, puis évaporée. Après séchage, on obtient 20 g de cristaux blancs.

HPLC : colonne n° 1, éluant n° 3, gradient linéaire de : 98/2 à 60/40 (v/v) en 50 min : tr = 7 min (massif) (tr = 19 min pour le produit de départ).

**EXEMPLE 7**

Composé de formule I1 où W1 est

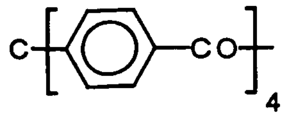

m est 4,

A1 représente le groupe de formule II"' 1 où x = 2 et R =

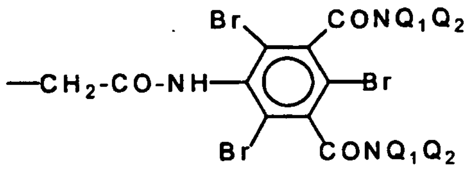

avec $Q_1 = CH_2CHOHCH_2OH$ et $Q_2 = CH_2(CHOH)_4CH_2OH$

Dans une solution de 30 ml de diméthylsulfoxyde contenant 0,5 g d'acide tétrakis-(4-carboxyphényl)méthane et 20 g du composé obtenu selon l'étape b) de l'exemple 6, on ajoute 1,6 g d'EDCI, 1,2 g de 1-hydroxybenzotriazole (HOBT) et 1,7 ml de triéthylamine.

Après agitation 36 h à 25° C, la solution est versée dans 100 ml de $C_2H_5OH$ et le précipité obtenu est séparé par filtration puis redissous dans 40 ml d'eau. La solution est ultrafiltrée sur une membrane en polyéther sulfone (Pall®) avec un seuil de coupure de 5 KD puis sur membrane avec un seuil de coupure de 10 KD. Après évaporation de l'eau, on obtient 10 g de cristaux.

HPLC : colonne n° 1, éluant n° 3, gradient linéaire de : 98/2 à 50/50 (v/v) en 50 min : tr = 13 min.

CES (chromatographie d'exclusion stérique) :

conditions n° 1 : effectuées sur une succession de 4 colonnes (d = 8 mm, l = 30 cm) commercialisées par Shodex® (JP) sous les références OH Pack SB-HQ, contenant du gel de polyhydroxyméthacrylate, dont les limites d'exclusion, déterminées avec du Pullulan® sont successivement : $10^6$ KD (SB-804) ; $10^5$ KD (SB-803) ; $10^4$ KD (SB-802.5) ; $10^4$ KD (SB 802.5) ; éluant : solution aqueuse NaCl (0,16M)/$CH_3CN$ 70/30 v/v, debit 0,8 ml/min. T = 30° C : tr = 36 min.

**EXEMPLE 8**

Composé de formule I1 avec W1 est

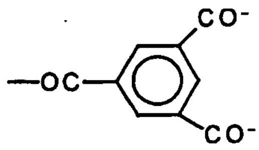

m = 3

A1 représente le même groupe que celui de l'exemple 7.

En appliquant le même mode opératoire que pour l'exemple 7, on obtient à partir de 0,6 g d'acide 1,3,5-benzènetricarboxylique et après élimination de l'excès de $RNH_2$, 26 g de solide blanc.

HPLC : colonne n° 1, éluant n° 3 ; gradient linéaire de : 98/2 à 50/50 (v/v) en 50 min : tr = 12 min.

CES : conditions n° 1 : tr = 37 min.

## EXEMPLE 9

Composé de formule I1 avec W1 est

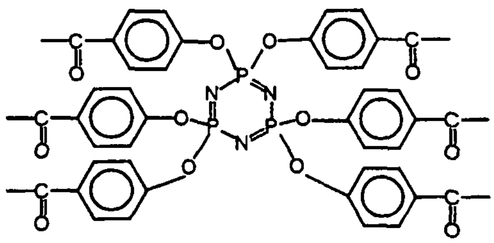

m=6

et A1 représente le même groupe que dans l'exemple 7.

En appliquant le même mode opératoire que pour l'exemple 7, on obtient, après élimination de l'excès de $RNH_2$, 8 g de cristaux à partir de 0,5 g de l'hexaacide dérivé du cyclotriphosphazène de formule :

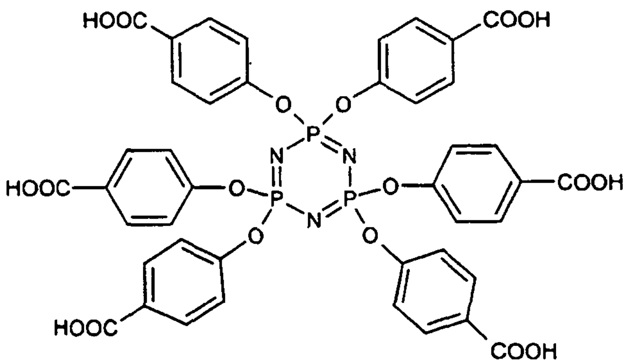

HPLC : colonne n° 1, éluant n° 3, gradient linéaire de : 98/2 à 50/50 (v/v) en 50 min : tr = 14 min.

CES : conditions n° 1 : tr = 35 min.

## EXEMPLE 10

Composés selon la formule I1 avec W1

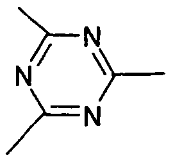

m=3

et A1 représente le groupe de formule II' 1 où x = 2, -GNH- est

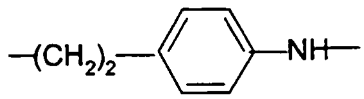

et R est

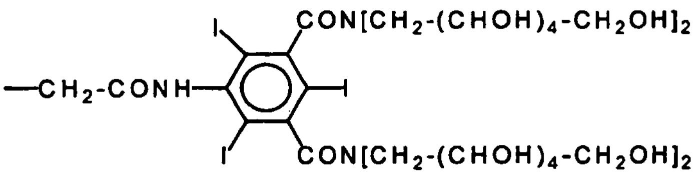

a) Condensation sur le noyau central

A une solution contenant 4 g du composé obtenu selon l'étape e) de l'exemple 4 dans 30 ml d'eau distillée chauffée à 60° C, on ajoute en 10 min une solution de 0,2 g de 2,4,6-trichloro-1,3,5-triazine dans le dioxanne puis le pH est amené à 8,4 par addition d'une solution aqueuse de NaOH 1 N.

Le milieu réactionnel est agité 4 jours durant lesquels le pH est régulièrement amené à 8,4 par addition d'une solution aqueuse de NaOH 0,2M.

Après addition d'un volume d'eau au milieu réactionnel puis ultrafiltration sur une membrane en polyéther sulfone (Pall®) avec un seuil de coupure de 1 KD, le rétentat (40 ml) est concentré jusqu'à un volume de 10 ml puis versé sur une solution aqueuse de HCl 1N. Le précipité formé est isolé. m = 2,5 g.

CES : conditions n° 1 : tr = 40 min.

b) Réaction d'amidification

On introduit, dans une solution à pH 6 contenant 2,5 g du composé obtenu à l'étape précédente a) et 11,5 g de l'amine iodé de formule $RNH_2$ dans 40 ml d'$H_2O$, 2,3 g d'EDCI et 0,2 g de NHS ; le mélange est agité 3 h à 25° C puis la solution est versée dans 200 ml de $C_2H_5OH$ froid.

Le précipité obtenu est redissous dans 100 ml d'eau pour être purifié par ultrafiltration sur une membrane en polyéther sulfone (Pall®) de seuil de coupure de 10 KD.

On isole 9 g de solide blanc par élimination du solvant du rétentat.

CES : conditions n° 1 : tr = 37 min (44 min pour $RNH_2$).

## EXEMPLE 11

Composé selon la formule I1 où W1 est

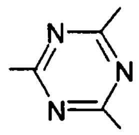

m = 3

et A1 représente le groupe de formule II' où x = 2, et GNH- est

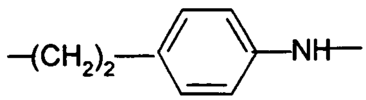

et R est

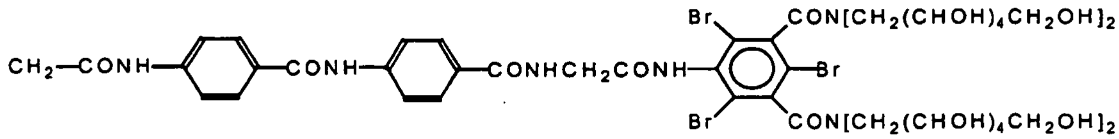

En appliquant les mêmes modes opératoires que pour l'exemple 10 étape b), on obtient, en partant de 2,5 g du composé de l'exemple 10a et de 14 g d'amine $RNH_2$ puis élimination des impuretés de faible masse moléculaire, 10 g de produit blanc.

CES : conditions n° 1 : tr = 36 min (tr = 45 min pour $RNH_2$)

## EXEMPLE 12

Composé selon la formule I1 où W1 est

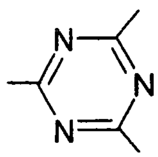

m = 3

et A1 représente le groupe de formule II' où x = 2, -GNH- est

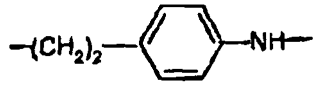

et R est

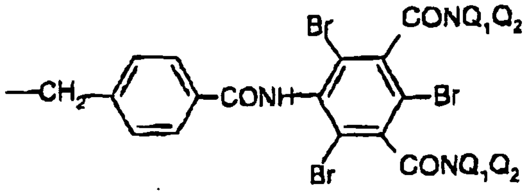

avec $Q_1 = CH_2CHOHCH_2OH$
$Q_2 = CH_2(CHOH)_4CH_2OH$

CES : conditions n° 1 ; tr = 37 min (tr = 45 min pour $RNH_2$).

**Exemple 13**

Composé selon la formule I1 ou W1 est :

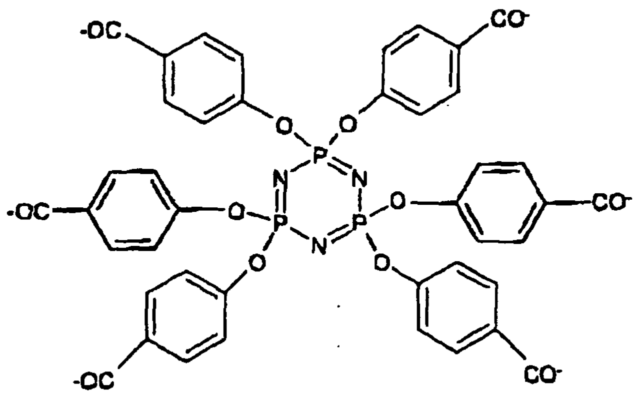

m= 6
et A1 représente la formule II''' 1 où x=2
R est

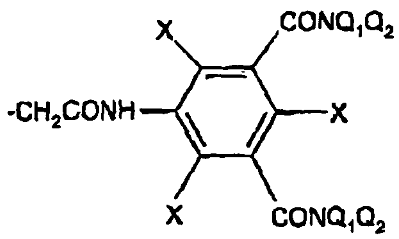

avec $Q_1 = Q_2 = CH_2(CHOH)_4CH_2OH$ et X = Br

a) préparation du chelate intermédiaire de formule VIII

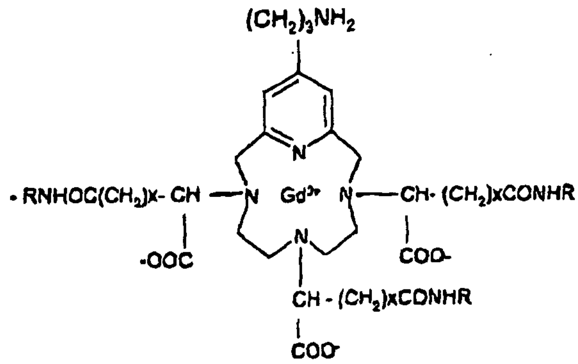

et R représente

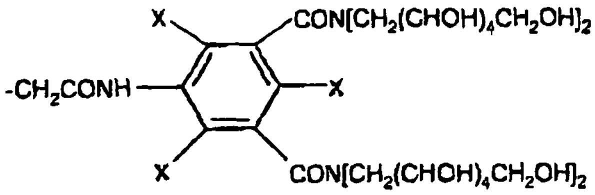

1 - 6g de composé obtenu à l'étape e) de l'exemple 1 et 26,5g de l'amine $RNH_2$ sont dissous dans 200 ml d'eau et on ajoute 7,6g d'EDCI et 0,4g de NHS. Le mélange est maintenu sous agitation vers pH6, pendant 24 heures, avec addition d'une solution aqueuse de NaOH ou HCl N si nécessaire.

Après évaporation du solvant, on fait cristalliser le résidu par addition d'éthanol. Les 35g de cristaux jaunes obtenus sont dissous dans 200 ml d'eau et la solution ultrafiltrée avec une membrane en polyéthersulfone (Pall®) de 1 kD de seuil de coupure.

Le rétentat est concentré et purifié par chromatographie sur colonne de silice silanisée (Merck ®) (diamètre : 7 cm, hauteur : 33 cm) en éluant avec de l'eau puis des mélanges eau/méthanol (90/10 V/V à 80/20). Les fractions contenant le produit cherché sont concentrées jusqu'à élimination des solvants.

Le résidu, dissous dans 50 ml d'eau, est traité par 20 ml de résine anionique sous forme $OH^-$ (HP 661 de chez Rohm et Haas) puis traité avec du noir de carbone à 45°C.

Après filtration et élimination des solvants, on isole 10g de cristaux blancs. HPLC : colonne n° 1, éluant n°7 : $H_2O/CH_3CN$, gradient linéaire 98/2 à 60/40 (V/V) en 50 min., débit 1 ml/min.
tr = 15 min (massif)
CES : conditions n° 1 tr = 40 min
2 - Le solide obtenu ci-dessus est dissous dans 200 ml d'acide trifluoroacétique. Après 3 heures d'agitation à température ambiante, le liquide est éliminé sous pression réduite et le résidu cristallisé par addition d'éther diéthylique. On obtient ainsi 8,8g de cristaux blancs, trifluoroacétate de l'amine de formule VIII.
HPLC : colonne n° 1, éluant n° 7 : gradient linéaire 98/2 à 60/40 (VN) en 50 minutes, débit 1 ml/min.
tr = 76 min (pic large)

b) On introduit dans 55 ml de diméthylsulfoxyde 131 mg de l'hexaacide dérivé du cyclotriphosphazène, déjà mis en oeuvre dans l'exemple 9, avec 4,4g du solide obtenu à l'étape a) et 0,42 mg de triéthylamine, puis 350 mg d'EDCI et 250 mg de HOBT. Après 24 heures d'agitation à température ambiante, la solution est introduite dans 300 ml d'éthanol ; le précipité alors formé est dissous dans 200 ml d'eau et la solution ultrafiltrée sur une membrane en polyéthersulfone de seuil de coupure de 10 kD. Après évaporation du rétentat, on traite le résidu dans 50 ml d'eau successivement par une résine anionique sous forme $OH^-$ puis cationique, avant d'éliminer l'eau.

Les cristaux ainsi isolés sont un mélange du produit attendu, avec celui dans lequel seulement 5 des fonctions acides sont amidifiées. Les 2 produits sont séparés par HPLC préparative.
HPLC : colonne n° 4 : Zorbax 300 SB, C18, 100 Å, 5 $\mu$m ;
L = 25 cm ; d = 4,6 mm (Hewlett Packard ®) ; éluant n° 7 : gradient linéaire 95/5 à 90/10 (VN) en 10 min, et après 5 min 90/10 à 85/15 (VN) en 5 min, puis gradient linéaire jusqu'à 70/30 en 5 min tr = 17 min (pic large)

(et tr=23 min pour le pentaamidifié)
CES : conditions n° 1 tr = 35 min.

Dans les exemples suivants, les conditions CES et HPLC sont les suivantes : CES ( Chromatographie d'exclusion stérique ): effectuées sur une succession de 4 colonnes (d = 8 mm, L = 30 cm ) commercialisées par Shodex® sous les références OH Pack SB-HQ® contenant du gel de polyhydroxyméthacrylate, dont les limites d'exclusion déterminées avec du Pullulan sont successivement : $10^6$ KD (SB-804) ; $10^5$ KD ( SB-803) ; $10^5$ KD ( SB-803) ; $10^4$ KD (SB-802.5) ; éluant : solution aqueuse NaCl (0.16M)/CH3CN 70/30 v/v, débit 0.8 ml/mn T =30°C.
HPLC, Colonnes :

Zorbax® 300SB-CN, 5 μm, L = 25 cm, d = 4,6mm (Interchim®)
Symmetry® C18, 100 Å, 5 μm, L = 25 cm, d = 4,6 mm (Waters®).
Lichrospher® RP-select B, 60 Å, 5 μm, L = 12,5 cm, d = 4 mm (Merck®).
Lichrospher® RP18, 100 Å, 12 μm, L = 25 cm, d = 50 mm (Merck®).
PLRP-S, 300 Å, 7μm, L = 15 cm, d = 4,6 mm ( Polymer Laboratories®).
Hypercarb® 100 % C, 100 Å, 5 μm, L = 25 cm, d = 4,6 mm (Thermo Hypersil-Keystone®)
X-TerraMS® C18, 125 Å, 5 μm, L = 25 cm, d = 4,6 mm (Waters®)

**Exemple 14 (MC 687) :**

Condensation d'un composé de formule

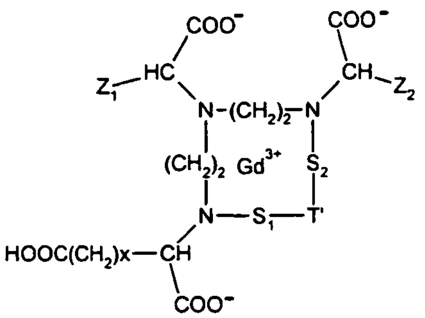

V1

dans laquelle x = 2 et $-S_1-T'-S_2-$ est :

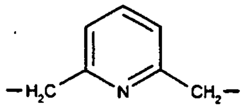

avec $S_1 = S_2 = CH_2$
$Z_2$ est

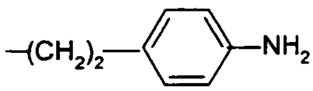

et $Z_1$ est $(CH_2)_2COOH$.

Sur le 2,4,6-trichloro-1,3,5-triazine (chlorure de cyanuril), 0.5 g du composé de l'étape e) de l'exemple 3, sont ajoutés à 2.5 ml d'eau. Le produit est solubilisé en amenant le pH de la solution à 7 par ajout d'une solution de soude (0.1 N). Le milieu réactionnel est chauffé à 60°C et une solution homogène formée par 0.029 g de 2,4,6-trichloro-1,3,5-triazine dans 1 ml de dioxane est additionnée en une seule fois.

L'ensemble est agité 24 heures à température ambiante en maintenant le pH de la solution à 8.4 par ajout d'une solution de soude (0.1N). Le milieu réactionnel est ultrafiltré par centrifugation dans 4 tubes MICROSEP® (1 KD)

de chez FILTRON®. Le retentat est concentré sous vide et séché à 50°C dans une étuve ventilée en présence de $P_2O_5$. 0.33 g sont isolés avec un rendement brut de 84 %.
<u>Spectre de masse :</u>

Mode $ES^-$ m/z = 2468.40 avec z = 1
HPLC :
Colonne Symmetry® C18
eau-TFA pH 3 / $CH_3CN$
tr : massif 15-17 min

**Exemple 15 (MC 611) : (PI 861)**

Composé de formule : II'$_2$ avec x=2
-GNH- est

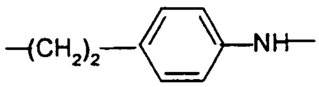

R est

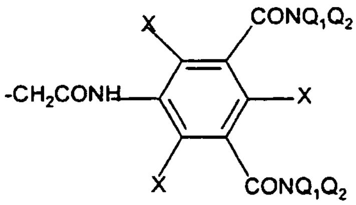

avec $Q_1 = Q_2 = CH_2(CHOH)_4CH_2OH$ et X = Br (branche dite AAG1 AA28 Br) D - H = D' s'écrivant :

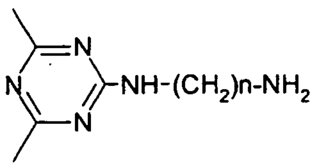

avec n=3
On explicite ici (la démarche sera analogue pour les exemples suivants) que : D du composé II ' 2 s'écrit :

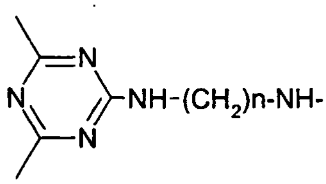

le composé MC611 obtenu comprenant D'=DH
Le composé MC611, dit de type P730 (le coeur Gd est un coeur de type DOTA avec carbone en alpha substitué) avec branche AAG1 AA28 Br a la formule suivante :

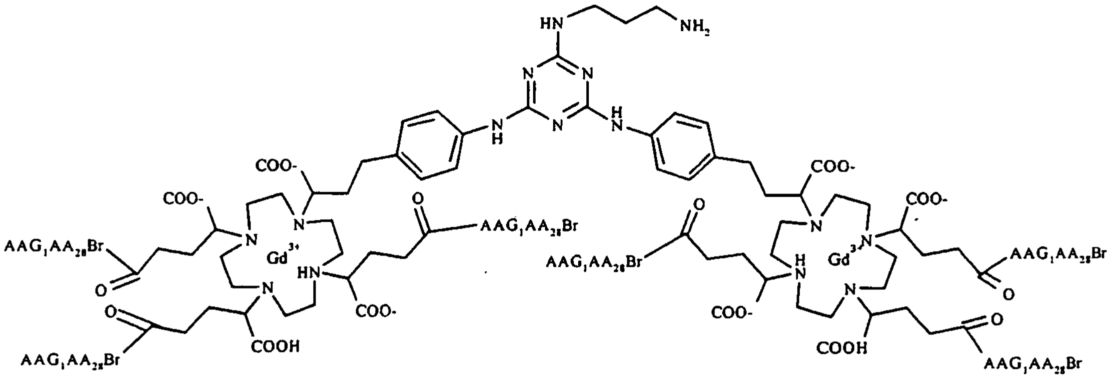

a) condensation sur le cycle triazine

A une solution de 7,6 g du composé de l'étape e) exemple 4 dans 75 ml d'eau en présence de $NaHCO_3$ de pH = 7,7 est ajoutée, sous agitation, une solution de 0,66 g de 2,4,6-trichloro-1,3,5-triazine dans 9 ml de dioxane. Après 6 h d'agitation à température ambiante, le milieu réactionnel est stocké la nuit à 4°C.
Spectre de masse :

Mode $ES^+$ m/z = 950 avec z = 2
HPLC :
Colonne Symmetry® C18
eau-TFA pH 3 / $CH_3CN$
tr : 41 min.

b) couplage de l'amine R-NH2

A température ambiante et sous bonne agitation, on ajoute à la solution de l' étape a), 30,06 g d'amine R-NH2, pH = 6,65. On ajoute 0,2 ml d'HCl 6N pour obtenir un pH = 6,2. 0,47 g de NHS puis 5,8 g d'EDCI sont ensuite ajoutés au milieu réactionnel. Après 3 h d'agitation à température ambiante on ajoute un volume d'eau au milieu réactionnel, puis on ultrafiltre sur une membrane en polyéther sulfone (Pall®) avec un seuil de coupure de 1 KD, le rétentat est évaporé jusqu'à un volume de 100 ml, puis versé sur 1000 ml d'EtOH à froid. Le précipité formé est isolé. Masse obtenue = 29 g.
Spectre de masse :

Mode $ES^-$ m/z = 2141,6 avec z = 4
HPLC :
Colonne Symmetry® C18
eau-TFA pH 2.8 / $CH_3CN$
tr : 22 min.

c) introduction de la diamine

29 g d'Intermédiaire de l'étape b) sont dissous dans 90 ml de diaminopropane à 50°C. Après 1 h d'agitation le milieu réactionnel est versé à froid dans 900 ml d'éthanol. Le précipité formé est isolé, séché sous vide en présence de $P_2O_5$. Le produit obtenu est redissout dans 400 ml d'eau pour être purifié par ultrafiltration sur une membrane en polyéther sulfone (Pall®) de seuil de coupure 1 KD. On isole 22 g de solide blanc par élimination du solvant du rétentat. Le produit est purifié en HPLC préparative : Masse obtenue = 17 g.
HPLC :

Colonne Licrospher® RP18
eau / $CH_3CN$
tr = 25 min (pic large).
Spectre de masse :
Mode $ES^-$ m/z = 8605 avec z = 1

**Exemple 16 (MC 602) :** (PI854)

Composé de formule : $II'_2$ avec x=2
-GNH- est

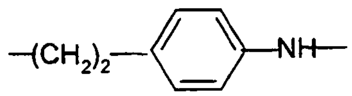

R est

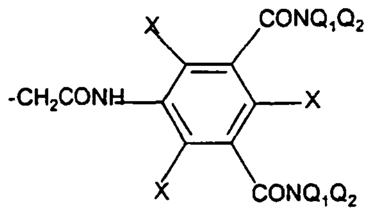

avec $Q_1 = Q_2 = CH_2(CHOH)_4CH_2OH$ et X = Br
D' est D - H

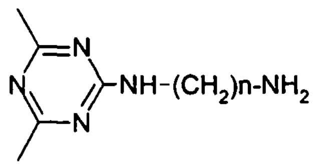

avec n=2

a) introduction de la diamine

8 g de l'Intermédiaire b) de l'exemple 15 sont dissous dans 80 ml d'eau et 0,75 ml de diaminoéthane, à 50°C. Après 3 h 30 d'agitation à 50°C, le milieu réactionnel est versé dans 800 ml d'éthanol. Le précipité obtenu est filtré, lavé à l'éthanol et séché dans un dessiccateur sous vide en présence de $P_20_5$.
Masse obtenue = 8 g.
HPLC :

Colonne Symmetry® C18
eau-TFA pH 2.7 / $CH_3CN$
tr = 17,9 min.
Spectre de masse :
Mode $ES^-$ m/z= 2146,9 avec z = 4

**Exemple 17 (MC 703) :** (PI933)

Composé de formule : $II'_2$ avec x=2
-GNH- est

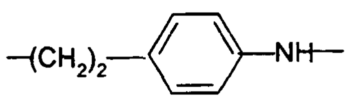

et R est

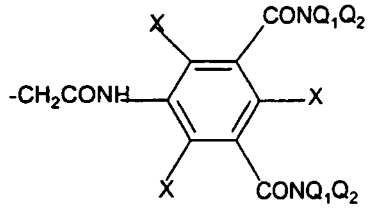

avec $Q_1 = CH_2CHOHCH_2OH$ et $Q_2 = CH_2(CHOH)_4CH_2OH$ et x= Br (branche dite AAG1 AA29 Br)
D' est D-H :

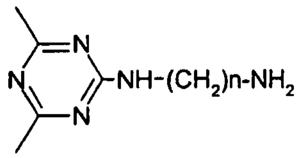

avec n=3

a) couplage de l'amine R-NH2

On introduit, à la solution eau-dioxane contenant 5.3 g du composé obtenue à l'étape a) de l'exemple 15, 20.02 g de l'amine de formule $RNH_2$, 0.367 g de sel de sodium de l'acide (N-hydroxysuccinimidyl)-3-sulfonique (NHS), et 4.54 g de chlorhydrate de 1-(3-dimethyl-aminopropyl)-3-ethyl-carbodiimide (EDCI). Le mélange est agité 4 heures à 25 °C, durant lesquelles le pH est régulièrement amené à 6 par addition d'une solution aqueuse de NaOH ou HCl N. La solution est versée dans 800 ml d'éthanol. Le précipité obtenu est redissout dans 800 ml d'eau pour être purifié par ultrafiltration sur une membrane en polyethersulfone (Pall®) de seuil de coupure 1 KD. On isole 20 g de solide par élimination du solvant du rétentat.
Spectre de masse :

Mode $ES^-$ m/z = 1497.2 avec z = 5
HPLC :
Colonne Symmetry® C18
eau-TFA pH 3 / $CH_3CN$
tr = 26 min

b) introduction de la diamine

Au départ de 19.85 g du composé obtenu à l'étape a) on obtient selon le procédé décrit à l'étape c) de l'exemple 15 et après une purification par HPLC préparative 3.3 g de poudre.
Spectre de masse :

Mode ES- m/z = 1503.9 avec z = 5
HPLC :
Colonne Symmetry® C18
eau-TFA pH 3 / $CH_3CN$
tr = 20 min

**Exemple 18 (MC 712) :** (PI938)

Composé de formule : $II'_2$ avec x=2
-GNH- est

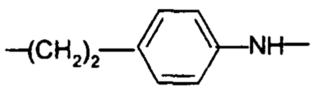

et R est une branche dit flash :

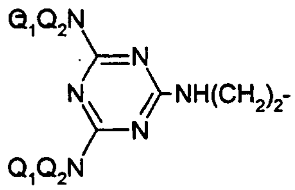

avec $Q_1 = Q_2 = CH_2(CHOH)_4CH_2OH$
D' est D-H :

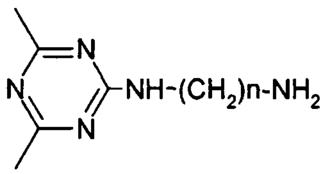

avec n=3

a) Réaction de l'amine $Q_1Q_2NH$ (6-[(2,3,4,5,6-pentahydroxyhexyl)amino]-1,2,3,4,5-hexanepentol) sur le 2,4,6-trichloro-1,3,5-triazine (chlorure de cyanuril)

Dans un ballon de 1 L, 103,65 g (0,3 mol) d'amine $Q_1Q_2NH$ sont introduits dans une suspension de 27,6 g (0,15 mol) de chlorure de cyanuril dans 300 ml d'eau. Le pH est ajusté à 7,5 - 8 avec une solution de soude 5 N. La suspension est agitée pendant une nuit à température ambiante.
HPLC :

Colonne Hypercarb®
eau - $H_2SO_4$ 0.037 N / $CH_3CN$
tr = 27 min.

b) condensation de l'éthylène diamine 200,5 ml d'éthylène diamine (3 mol) sont ensuite additionnées au mélange réactionnel.

L'agitation est maintenue 1 h30, puis le milieu réactionnel est concentré sous vide. 250 ml d'eau sont ajoutés à l'huile obtenue précédemment puis le mélange est à nouveau concentré sous vide éliminant ainsi une partie de l'éthylène diamine en excès. Le produit brut est purifié sur 3 kg de silice silanisée (élution à l'eau). 155.3 g de produit sont ainsi obtenus
H PLC :

Colonne Hypercarb®
eau - $H_2SO_4$ 0.037 N / $CH_3CN$
tr = 20 min .
Spectre de masse :
Mode $ES^+$ m/z = 826.7 avec z = 1.

c) couplage de l'amine R-NH2

En appliquant le même mode opératoire que pour l'exemple 15 étape b), on obtient, en partant de 5.3 g du composé a) de l'exemple 15 et de 17.7 g d'amine $RNH_2$ préparée à l'étape b), puis élimination des impuretés de faible masse moléculaire, 20.4 g de solide.
<u>HPLC :</u>

Colonne Symmetry® C18
eau-TFA pH 3 / $CH_3CN$
tr = 18-20 min.
<u>Spectre de masse :</u>
Mode $ES^-$ m/z = 1348.5 avec z = 5.

d) introduction de la diamine

En appliquant le même mode opératoire que pour le procédé décrit à l'étape c) de l'exemple 15 on obtient, à partir de 19.4 g du composé obtenu à l'étape précédente c) et de 60 ml de diaminopropane, 5 g de solide.
<u>HPLC :</u>

Colonne Symmetry® C18
eau-TFA pH 3 / $CH_3CN$
tr = 21-30 min.
<u>Spectre de masse</u> :
Mode $ES^-$ m/z = 1132.0 avec z = 6.

**<u>Exemple 19 (MC 708) :</u>** <u>(PI942)</u>

Composé de formule : $II'_2$ avec x=2
-GNH- est

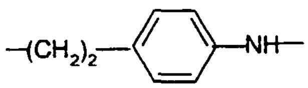

et R est

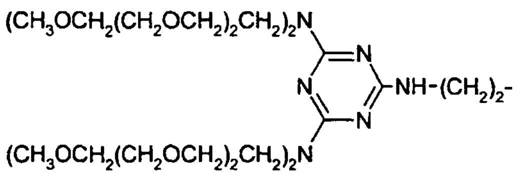

D' est D-H :

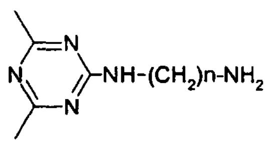

avec n=3

a) tosylation

A une solution de 108 g de triéthylène glycol monométhyléther dans 160 ml de pyridine, refroidie à 0°C par un bain glace-acétone, une solution de 251.6 g de chlorure de tosyle dans 260 ml de pyridine est additionnée goutte à goutte à +5°C. Le mélange obtenu est agité à température ambiante 12 heures, puis versé dans 500 ml d'eau froide et extrait avec 2 fois 500 ml d'éther ethylique. Après concentration de la phase organique 204 g de produit sont obtenus.
CCM :

Eluant : $CH_2Cl_2$ 90%-MeOH 10%- $SiO_2$
Révélateur : UV
Rf : 0,6.

b) condensation sur la benzylamine

Une solution de 199 g de l'intermédiaire obtenu à l'étape précédente a) dissous dans 800 ml d'acétonitrile, est additionnée goutte à goutte et à température ambiante à une solution composée de 31 ml de benzylamine et de 66.3 g (0.624 mole) de $Na_2CO_3$ dissous dans 400 ml de $CH_3CN$. Le milieu est agité à reflux pendant 24 heures. Après filtration de l'insoluble, le milieu est concentré à sec. 111 g de produit sont obtenus.
CCM:

Eluant : $CH_2Cl_2$ 90%-MeOH 10%- $SiO_2$
Révélateur : UV
Rf : 0,8.

c) déprotection de l'amine

70 g (0.175 mole) de produit issus de l'étape b) sont introduits en présence de 470 ml d'éthanol et de 15 g de Pd sur charbon à 10% dans un autoclave de 1 litre. Le milieu est hydrogéné sous 12 bars à 45°C pendant 6 heures. Après filtration et concentration, 50 g de produit sont obtenus.
Spectre de masse :

Mode $ES^+$ m/z = 309.9 avec z = 1
CCM :
Eluant : $CH_2Cl_2$ 90%-MeOH 10%- $SiO_2$
Révélateur : réactif de Dragendorff
Rf : 0,25.

d) condensation sur le 2,4,6-trichloro-1,3,5-triazine

Une solution composée de 50 g de produit obtenu à l'étape c), de 49.5 g de carbonate de potassium, de 14.93 g de chlorure de cyanuril dissous dans 1.5 litre de 1,4-dioxane est agité à 42 °C pendant 48 heures. Après filtration du milieu et concentration, 56 g de produit sont obtenus (rendement 94 %).
Spectre de masse :

Mode $ES^+$ m/z = 730.4 avec z = 1
HPLC :
Colonne Symmetry® C18
eau-TFA pH 3.16 / $CH_3CN$
tr = 37.7 min.

e) condensation de l'amine

Une solution composée de 56 g de composé obtenu à l'étape d), de 25.75 ml d'éthylène diamine, de 21.3 g de carbonate de potassium dans 1.1 litre de 1,4-dioxane, est agitée à 42°C pendant 24 heures. Le produit est purifié par chromatographie sur silice en éluant avec un mélange composé de $CH_2Cl_2$ 95 % / MeOH 5 %. 30 g de produit sont obtenus.
Spectre de masse :

Mode $ES^+$ m/z = 377.4 avec z = 2
HPLC :
Colonne Symmetry® C18
eau-TFA pH 3.20 / $CH_3CN$
tr = 16.95 min.

f) couplage de l'amine R-NH2

Le couplage est réalisé selon le protocole décrit à l'étape b) de l'exemple 15, au départ de 17,5 g de l'amine R-$NH_2$ précédemment préparée dissous dans le minimum d'eau. Le milieu réactionnel est dilué dans 300 ml d'eau pour être ultrafiltré sur une membrane en polyéther sulfone (Pall®) de 1 KD. Le rétentat est évaporé à sec. 15 g d'huile sont obtenus.
Spectre de masse :

Mode $ES^+$ m/z = 6316,15 avec z = 1
HPLC :
Colonne Symmetry® C18
eau-TFA pH 2.7 / $CH_3CN$
tr = 21 min.

g) introduction de la diamine

Au départ de 2,4 g de l'intermédiaire précédemment préparé, selon le protocole décrit à l'étape c) de l'exemple 15 on obtient, après évaporation du solvant et purification de l'huile obtenue par HPLC préparative 3,4 g de produit.
Spectre de masse :

Mode $ES^+$ m/z = 6352,7 avec z = 1
HPLC :
Colonne Symmetry® C18
eau-TFA pH 2.8 / $CH_3CN$
tr = 16.5 min.

**Exemple 20 (MC 680)** : (PI879)

Composé de formule : II'$_2$ avec x=2
-GNH- est -(-$CH_2$)$_3$-NH.

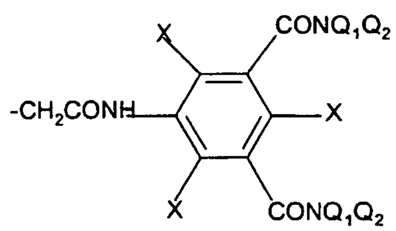

R est avec $Q_1 = Q_2 = CH_2(CHOH)_4CH_2OH$ et X = Br
D'est D-H

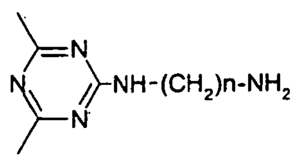

avec n=3

a) condensation sur le cycle triazine

Au départ de 0.2 g d'amine obtenue exemple 5 d) et de 19.3 mg de 2,4,6-trichloro-1,3,5-triazine selon le protocole décrit exemple 15 a), 0.18 g de produit est obtenu.
Spectre de masse :

Mode $ES^-$ m/z = 1777 avec z = 1
HPLC :
Colonne Symmetry® C18
eau-TFA pH 2.8 / $CH_3CN$
tr = 30,31,32 min;

b) couplage de l'amine R-NH2

En appliquant le protocole décrit exemple 15 b), 0.46 g d'amine $R\text{-}NH_2$ (0.41 mmol) et 0.1 g (0.056 mmol) de l'intermédiaire a) préparé précédemment conduisent à 0.25 g de produit.
Spectre de masse :

Mode ES- m/z = 8442 avec z = 1
HPLC :
Colonne Symmetry® C18
eau-TFA pH 2.8 / $CH_3CN$
tr = 16.5 min;

c) introduction de la diamine

En appliquant le protocole de l'exemple 15 c) sur 0.2 g de dérivé précédemment préparé b), 0.1 g de produit est obtenu.
Spectre de masse :

Mode $ES^-$ m/z = 8483 avec z = 1
HPLC :
Colonne Symmetry® C18
eau-TFA pH 2.8 / $CH_3CN$
tr = 14.4 min

**Exemple 21 (MC 691):** (PI932)

Composé de formule : $II''_{a2}$ avec x=2
-GNH- est

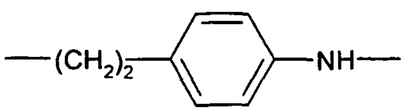

R est

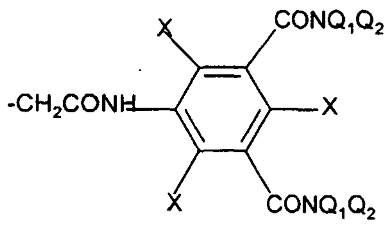

avec $Q_1 = CH_2CHOHCH_2OH$ et $Q_2 = CH_2(CHOH)_4CH_2OH$, X = Br
D' est D-H :

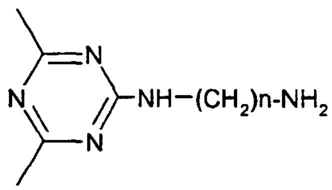

avec n=3

a) condensation sur le cycle triazine

5 g du composé obtenu à l'étape e) de l'exemple 3, sont condensés sur le 2,4,6-trichloro-1,3,5-triazine selon le protocole décrit à l'étape a) de l'exemple 15. Après 3 h de réaction, le pH est ramené à 7 par une solution d'hydrogénocarbonate de sodium. La solution obtenue est conservée une nuit au réfrigérateur.
Spectre de masse :

Mode $ES^-$ m/z = 852.7 avec z = 2
HPLC :
Colonne Symmetry® C18
eau-TFA pH 2.95 / $CH_3CN$
tr = 20-25 min.

b) couplage de l'amine R-NH2

A la solution contenant le composé préparé à l'étape a) sont ajoutés 12.5 g de l'amine $R-NH_2$. Le couplage peptidique est réalisé selon le protocole décrit à l'étape a) de l'exemple 17. 12.61 g de produit sont isolés.
Spectre de masse :

Mode $ES^-$ m/z = 1810.2 avec z = 3
HPLC :
Colonne Symmetry® C18
eau-TFA pH 2.90 / $CH_3CN$
tr = 10-16 min.

c) introduction de la diamine

12 g du produit préparé à l'étape b) sont solubilisés par 90 ml de 1,3-diaminopropane. En utilisant le protocole décrit à l'étape c) de l'exemple 15, 7.5 g de produit sont isolés avec un rendement de 62 %.
Spectre de masse :

Mode $ES^-$ m/z = 1822.6 avec z = 3
HPLC :
Colonne X-TERRA MS®
eau/ $CH_3CN$

tr = 9-13 min.

**Exemple 22 (MC 768) :** (PI955)

Composé de formule : II"$_{a2}$ avec x=2
-GNH- est

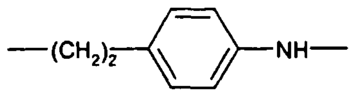

R est

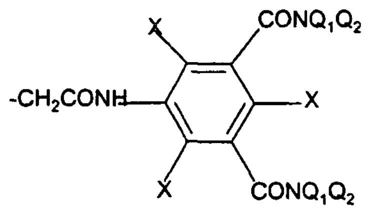

avec $Q_1 = Q_2 = CH_2(CHOH)_4CH_2OH$ et X = Br
D'est D-H

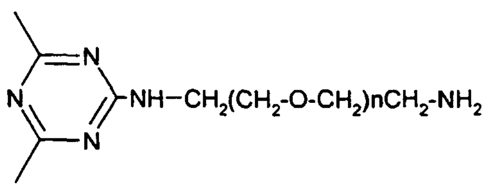

avec n=2

a) couplage de l'amine R-NH2

A la solution eau-dioxane contenant 4.2 g du composé obtenue à l'étape a) de l'exemple 21, sont ajoutés 14 g de l'amine de formule $RNH_2$, 2.68 g de chlorhydrate de 1-(3-dimethyl-aminopropyl)-3-ethyl-carbodiimide (EDCI), et 0.326 g de sel de sodium de l'acide (N-hydroxysuccinimidyl)-3-sulfonique (NHS). Le couplage est réalisé selon le protocole décrit à l'étape b) de l'exemple 15 pour obtenir 18 g de produit.
<u>Spectre de masse :</u>

Mode $ES^-$ m/z = 1024.9 avec z = 6
<u>HPLC :</u>
Colonne Symmetry® C18
eau-TFA pH 2.7 / $CH_3CN$
tr = 13 min.

b) introduction de la diamine

Dans une solution de 70 ml de dimethylsulfoxyde, contenant 18 g du composé obtenu à l'étape a), sont introduits 20 ml de 2-[2-(2-aminoethoxy)ethoxy]ethylamine. Le mélange est agité durant 1 heure à 50°C. Après refroidissement à 25 °C, la solution est versée sur 1000 ml d'éthanol, le précipité formé est dissous dans 400 ml d'eau et la solution ultra filtrée sur une membrane en polyethersulfone (Pall®) de seuil de coupure 1 KD. Après évaporation du rétentat, le

produit obtenu est purifié par HPLC préparative. 1.5 g de solide sont ainsi obtenus.
Spectre de masse :

Mode $ES^-$ m/z = 2087.2 avec z = 3
HPLC :
Colonne Symmetry® C18
eau-TFA pH 2.7 / $CH_3CN$
tr = 11 min.

**Exemple 23 (MC 695):** (PI953)

Composé de formule : II'''$_2$ avec x=2
-GNH- est -(-$CH_2$)$_3$-NH.
R est

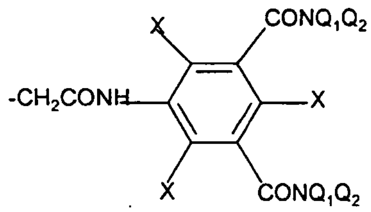

avec $Q_1$ = $CH_2CHOHCH_2OH$ et $Q_2$ = $CH_2(CHOH)_4CH_2OH$, X= Br
D'est D-H

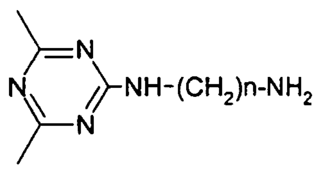

avec n=3

a) condensation sur le cycle triazine

A une solution contenant 8,5 g du composé obtenu selon l'étape b) de l'exemple 6 dans 56 ml d'eau distillée sont rajoutés 0,324 g de carbonate de potassium, et 0,190 g de 2,4,6-trichloro-1,3,5-triazine dans 11 ml de dioxane puis le pH est amené à 8,4 par ajout de $K_2CO_3$. Le milieu réactionnel est agité 18h durant lesquels le pH est amené à 8,4 par ajout de $K_2CO_3$. Après neutralisation par résine cationique sous forme $H^+$, les solvants sont évaporés et le résidu est repris dans l'éthanol absolu. Le précipité est isolé : m=8,3 g
Spectre de masse :

Mode ES- m/z = 2437 avec z = 3
HPLC :
Colonne Symmetry® C18®
Eau / $CH_3CN$
tr 13.3 min.

b) introduction de la diamine

A une solution contenant 8,3 g du composé obtenu à l'étape a) dans 70 ml de diméthylsulfoxide, on ajoute 0,317 g de carbonate de potassium et 1,14 ml de diaminopropane. Le milieu est agité 18h à 42°C sous argon. Le mélange est versé dans 300 ml d'éthanol et le précipité formé est filtré puis lavé par de l'éther éthylique, essoré et séché. Après

purification par HPLC préparative et ultrafiltration sur membrane en polyéthersulfone (Pall®) avec seuil de coupure de 3KD on obtient après évaporation de l'eau 3,9 g de cristaux.
Spectre de masse :

Mode ES- m/z = 1469.8 avec z = 5
HPLC :
Colonne Lichrospher C18®
eau-TFA pH 3.3 / $CH_3CN$
tr = 16.5 min.

**Exemple 24 (MC 766):**

Composé de formule : II'''$_2$ avec x=2
-GNH- est $-(CH_2)_3-NH_-$
R est

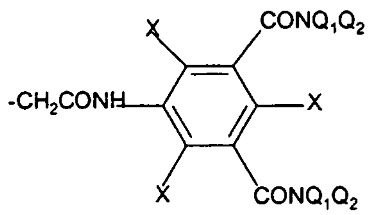

avec $Q_1 = Q_2 = CH_2(CHOH)_4CH_2OH$ et X = Br
D'est D-H

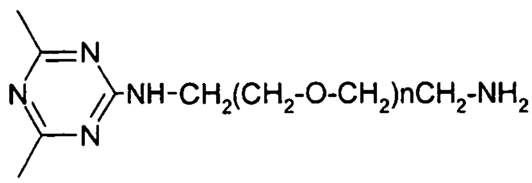

avec n=2

a) condensation sur le cycle triazine

A une solution contenant 4g du composé obtenu selon l'étape a) de l'exemple 13 dans 25 ml d'eau distillée est rajouté 0,132 g de carbonate de potassium. On rajoute une solution de 0,080 g de 2,4,6-trichloro-1,3,5-triazine dans 5,5ml de dioxane puis le pH est amené à 8,4 par ajout de $K_2CO_3$. En suivant le protocole de l'exemple 23 a), 3.7 g de produit sont isolés.
Spectre de masse :

Mode $ES^-$ m/z = 2099 avec z = 4
HPLC :
Colonne Symmetry® C18
eau / $CH_3CN$
tr = 10.8 min.

b) introduction de la diamine

Au départ d'une solution contenant 3,7 g du composé obtenu à l'étape a) dans 30 ml de diméthylsulfoxide, sont rajoutés 0,220 g de carbonate de potassium et 1,3 g de diamine 2-[2-(2-aminoethoxy)ethoxy]ethylamine selon le protocole décrit à l'étape b) de l'exemple 23. Après purification par HPLC préparative, 1g de produit est obtenu.
Spectre de masse :

Mode $ES^-$ m/z = 1700.3 avec z = 5
HPLC :
Colonne Lichrospher C18®
eau- TFA pH 3.3 / $CH_3CN$
tr = 13.8 min.

**Exemple 25 (MC 723) P934 :**

Composé de formule : $I_2$ avec
$W_2$:

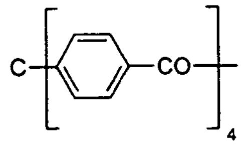

$A_2$ est $II'_2$ avec x=2
G-NH, R et D'=D-H tels que définis exemple 17.
$m_2$=4

Dans une solution de 8 ml de dimethylsulfoxyde contenant 0.041 g d'acide tétrakis-(4-carboxyphenyl)methane et 3.1 g du composé MC703 obtenu à l'étape b) de l'exemple 17, on ajoute à 40 °C, 116 microlitres de triéthylamine, 0.088 g de chlorhydrate de 1-(3-dimethyl-aminopropyl)-3-ethyl-carbodiimide (EDCI) et 0.062 g d'hydrate d' hydroxy-1-benzotriazole. Après agitation 18 heures à 40 °C, la solution est versée dans 80 ml d'éthanol et le précipité obtenu est séparé par filtration puis redissous dans 100 ml d'eau. La solution est ultrafiltrée sur une membrane (Millipore®) de seuil de coupure 10 KD. Après évaporation de l'eau du rétentat, on obtient 2.3 g de produit.
Spectre de masse :

Mode $ES^-$ m/z = 2773.3 avec z = 11
CES:
Colonnes OH Pack SB-HQ ® SB-804, 2x SB-803, SB-802.5
Eau- NaCl 0.16M / $CH_3CN$ 70/30
tr = 34.6 min.

Le composé MC 723 a pour formule :

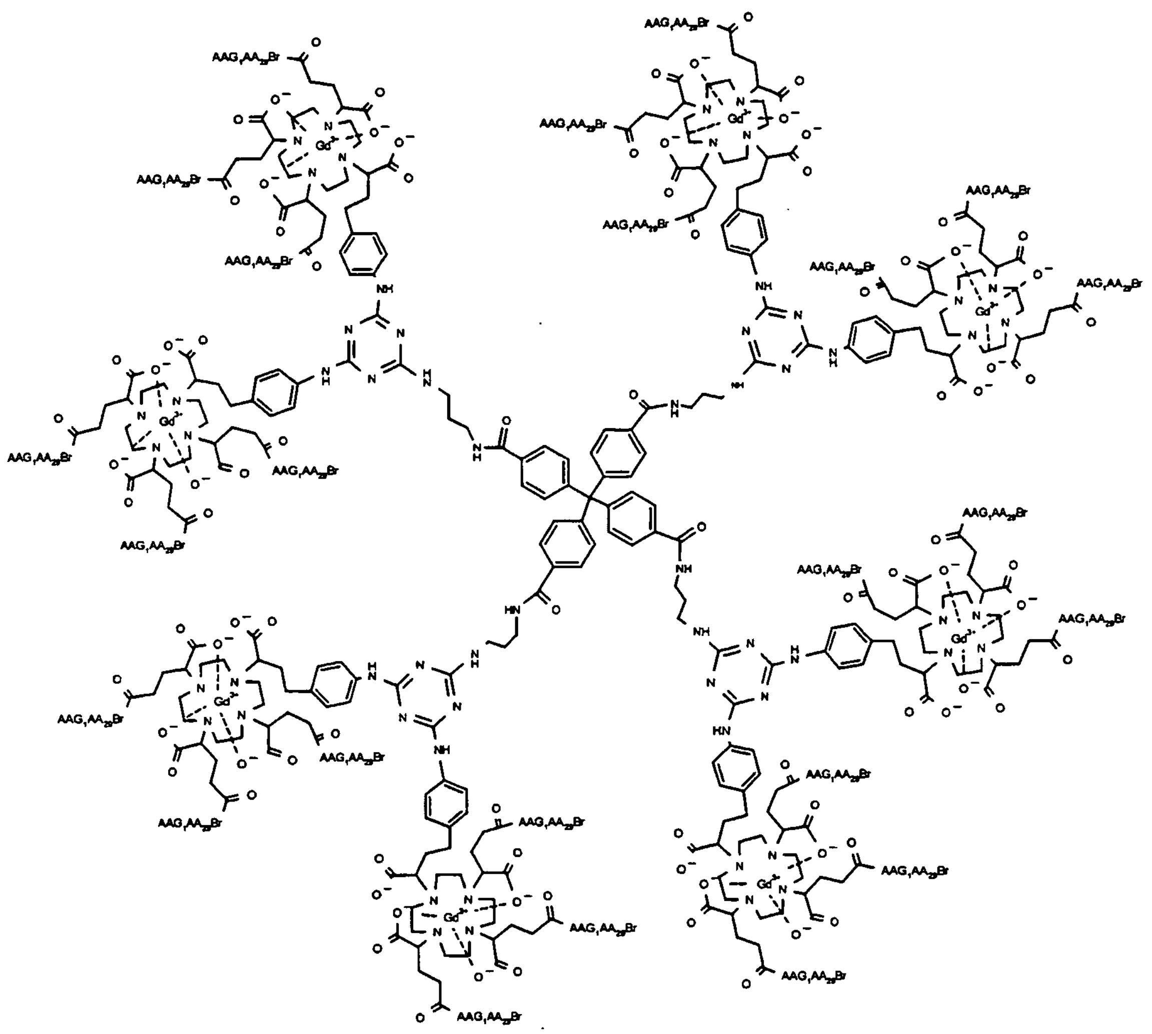

**Exemple 26 (MC 716) P928 :**

Composé de formule : $I_2$ avec
$W_2$:

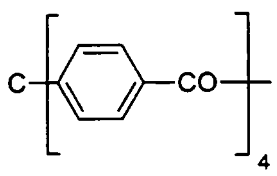

$A_2$ est $II'_2$ avec x=2
G-NH, R et D'=D-H tels que définis exemple 19.
$m_2$=4

2,7 g d'Intermédiaire g) de l'exemple 19 (MC708) et 50 mg d'Intermédiaire tétrakis-(4-carboxyphenyl)methane sont dissous dans 6 ml de DMSO à 80°C. Le couplage est réalisé selon le protocole décrit exemple 25. Après ultrafiltration et évaporation on obtient 1,3 g de gomme ambrée.

Spectre de masse :

Mode $ES^-$ m/z = 25835 avec z = 1
CES :

Colonnes OH Pack SB-HQ ® SB-804, 2x SB-803, SB-802.5
Eau- NaCl 0.16M/ $CH_3CN$ 70/30
tr = 35,25 min.
HPLC:
Colonne Symmetry® C18
eau-TFA pH 2.8 / $CH_3CN$
tr = 37.8 min.

**Exemple 27 (MC 755) P944 :**

Composé de formule : $I_2$ avec
$W_2$:

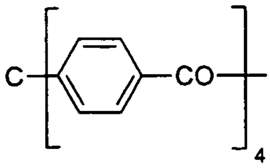

$A_2$ est II'$_2$ avec x=2
G-NH, R et D'=D-H tels que définis exemple 18.
$m_2$=4

En appliquant le même mode opératoire que pour l'exemple 25, on obtient, à partir de 1.87 g du composé obtenu à l'étape d) de l'exemple 18 et de 0.028 g d'acide tétrakis-(4-carboxyphenyl)méthane, 0.938 g de cristaux.
Spectre de masse :

Mode $ES^-$ m/z = 3442.5 avec z = 8
CES :
Colonnes OH Pack SB-HQ ® SB-804, 2x SB-803, SB-802.5
Eau- NaCl 0.16M/ $CH_3CN$ 70/30
tr = 35min.

**Exemple 28 (MC 606) P855 :**

Composé de formule : $I_2$ avec
$W_2$:

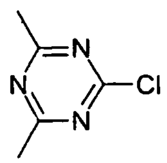

$A_2$ est II'$_2$ avec x=2
G-NH, R et D'=D-H tels que définis exemple 16.
$m_2$=2

Le composé préparé exemple 16 (MC602) est mis en solution dans 1 ml d'eau en présence de 9,2 mg de $NaHCO_3$ pour avoir un pH = 7. Une solution de chlorure de cyanuril est préparée à partir de 0,32 g dissous dans 10 ml de dioxane. On y prélève 100 µl de solution pour l'ajouter au milieu réactionnel à température ambiante. Après une nuit d'agitation le milieu réactionnel est dilué dans 75 ml d'eau, puis ultrafiltré sur une membrane en polyéther sulfone (Pall®) de 10 KD. Après évaporation de l'eau, on obtient 150 mg de cristaux blancs.
Spectre de masse :

Mode $ES^-$ m/z = 17297 avec z =1

HPLC :
Colonne Symmetry® C18
eau- TFA pH 2.8 / $CH_3CN$
tr = 21 min.

**Exemple 29 (MC 607) P856 :**

Composé de formule : $I_2$ avec
$W_2$:

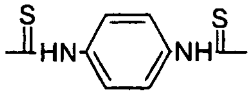

$A_2$ est II'$_2$ avec x=2
G-NH, R et D'=D-H tels que définis exemple 16.
$m_2$=2

Le composé préparé exemple 16 (MC602) est dissous dans 1 ml d'eau en présence de $NaHCO_3$ pour avoir un pH = 6. Une solution de phényl-1,4- diisothiocyanate est préparée à partir de 330 mg de produit dissous dans 10 ml de dioxane. 100 µl de cette solution sont ajoutés au milieu réactionnel à 50°C. Après 18 h d'agitation, le milieu réactionnel est dilué dans 50 ml d'eau puis ultrafiltré sur une membrane en polyéther sulfone (Pall®) de 10 KD.
Après évaporation de l'eau, on obtient 80 mg de cristaux blancs.
Spectre de masse :

Mode ES- m/z = 17387,47 avec z = 1
HPLC :
Colonne Symmetry® C18
eau- TFA pH 2.8 / $CH_3CN$
tr = 21,9 min.

**Exemple 30 (MC 616) P858 :**

Composé de formule : $I_2$ avec $W_2$ :

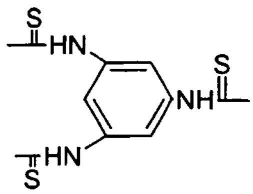

$A_2$ est II'$_2$ avec x=2
G-NH, R et D'=D-H tels que définis exemple 15.
$m_2$=3

a) réduction catalytique

5 g d'amino-3,5-dinitrobenzène sont dissous dans 150 ml de MeOH en présence de 0,5 g de Palladium sur charbon à 5 % sous pression d'hydrogène de 2 Atm. Après 6 h de réaction à température ambiante, le milieu réactionnel est filtré sur papier, évaporé et mis en suspension dans 25 ml d'$Et_2O$. Après filtration, 2,9 g de cristaux noirs sont obtenus.
CCM:

Eluant : $CH_2Cl_2$ 8/MeOH 2 - $SiO_2$
Révélateur : UV
Rf : 0,15;

b) thiophosgénation

0,5 g d'Intermédiaire préparé à l'étape a) est dissous dans 45 ml de $CH_3CN$ et 5 ml de MeOH. A -5°C sont introduits rapidement et sous forte agitation 2 ml de thiophosgène. Après 20 min de réaction à température ambiante, le milieu réactionnel est versé sur 100 ml de $NaH_2PO_4$ 0,5 M et 50 ml d'$Et_2O$. La phase éthérée est séchée sur $MgSO_4$, filtrée et évaporée. L'huile obtenue est purifiée par flash chromatographie sur silice (Merck®, 40-60 μm) en éluant avec heptane/AcOEt (90/10, v/v). Après élimination du solvant, on obtient 0,2 g du produit solide.
<u>CCM :</u>

Eluant : Heptane 8/AcOEt 2 - $SiO_2$
Révélateur : UV
Rf : 0,5
<u>HPLC :</u>
Colonne Symmetry® C18
eau- TFA pH 2.8 / $CH_3CN$
tr = 26 min.

c) condensation sur le noyau central

3 g d'Intermédiaire décrit exemple 15 c) (MC611) sont dissous dans 6 ml d'eau à 50°C. Le pH de la solution obtenue est ajusté à 7,8 avec du $NaHCO_3$. On ajoute une solution de 27,9 mg d'intermédiaire préparé à l'étape c) dans 3 ml de dioxane. Après 5 h de réaction à 50°C, le milieu réactionnel est refroidi, dilué dans 100 ml d'eau, purifié par ultrafiltration sur membrane en polyéthersulfone (Pall®) avec un seuil de coupure de 10 KD.
Après évaporation de l'eau, on obtient 1,8 g de cristaux blancs.
<u>Spectre de masse :</u>

Mode ES- m/z = 26035,6 avec z = 1
<u>CES:</u>
Colonnes OH Pack SB-HQ ® SB-804, 2x SB-803, SB-802.5
Eau- NaCl 0.16M / $CH_3CN$ 70/30
tr = 35min.

**Exemple 31 (MC 651) P870 :**

Composé de formule : $I_2$ avec
$W_2$:

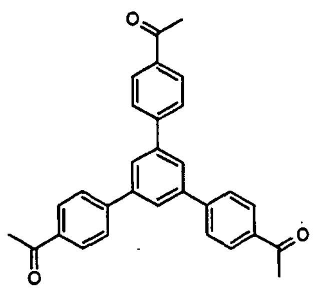

$A_2$ est $II'_2$ avec x=2
G-NH, R et D'=D-H tels que définis exemple 15.
$m_2$=3
47,6 mg d'Intermédiaire tris-1,3,5-(4-carboxyphényl)benzène et 3 g d'Intermédiaire décrit exemple 15 c) (MC611) sont dissous dans 6 ml de DMSO à 80°C. A température ambiante on ajoute 61,4 mg d'HOBT (1-hydroxybenzotriazole) et 87,4 mg d'EDCI (1-(3-diméthyl-aminopropyl)-3-éthyl-carbodiimide chlorhydrate). Après 20 h de réaction à 40°C, le milieu réactionnel est versé dans 90 ml d'éthanol. Le précipité obtenu est filtré, lavé et séché dans un dessiccateur sous vide. Le produit obtenu est redissous dans 100 ml d'eau pour être ultrafiltré sur une membrane en polyéther sulfone (Pall®) avec un seuil de coupure de 10 KD puis de 30 KD. Après évaporation de l'eau, on obtient 1,1 g de cristaux blancs.

Spectre de masse :

Mode ES- m/z = 26199 avec z = 1
CES :
Colonnes OH Pack SB-HQ ® SB-804, 2x SB-803, SB-802.5
Eau- NaCl 0.16M/ $CH_3CN$ 70/30
tr = 35min.

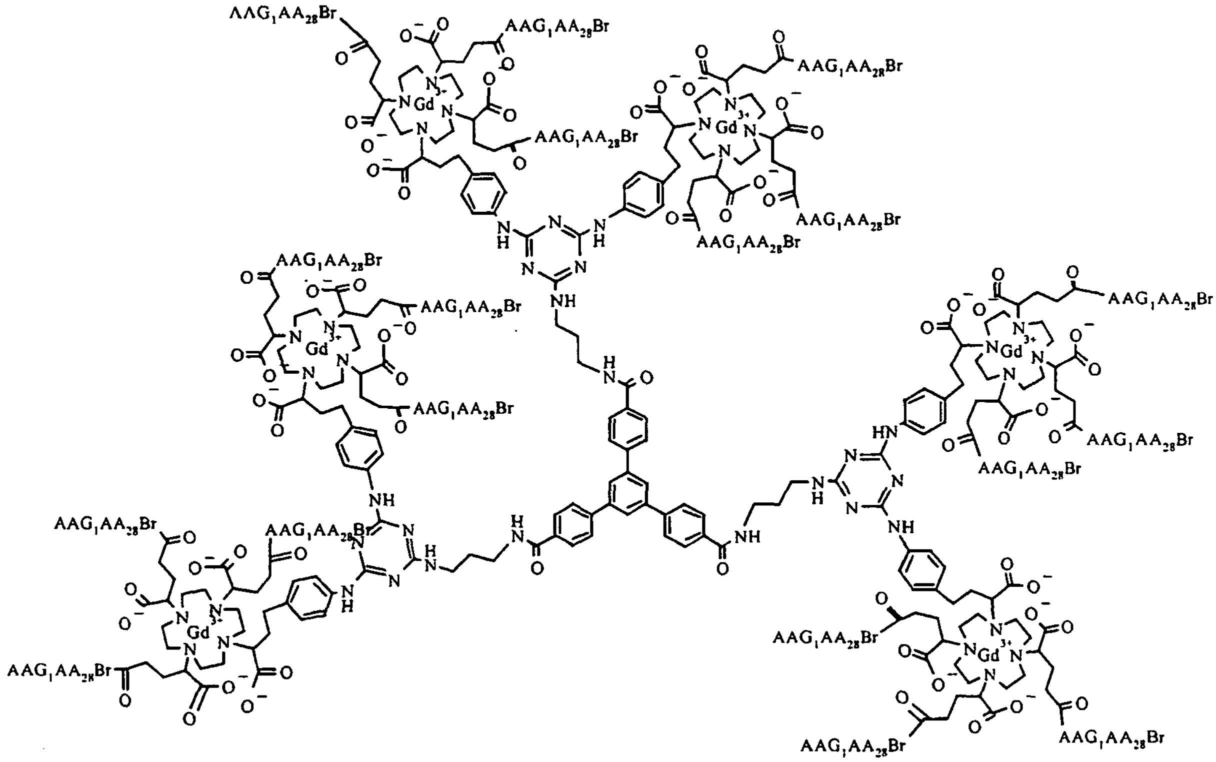

**Exemple 32 (MC 635) P898 :**

Composé de formule : $I_2$ avec
$W_2$:

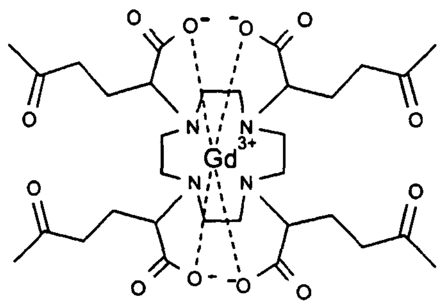

$A_2$ est $II'_2$ avec x=2
G-NH, R et D'=D-H tels que définis exemple 15.
$m_2$=4

26 mg de P730 (acide 2-[4,7,10-tris(1,3-dicarboxypropyl)-1,4,7,10-tetraazacyclo-dodecan-1-yl]pentanedioique) et 1 g d'intermédiaire décrit exemple 15 c) (MC611) sont dissous dans 2 ml d'eau. Le pH de la solution obtenue est ajusté à 6,2 à l'aide d'HCl 0,1N. 4,8 mg de l'acide N-hydroxysulfosuccinimide (NHS) et 29,6 mg d'EDCI (1-(3-diméthylaminopropyl)-3-éthyl-carbodümide, chlorhydrate) sont ajoutés. Après 48 h de réaction à 40°C, le milieu réactionnel est dilué dans 150 ml d'eau et purifié par ultrafiltration sur une membrane en polyéther sulfone (Pall®) avec un seuil de coupure de 10 KD. Après évaporation de l'eau, on obtient 500 mg de cristaux blancs.

Spectre de masse :

Mode $ES^-$ 35201,82 avec z = 1
CES:
Colonnes OH Pack SB-HQ ® SB-804, 2x SB-803, SB-802.5
Eau- NaCl 0.16M/ $CH_3CN$ 70/30
tr = 31 min.

**Exemple 33 (MC 636) P865 :**

Composé de formule : $I_2$ avec
$W_2$:

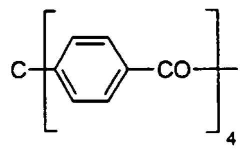

$A_2$ est $II'_2$ avec x=2
G-NH, R et D'=D-H tels que définis exemple 15.
$m_2$=4

4,3 g d'Intermédiaire décrit exemple 15 c) (MC611) sont dissous dans 10 ml de DMSO avec 58,48 mg de tétrakis-(4-carboxyphenyl)méthane, à 80°C. On laisse revenir à température ambiante avant d'ajouter 172 µl de triéthylamine, 89,87 mg de 1-hydroxybenzotriazole (HOBT) et 127,26 mg de 1-(3-diméthylaminopropyl)-3-éthylcarbodiimide chlorhydrate (EDCI). Après 3 h d'agitation à 40°C, le milieu réactionnel est refroidi et dilué dans 100 ml d'eau. La solution est ultrafiltrée sur une membrane en polyéther sulfone (Pall®) avec un seuil de coupure de 10 KD puis de 30 KD. Après évaporation de l'eau, on obtient 1,5 g de cristaux blancs.

Spectre de masse :

Mode ES- 34855,94 avec z = 1
CES :
Colonnes OH Pack SB-HQ ® SB-804, 2x SB-803, SB-802.5
Eau- NaCl 0.16M / $CH_3CN$ 70/30
tr = 34.7min.

**Exemple 34 (MC 647) :**

Composé de formule : $I_2$ avec
$W_2$:

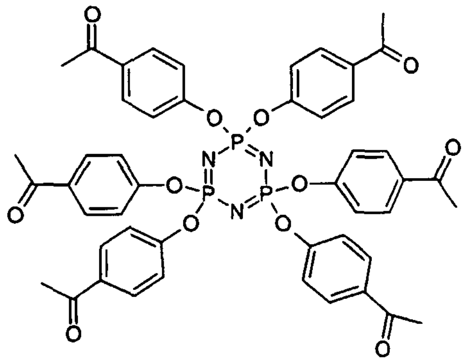

$A_2$ est $II'_2$ avec x=2
G-NH, R et D'=D-H tels que définis exemple 15.
$m_2$ =6

1,0 g d'Intermédiaire décrit exemple 15 c) (MC611) et 17,6 mg de l'acide 4-{[2,4,4,6,6-pentakis(4-carboxyphenoxy)-1,3,5,2λ⁵,4λ⁵,6λ⁵-triazatriphosphinin-2yl]oxy} benzoique sont dissous à 80°C dans 1 ml de DMSO. A température ambiante, on ajoute 40 μl de triéthylamine, 20,9 mg de N-hydroxysuccinimide (NHS) et 29,6 mg d'EDCI (1-(3-diméthylaminopropyl)-3-éthyl-carbodiimide, chlorhydrate). Après 18 h de réaction à 40°C le milieu réactionnel est dilué dans 150 ml d'eau et purifié par ultrafiltration sur une membrane de polyéthersulfone (Pall®) avec un seuil de coupure de 30 KD. Après évaporation de l'eau, 0,7 g de cristaux blancs sont obtenus.

Spectre de masse :

Mode $ES^-$ m/z = 52527 avec z = 1
CES:
Colonnes OH Pack SB-HQ ® SB-804, 2x SB-803, SB-802.5
Eau- NaCl 0.16M / $CH_3CN$ 70/30
tr = 33.4min.

**Exemple 35 (MC 711) P 949 :**

Composé de formule : $I_2$ avec
$W_2$:

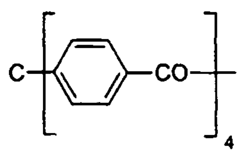

$A_2$ est $II''_{a2}$ avec x=2
G-NH, R et D'=D-H tels que définis exemple 21.
$m_2$ =4
de formule (composé PCTA N fonctionnalisé) :

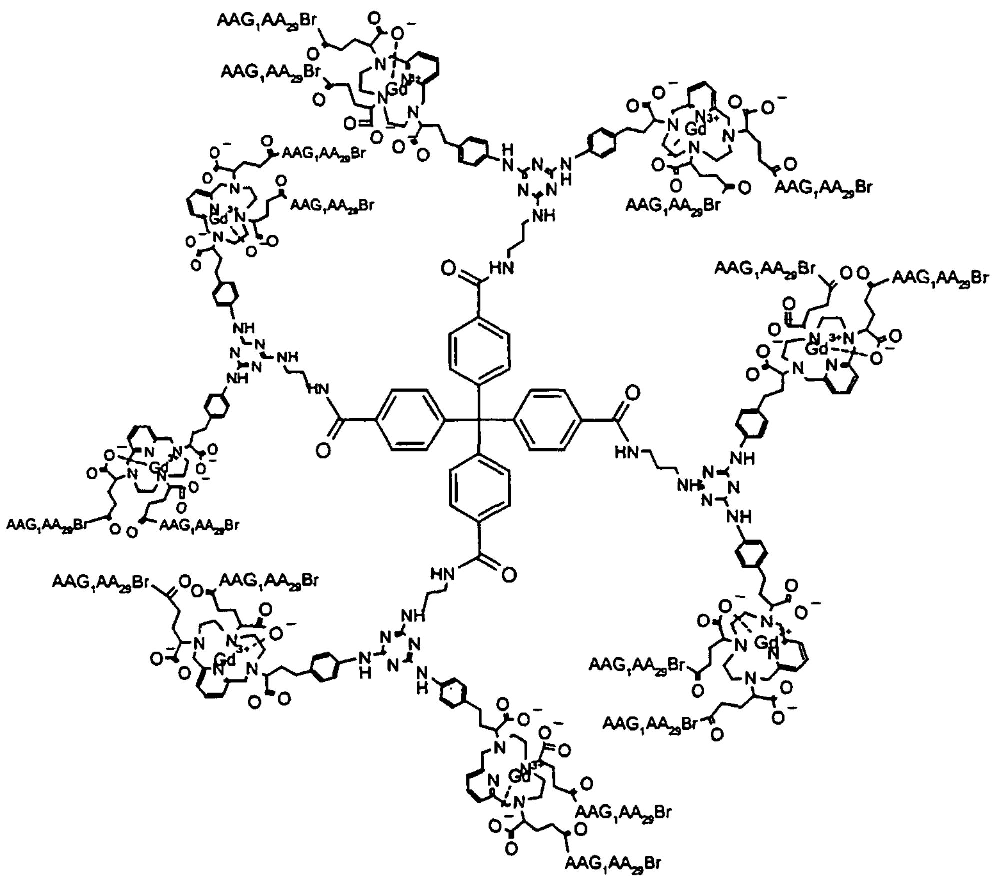

En appliquant le protocole décrit exemple 33 au départ de 0.2 g du composé obtenu exemple 21 c) et 4.1 mg de tétrakis-(4-carboxy-phenyl)méthane, 0.18 g de produit sont isolés.

Spectre de masse :

Mode $ES^-$ m/z = 1858.5 avec z = 12

HPLC :

Colonne PLRP-S®

eau-TFA pH 3.0 / $CH_3CN$

tr = 21-26 min.

**Exemple 36 (MC 718) P 943 :**

Composé de formule: $I_2$ avec

$W_2$:

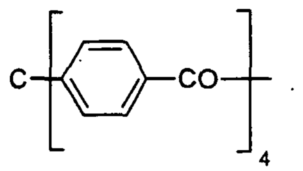

$A_2$ est $II'''_2$ avec x=2

G-NH, R et D'=D-H tels que définis exemple 23.

$m_2$=4

Dans une solution de 14,3 ml de diméthylacétamide contenant 0,0393 g d'acide tétrakis-(4-carboxyphényl) méthane et 2.7 g du composé obtenu exemple 23 b) sont ajoutés 0,121 g d'EDCI et 0,086 g de 1-hydroxybenzotriazole

(HOBT). Après 20 h à 45°C la solution est versée dans 100 ml de $C_2H_5OH$ et le précipité obtenu est séparé par filtration puis redissous dans 40 ml d'eau. La solution est ultrafiltrée sur une membrane en polyéther sulfone (Pall®) avec un seuil de coupure de 10 KD. Après évaporation de l'eau 1,7 g de cristaux sont isolés.
Spectre de masse :

Mode $ES^-$ m/z = 3726 avec z = 8
HPLC :
Colonne ZORBAX 300SB-CN ®
$CH_3COONH_4$ 100 mM / $CH_3CN$
tr = 57 min.

**Exemple 37 (MC 797)**

Composé de formule : $II''_{a2}$ avec x=2
-GNH- est

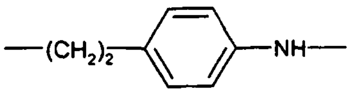

R est

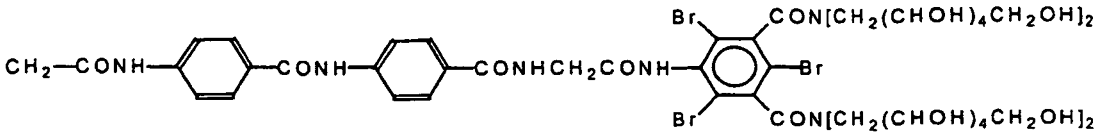

D' est D-H :

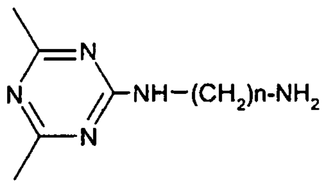

avec n=3

a) couplage de l'amine R-NH2

A la solution eau-dioxane contenant 2.56 g du composé obtenue à l'étape a) de l'exemple 21, sont ajoutés 10.28 g de l'amine de formule $RNH_2$, 1.61 g de chlorhydrate de 1-(3-dimethyl-aminopropyl)-3-ethyl-carbodiimide (EDCI), et 0.196 g de sel de sodium de l'acide (N-hydroxysuccinimidyl)-3-sulfonique (NHS). Le couplage est réalisé selon le protocole décrit à l'étape b) de l'exemple 15 pour obtenir 12 g de produit.
Spectre de masse :

Mode $ES^-$ m/z = 1220,7 avec z = 6
HPLC :
Colonne Symmetry® C18
eau-TFA pH 2.9 / $CH_3CN$
tr = 18,6 min.

b) introduction de la diamine

6 g du produit préparé à l'étape b) sont solubilisés par 27 ml de 1,3-diaminopropane. En utilisant le protocole décrit à l'étape c) de l'exemple15, 5.5 g de produit sont isolés.
Spectre de masse :

Mode $ES^-$ m/z = 1842,6 avec z = 4
HPLC :
Colonne Symmetry® C18
eau-TFA pH 2.9 / $CH_3CN$
tr = 12,8 min

**Exemple 38 (MC 798)**

Composé de formule : $I_2$ avec
$W_2$:

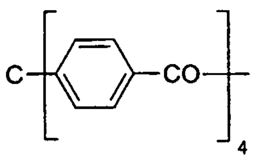

$A_2$ est $II''_{a2}$ avec x=2
G-NH, R et D'=D-H tels que définis exemple 37
$m_2$=4
En appliquant le protocole décrit exemple 33 au départ de 0.5 g du composé obtenu exemple 37 étapeb) et 7.6 mg de tétrakis-(4-carboxy-phenyl)méthane, 0.36 g de produit sont isolés.
Spectre de masse :

Mode ES- m/z = 2491.8 avec z = 12
HPLC :
Colonne PLRP-S ®
eau-TFA pH 3.0 / $CH_3CN$
tr = 23-28 min.

Exemple 39

Composé de formule A1 H dans laquelle A1 est la formule II"' 1, avec x = 2 et R =

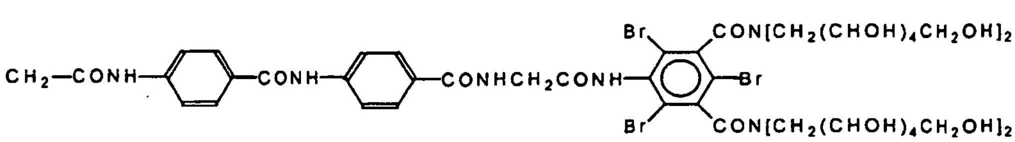

a) couplage de l'amine $RNH_2$

5 g du composé obtenu à l'étape e) de l'exemple 1 et 24,8 g de l'amine $RNH_2$ sont couplés en présence de 6,25 g de 1-(3-diméthylaminopropyl)-3-éthylcarbodiimide, chlorhydrate (EDCI) et de 0,34 g de l'acide N-hydroxysulfosuccinimide (NHS) selon le protocole décrit à l'étape a) de l'exemple 6 pour obtenir 8,5 g de produit.
HPLC :

Colonne Symmetry® C18

eau-TFA pH 2.9 / $CH_3CN$
tr = 21 min

b) Déprotection du groupe amino

8,5 g du composé précédent sont déprotégés en présence de 300 ml de $CF_3COOH$ selon le protocole décrit à l'étape b) de l'exemple 6. Après séchage, on obtient 7,5 g de produit.
HPLC :

Colonne Symmetry® C18
eau-TFA pH 2.9 / $CH_3CN$
tr = 9 min

**Exemple 40 (MC808)**

Composé de formule : II'''$_2$ avec x=2
-GNH- est $-(-CH_2)_3-NH-$
R est

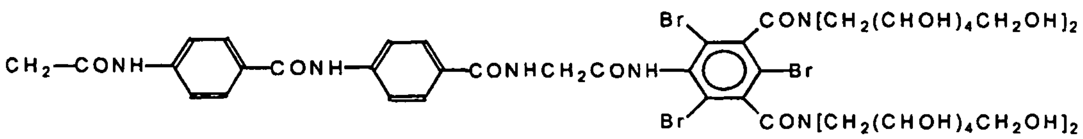

D' est

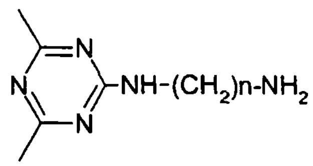

avec n=3

a) condensation sur le cycle triazine

7,5 g du composé obtenu selon l'étape b) de l'exemple 39 sont condensés sur 0,135 g de 2,4,6-trichloro-1,3,5-triazine en présence de 0,233 g de carbonate de potassium selon le protocole décrit dans l'étape a) de l'exemple 23. Après isolement du produit on obtient m = 7 g.
Spectre de masse :

Mode $ES^-$ m/z = 1693,7 avec z = 6
HPLC :
Colonne Symmetry® C18 ®
Eau / $CH_3CN$
tr 15 min (massif)

b) introduction de la diamine

Les 7 g du composé obtenu à l'étape a) sont mis en réaction dans 60 ml de diméthylsulfoxide, en présence de 0,21 g de carbonate de potassium et 0,75 ml de diaminopropane selon le protocole décrit dans l'étape b) de l'exemple 23. On obtient 3,6 g de cristaux.
Spectre de masse :

Mode $ES^-$ m/z = 1700 avec z = 6
HPLC :
Colonne Lichrospher C18®
eau-TFA pH 3.3 / $CH_3CN$
tr = 19 min (massif)

**Exemple 41 (MC809)**

Composé de formule : $I_2$ avec
$W_2$:

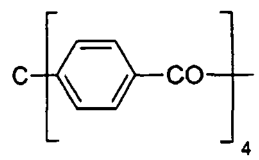

$A_2$ est II'''$_2$ avec x=2
G-NH, R et D'=D-H tels que définis exemple 40
$m_2$ =4

En appliquant le protocole décrit exemple 36 au départ de 1,6 g du composé obtenu exemple 40b) et 17 mg de tétrakis-(4-carboxy-phenyl)méthane, 0.91 g de produit sont isolés.
Spectre de masse :

Mode $ES^-$ m/z = 41247 avec z = 1
HPLC :
Colonne ZORBAX 300SB-CN ®
$CH_3COONH_4$ 100 mM / $CH_3CN$
tr = 59 min (pic large)

**Exemple 42 (MC 802)**

Composé de formule : II'$_2$ avec x=2
-GNH- est

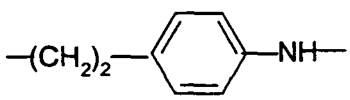

et R est

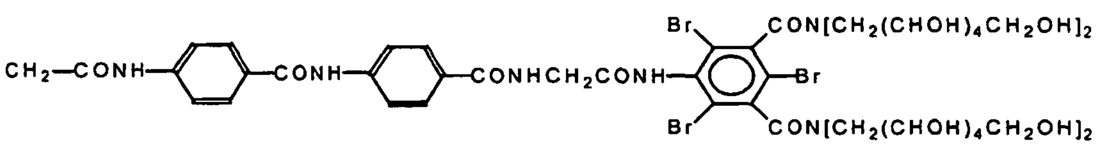

D' est D-H :

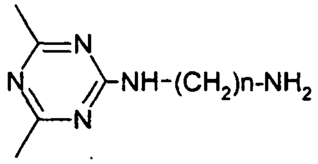

avec n=3

a) couplage de l'amine R-NH2

On introduit, à la solution eau-dioxane contenant 2.9 g du composé obtenue à l'étape a) de l'exemple 15, 15.7 g de l'amine de formule $RNH_2$, 0.2 g de sel de sodium de l'acide (N-hydroxysuccinimidyl)-3-sulfonique (NHS), et 2,45 g de chlorhydrate de 1-(3-dimethyl-aminopropyl)-3-ethyl-carbodiimide (EDCI). Le couplage est réalisé selon le protocole décrit à l'étape b) de l'exemple 15 pour obtenir 13,15 g de produit.
Spectre de masse :

Mode ES- m/z = 1722.4 avec z = 6
HPLC :
Colonne Symmetry® C18
eau-TFA pH 2.8 / $CH_3CN$
tr = 24 min (massif)

b) introduction de la diamine

Au départ de 13.15 g du composé obtenu à l'étape a) on obtient selon le procédé décrit à l'étape c) de l'exemple 15 et après une purification par HPLC préparative 8.65 g de poudre.
Spectre de masse :

Mode $ES^-$ m/z = 1728.7 avec z = 6
HPLC :
Colonne Licrospher® RP18
Eau/$CH_3CN$
tr = 27 min (pic large)

**Exemple 43 (MC 803)**

Composé de formule : $I_2$ avec
$W_2$:

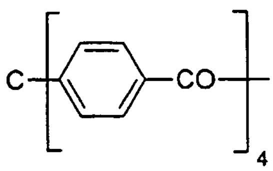

$A_2$ est $II'_2$ avec x=2
G-NH, R et D'=D-H tels que définis exemple 42
$m_2$=4
En appliquant le protocole décrit exemple 33 au départ de 1,5g du composé obtenu exemple 42b) et 17 mg de tétrakis-(4-carboxy-phenyl)méthane, 0.52 g de produit sont isolés.
Spectre de masse :

Mode $ES^-$ 41950 avec z = 1
CES :

Colonnes OH Pack SB-HQ ® SB-804, 2x SB-803, SB-802.5
Eau- NaCl 0.16M / $CH_3CN$ 70/30
tr = 38min (pic large)

On décrit maintenant des résultats de relaxivité et des données biologiques illustrant l'efficacité des composés selon l'invention.

Les tableaux suivants présentent des exemples de résultats de relaxivité et d'efficacité massique obtenus, démontrant l'efficacité massique em nettement supérieure des composés polymétalliques de dérivés RR (qu'il s'agisse de polymétalliques de monomères ou polymétalliques de dimères), par rapport aux monomères correspondants : de l'ordre de 80 à 100% pour la famille de composés de type P730, et de l'ordre de 30 à 60 % pour la famille des composés de type PCTA C fonctionnalisés. La demanderesse a ainsi vérifié la validité des résultats en comparant les polymétalliques avec des monométalliques de structure de branche identique ou quasi identiques.

Par exemple, pour les polymétalliques de monomères de la famille des P730, l'em au champ de 0.47 T (20 MHz) du composé P799 est de 7.3, à comparer à em=4.7 pour le composé monomère correspondant P761

Par exemple, pour les polymétalliques de dimères de la famille des P730, l'em (0.47 T) du composé P855 est de 8.1, à comparer à em=4.67 pour le composé monomère correspondant P760.

Tableau 3 : Efficacité des composés polymétalliques de dimères (au champ de 0.47 T, 20 MHz)

| composé monométallique de référence | composé de polymétallique dimères testé | numéro d'exemple | Famille | poids moléculaire | Nombre de Gd | relaxivité r1 par Gd ($mM^{-1}.Gd.s^{-1}$) | r1/ mole ($mM^{-1}$ $s^{-1}$) | Efficacité massique molaire em ($g^{-1}$ $s^{-1}$) | Taux de Gd (%) | Branche |
|---|---|---|---|---|---|---|---|---|---|---|
| | P 855 | 15 | P730 | 17292 | 4 | 35.1 | 140 | 8.1 | 3.64 | AAG1AA28 Br |
| | P 856 | 16 | P730 | 17381 | 4 | 34.8 | 139 | 8 | 3.62 | AAG1AA28 Br |
| | P 858 | 17 | P730 | 26068 | 6 | 39.9 | 239 | 9.2 | 3.62 | AAG1AA28 Br |
| | P 865 | 18 | P730 | 34850 | 8 | 37.9 | 303 | 8.7 | 3.61 | AAG1AA28 Br |
| | P 869 | 19 | P730 | 17290 | 4 | 33.6 | 134 | 7.8 | 3.64 | AAG1AA28Br |
| | P 870 | 20 | P730 | 26203 | 6 | 38.7 | 232 | 8.9 | 3.6 | AAG1AA28 Br |
| | P 876 | 21 | P730 | 11821 | 3 | 31 | 93 | 7.9 | 3.99 | AAG1AA28 Br |
| | P 877 | 22 | P730 | 15402 | 3 | 40.8 | 122 | 7.9 | 3.06 | AAG1AA28 Br |
| | P 928 | 23 | P730 | 25835 | 8 | 25.7 | 206 | 8 | 4.87 | AAG1AA28Br |
| | P 934 | 24 | P730 | 30534 | 8 | 32.6 | 261 | 8.5 | 4.12 | AAG1AA28 Br |
| P760 | | | P730 | 5292 | 1 | 24.7 | 24.7 | 4.67 | | AAG1AA28 Br |
| | P 867 | 25 | PCTA-C | 14833 | 4 | 44.3 | 177 | 11.9 | 4.24 | |
| | P 874 | 26 | PCTA-C | 10962 | 3 | 42.2 | 127 | 11.5 | 4.3 | |
| | P 878 | 27 | PCTA-C | 22462 | 6 | 48.9 | 293 | 13.1 | 4.2 | |
| | P 943 | 28 | PCTA-C | 29838 | 8 | 45.5 | 364 | 12.2 | 4.22 | |
| | P 949 | 29 | PCTA-N | 22306 | 8 | 37.3 | 298 | 13.4 | 5.64 | |
| P846 | | | PCTA | 3545 | 1 | 27.9 | 27.9 | 7.9 | 4.44 | |

On précise

Formule du P846

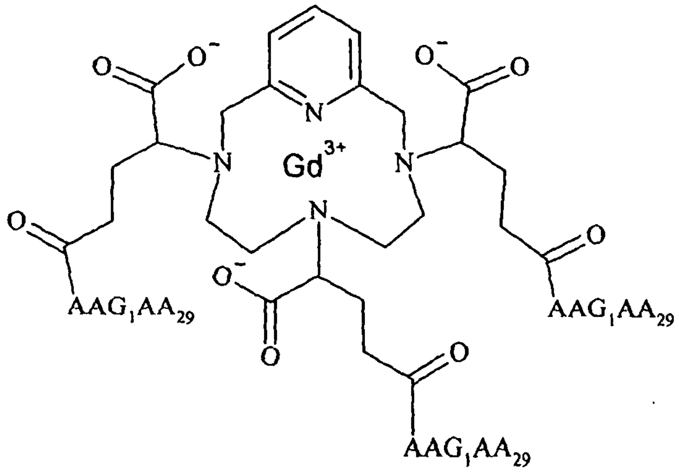

Formule du P760

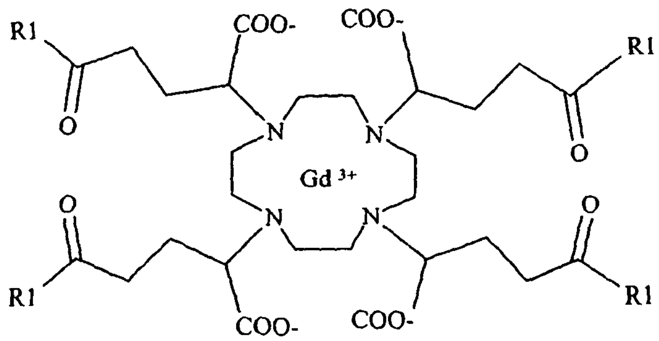

with R1 = AAG1AA28

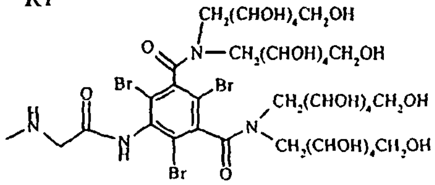

Tableau 4 : Efficacité des composés polymétalliques de monomères

| **Composé monométallique de référence** | Composé **polymétallique de monomères testé** | **Numéro d'exemple** | **Famille** | **Poids moléculaire** | **Nombre de Gd** | **Relaxivité r1 par Gd** | **r1/ mole** | Efficacité **massique molaire em r1** | **Taux de Gd** | **Branche** |
|---|---|---|---|---|---|---|---|---|---|---|
| | **P 799** | *10* | P730 | 14027 | 3 | 34.1 | 102.3 | 7.3 | 3.36 | AAG1 AA28 I |
| **P 761** | | | P730 | 5856 | 1 | 27.6 | 27.6 | 4.7 | 2.69 | AAG1 AA28 I |
| | | | | | | | | | | |
| | **P 876** | *12* | P730 | 11821 | 3 | 31 | 93.0 | 7.9 | 3.99 | monoarom_ G1AA 29Br |
| **P 796** | | | P730 | 5996.7 | 1 | 23.3 | 23.3 | 3.9 | 2.62 | monoarom_ G1AA 28Br |
| | | | | | | | | | | |
| | **P 877** | *Ex11* | P730 | 15402 | 3 | 40.8 | 122.4 | 7.9 | 3.06 | Bisarom_ G1AA28 Br |
| **P 792** | | | P730 | 6473.2 | 1 | 42 | 42.0 | 6.5 | 2.43 | Bisarom_ G1AA28 Br |
| | | | | | | | | | | |
| | **P 874** | *Ex 8* | PCTA C | 10962 | 3 | 42.2 | 126.6 | 11.5 | 4.30 | AAG1 AA29 Br |
| | **P 867** | *Ex 7* | PCTA C | 14833 | 4 | 44.3 | 177.2 | 11.9 | 4.24 | AAG1 AA29 Br |
| | **P 878** | *Ex 9* | PCTA C | 22462 | 6 | 48.9 | 293.4 | 13.1 | 4.20 | AAG1 AA29 Br |
| **P 846** | | | PCTA | 3545 | 1 | 27.9 | 27.9 | 7.9 | 4.44 | AAG1 AA29 Br |

Les données ont été mesurées à une fréquence de 20 MHz qui constitue une fréquence courante pour les indications diagnostiques concernées. Des résultats favorables ont été également obtenues à des fréquences supérieures, par exemple 40 MHz.

La demanderesse a ainsi réussi à obtenir des oligomères

- relaxant à plus de 75 mM-1 s-1, à partir de composés (monomères ou dimères) de type RR qui relaxent individuellement à plus de 25 mM-1 s-1 Gd-1
- et de fraction massique en Gd inférieure de l'ordre de 4 à 6 %, c'est à dire de l'ordre d'au moins trois fois inférieure à celle de composés de type dendrimères connus.
- des oligomères
- relaxant à plus de 75 mM-1 s-1, à partir de composés relaxant individuellement à plus de 25 mM-1 s-1 Gd-1
- dont la charge est modulable, et la couverture externe est modulable (hydrophilie)

On décrit maintenant des preuves expérimentales de l'utilisation diagnostique très avantageuse de différents polymétalliques de dimères selon l'invention.

Les inventeurs ont démontré des propriétés très avantageuses de BPA notamment chez le rat et chez l'homme. Avec un poids moléculaire élevé, les composés obtenus s'extravasent très peu. Les composés présentent un très avantageux profil pharmacocinétique de type BPA :

- une forte rémanence vasculaire (fraction circulante à 5 minutes C5 / C0 supérieure à 30%, et typiquement de 40 à 60%), deux à trois fois supérieure à celle de RC BPA décrits par exemple dans le brevet EP 922 700, persistant sur des délais tardifs ce qui est un atout lors de l'examen IRM (C15 / C0 = 20 à 35%) ; sachant que la concentration C0 représente le rapport entre la dose injectée et le volume plasmatique théorique de 25 ml/kg chez le rat ; ce qui confirme le comportement de type BPA souhaité ;
- associée à une très faible extravasation tissulaire (volume de distribution à l'équilibre $Vd_{ss}$ = 30 à 40 ml/kg), ce qui traduit un confinement vasculaire, et pour certains également à une clairance plasmatique nettement diminuée (1.2 à 1.8 ml/min/kg, à comparer à 4 à 6 pour les BPA de l'art antérieur), ce qui traduit un comportement de type SCBPA.

Les composés selon l'invention sont très utiles en imagerie diagnostique, notamment dans la caractérisations de tumeurs. Des essais concluants ont été obtenus par exemple sur des tumeurs mammaires humaines et chez le rat.

La différenciation des tumeurs bénignes et malignes a été nettement améliorée par exemple grâce à des composés exemplifiés ci-dessus qui présentent un profil pharmacocinétique de SCBPA.

Chez le rat, les inventeurs ont obtenu notamment les résultats suivants. Les témoins ont été le Dota-dysprosium, et le composé P792 (décrit dans le brevet délivré EP 922 700) qui est un produit monométallique à base de Gd dont le profil pharmacocinétique est celui d'un RCBPA. Les mesures seront réalisées 30 et 60 minutes post-injection.

Des doses d'agent cancérigène ENU de 45 mg/kg, pour induire majoritairement des fibroadénomes (tumeurs bénignes) et de 180 mg/kg, pour induire majoritairement des adénocarcinomes (tumeurs malignes) ont été testées.Les concentrations de produit de contraste mesurées dans les adénocarcinomes sont nettement supérieures à celles enregistrées au niveau du muscle (rehaussement tumeur / muscle) et à celles obtenues dans les fibroadénomes (rehaussement tumeur maligne / tumeur bénigne). On retrouve une accumulation prépondérante de polymétallique SCBPA (0.5 à 0.6 %ID/g) par rapport au Dota-Dy (0.15 %ID/g). De plus, pour le Dota-Dy, l'on considère les fibroadénomes ou les adénocarcinomes, le ratio de concentrations tumeur / muscle est voisin de 4. Dans le cas de polymétalliques selon l'invention exemplifiés ci-dessus, les rapports sont différents : le ratio est de 4 à 5 pour les fibroadénomes contre 8 à 9 pour les adénocarcinomes, ce qui permet de différencier les tumeurs bénignes et malignes, 1 heure après injection. Pour comparaison, Daldrup-Link et collaborateurs ont montré que le Gadomer-17 ne permet pas une telle différenciation en IRM dans ce modèle [Daldrup-Link H.E. et coll. Comparison of Gadomer-17 and gadopentetate dimeglumine for differenciation of benign from malignant breast tumors with MR imaging. Acad. Radilo. 2000. 7 : 934-944.]. Cette inefficacité du Gadomer-17 s'explique vraisemblablement par ses caractéristiques pharmacocinétiques : en effet, ce produit présente un rapport $C_5/C_0$ de 21 % (au lieu de 60% pour des polymétalliques obtenus par les inventeurs) une demi-vie plasmatique de 37 min (au lieu de 20 min pour des polymétalliques obtenus par les inventeurs), un volume de distribution de 130 ml/kg (au lieu de 40 ml/kg pour des polymétalliques obtenus par les inventeurs) et une clairance plasmatique totale de 8.1 ml/min/kg (*au lieu* de 1.5 ml/min/kg pour des polymétalliques obtenus par les inventeurs) [Misselwitz B. et al. Pharmacokinetics of Gadomer-17, a new dendritic magnetic resonance contrast agent. MAGMA. 2001. 12 : 128-134.]. Par conséquent, en dépit d'un poids moléculaire comparable, le Gadomer-17 a plutôt un comportement pharmacocinétique qui n'est pas adapté pour mettre en évidence des différences de perméabilité vasculaire entre les deux types de tumeurs, au contraire de composés polymétalliques selon l'invention. Des polymétalliques obtenus par les inventeurs permettent, au délai choisi, non seulement un rehaussement tumoral significatif du signal par rapport au muscle, mais aussi de différencier les tumeurs, les concentrations contenues dans les adénocarcinomes étant nettement supérieures

à celles retrouvées dans les fibroadénomes.

## Revendications

**1.** Composés de formule générale suivante :

W-(A-$_m$ (E)

dans laquelle:

- W est un NOYAU central
- A représente [ $(D)_q$-( $I_{a,b,c,d,e,f})_r$ ] ;
Avec :
  - q = 0 ou q=1
  - r=1 lorsque q=0, ou r est 2 ou 3 lorsque q=1
  - m est compris entre 3 et 6 lorsque q=0 et m est compris entre 2 et 4 lorsque q=1
  - D est une molécule polyfonctionnelle capable de se lier au NOYAU central W par un lien 2 et à au moins deux chélates métalliques par des liens 1
  - $I_{a,b,c,d,e,f}$ signifie $I_a$ ou $I_b$ ou $I_c$ ou $I_d$ ou $I_e$ ou $I_f$ , représentant des dérivés à Rotation Restreinte Ia , $I_b$ , $I_c$ ayant les significations :

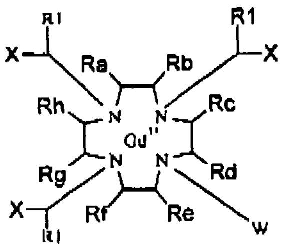

**Ia**

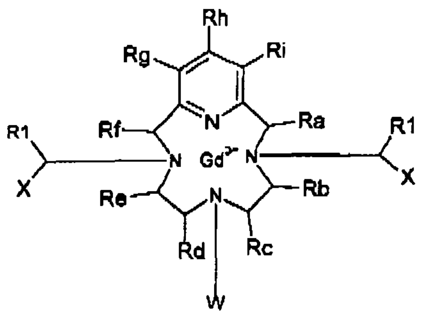

**Ib**

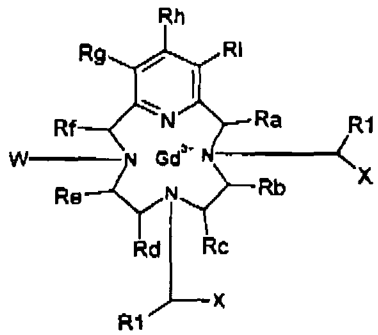

**Ic**

où :

- X représente $CO_2R'a$, CONR'bR'c ou $P(R'd)O_2H$, avec :

  R'a, R'b, R'c représentant H ou (C1-C8) alkyle, éventuellement hydroxylé ;
  R'd représente OH, (C1-C8) alkyle ou (C1-C8) alkoxy, (C1-C8) arylalkyle ou (C1-C8) alkoxyalkyle ;

- R1 représente un groupe hydrophile, de masse moléculaire supérieure à 300 et inférieure à 3000, comprenant au moins trois atomes d'oxygène, sélectionné parmi des groupes :

  - polyoxy (C2-C3) alkylène, notamment le polyéthylène glycol et ses monoéthers et monoesters en C1 à C3
  - Polyhydroxyalkyle
  - Polyol
  - $(R_2\ g)_e\ [(R_2\ g)_i\ R_3]_h$ où :

    - h = 1 ou 2 ; i = 0, 1 ou 2 ; e = 1 à 5
    - $R_2$ représente (les $R_2$ étant identiques ou différents) :

      - rien, un alkylène, un alkoxyalkylène, un polyalkoxyalkylène ;
      - un phénylène, ou un reste hétérocyclique saturé ou non, éventuellement substitué par OH, Cl, Br, I, (C1-C8)alkyle, (C1-C8)alkyloxy, NO2, $NR_XR_Y$, $NR_XCOR_Y$, $CONR_XR_Y$, $COOR_X$, Rx et $R_Y$

étant H ou (C1-C8)alkyle, et les groupes alkyle, alkylène, alkoxy, en C1 à C14 linéaires, ramifiés ou cycliques pouvant être hydroxylés ;

- g représente (les g étant identiques on différents) : rien ou une fonction O, CO, OCO, COO, SO3, OSO2, CONR', NR'CO, NR'COO, OCONR',NR', NR'CS, CSNR', SO2NR', NR'SO2, NR'CSO, OCSNR',NR'CSNR', P(O)(OH)NR', NR'P(O)-(OH), dans laquelle R' est H (C1-C8)alkyle ou $R_3$ ;
- $R_3$ représente alkyle, phényle, alkyle substitué ou interrompu par un ou des groupes phényles, alkyléneoxy; amino ou amido substitués ou non par alkyle éventuellement substitué ou interrompu par l'un des groupes précédents ; les groupes phényles, phénylènes et hétérocycliques pouvant être substitués par OH, Cl, Br, I, (C1-C8)alkyle, (C1-C8)alkyloxy, NO2, $NR_XR_Y$, $NR_XCOR_Y$, $CONR_XR_Y$, $COOR_X$, $R_X$ et $R_Y$ étant H ou (C1-C8)alkyle, et les groupes alkyle, alkylène, alkoxy, en C1 à C14 linéaires, ramifiés ou cycliques pouvant être hydroxylés

- $R_a$ à $R_i$ représentent indépendamment H, alkyle, hydroxyalkyle, alkylphényle, cycloalkyle.
- U est un groupe -CX R4-lien 1, CHR4CON-lien1, CHR4-CHR5OH-lien1
- R4, R5 représentant indépendamment H, alkyle, hydroxyalkyle.
- X ayant la signification ci-dessus.
- $I_d$, Ie, $I_f$ ayant les significations :

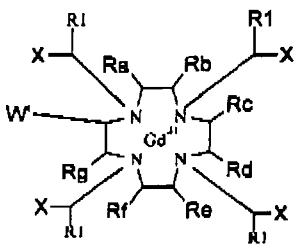

Id

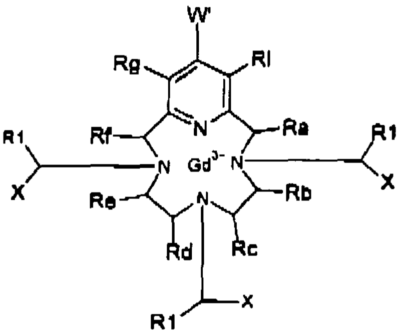

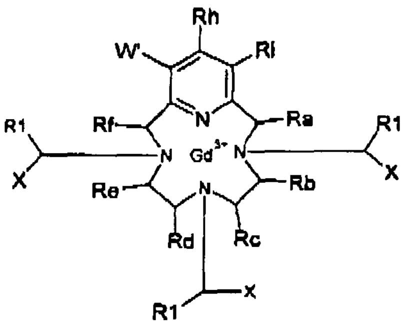

If

$I_e$

- X, R1, Ra à Ri ayant la même signification que ci-dessus.
- U' est un lien 1;

ainsi que les sels des composés de formule (E) avec des acides ou des bases minérales ou organiques pharmaceutiquement acceptables.

**2.** Composés selon la revendication 1, dans lesquels :

- D est un squelette aromatique polyfonctionnalisé par des groupements carboxylates et/ou aminés, de préférence de type 1,3,5 triazine, de formule :

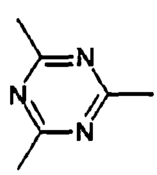

- le lien 1 et le lien 2 sont choisis parmi a) et b) de préférence a) :

a) (CH2)2 - φ - NH2 , (CH2)3 - NH2, NH-(CH2)2-NH, NH-(CH2)3-NH,

a) P1-I-P2, identiques ou différents, P1 et P2 étant choisis parmi OH, SH, NH2, rien, CO2H, NCS, NCO, SO3H,

Avec I = Alkylène, alkoxyalkylène, polyalkoxyalkylène, alkylène interrompu par phénylène, alkylidène, acylidène

- W est le radical d'une molécule organique portant m groupes carbonyle qui forment des groupes carboxamido avec A ; ou W est un groupe :

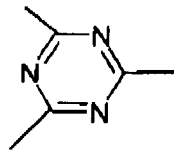

**3.** Composés selon la revendication 1 ou 2, dans lesquels R1 représente (CH2)xCONHR avec x=1, 2 ou 3, et R est un groupe hydrophile de poids moléculaire supérieur à 300, choisi parmi :

1) un groupement :

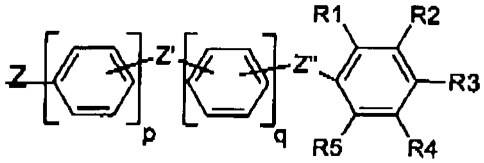

IV1

et Z est une liaison, $CH_2$, $CH_2CONH$ ou $(CH_2)_2NHCO$
Z' est une liaison, O, S, NQ, $CH_2$, CO, CONQ, NQCO, NQ-CONQ ou $CONQCH_2CONQ$,
Z" est une liaison, CONQ, NQCO ou $CONQCH_2CONQ$
p et q sont des nombres entiers dont la somme vaut 0 à 3 ;
$R_1$, $R_2$, $R_3$, $R_4$ ou $R_5$ représentent :

- soit indépendamment l'un de l'autre H, Br, Cl, I, $CONQ_1Q_2$ ou $NQ_1COQ_2$ avec $Q_1$ et $Q_2$ identiques ou différents sont H ou un groupe $(C_1\text{-}C_8)$alkyle mono- ou polyhydroxylé ou éventuellement interrompu par un ou des atomes d'oxygène, et au moins l'un et au plus deux des $R_1$ à $R_5$ sont $CONQ_1Q_2$ ou $NQ_1COQ_2$;
- soit $R_2$ et $R_4$ représentent

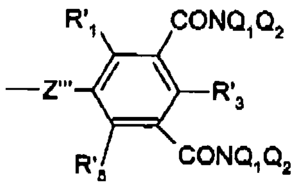

et $R_1$, $R'_1$, $R_3$, $R'_3$, $R_5$ et $R'_5$, identiques ou différents, représentent H, Br, Cl ou I, $Q_1$ et $Q_2$ ont la même signification que précédemment et Z"' est un groupe choisi parmi CONQ, $CONOCH_2CONQ$, $CONQCH_2$, NQCONQ, $CONQ(CH_2)_2NQCO$ et Q est H ou $(C_1\text{-}C_4)$alkyle, éventuellement hydroxylé, les groupes alkyle pouvant être linéaires ou ramifiés ;

2) une branche dite "flash

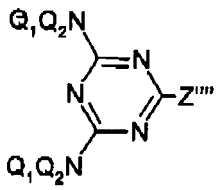

avec Z '''' étant $NQCH_2(CH_2OCH_2)_i(CH_2)_jNH_2$, avec i = 2 à 6 et j = 1 à 6

**4.** Composés selon la revendication 1, de formule :

W1-$(A1)_{m1}$ (i1) PolyM RR

dans laquelle :

- m1 est 3, 4, 5 ou 6 ;
- W1 est le radical d'une molécule organique portant m1 groupes carbonyle qui forment des groupes carboxamido avec A1 ;
ou W1 est un groupe :

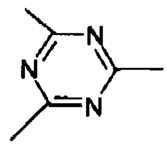

- A1 représente le groupe :

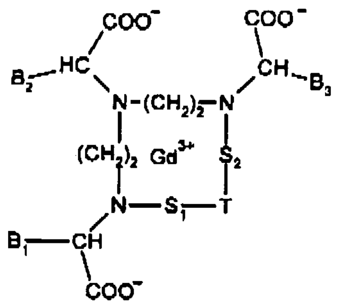

II 1

dans lequel :
- $S_1$-T-$S_2$- est

- soit

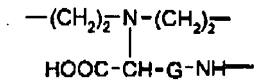

où $S_1 = S_2 = (CH_2)_2$
avec $B_1$, $B_2$, $B_3$ représentant tous les trois $(CH_2)_xCONHR$ avec x = 1, 2 ou 3

- soit

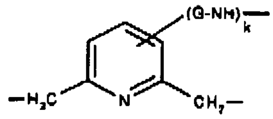

III1

avec k = 0 et $S_1 = S_2 = CH_2$
l'un des B1,B2,B3 représentant G-NH, et les autres représentant $(CH_2)_xCONHR$
- soit

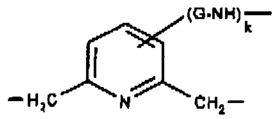

III1

avec k=1
$B_1$, $B_2$, $B_3$ représentant tous les trois $(CH_2)_xCONHR$ avec x = 1, 2 ou 3 et GNH choisi parmi :
les groupes $-(CH_2)_n-NH-$ avec n = 1 à 4,

ou

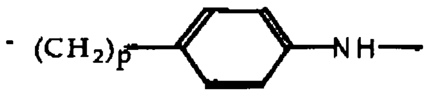 avec p = 0 à 3 ;

étant précisé que :

- R représente un groupe hydrophile de poids moléculaire supérieur à 200, de préférence R représente un groupement :

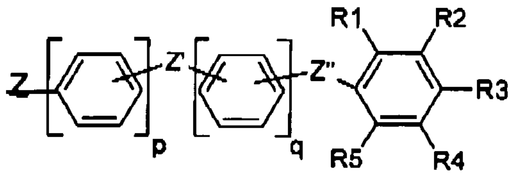

IV1

et Z est une liaison, $CH_2$, $CH_2CONH$ ou $(CH_2)_2NHCO$
Z' est une liaison, O, S, NQ, $CH_2$, CO, CONQ, NQCO, NQ-CONQ ou $CONQCH_2CONQ$,
Z" est une liaison, CONQ, NQCO ou $CONQCH_2CONQ$
p et q sont des nombres entiers dont la somme vaut 0 à 3 ;
$R_1$, $R_2$, $R_3$, $R_4$ ou $R_5$ représentent:

- soit indépendamment l'un de l'autre H, Br, Cl, I, $CONQ_1Q_2$ ou $NQ_1COQ_2$ avec $Q_1$ et $Q_2$ identiques ou différents sont H ou un groupe $(C_1\text{-}C_8)$alkyle mono- ou polyhydroxylé ou éventuellement interrompu par un ou des atomes d'oxygène, et au moins l'un et au plus deux des $R_1$ à $R_5$ sont $CONQ_1Q_2$ ou $NQ_1COQ_2$ ;
- soit $R_2$ et $R_4$ représentent

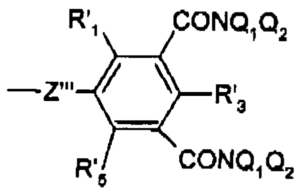

et $R_1$, $R'_1$, $R_3$, $R'_3$, $R_6$ et $R'_5$, identiques ou différents, représentent H, Br, Cl ou I, $Q_1$ et $Q_2$ ont la même signification que précédemment et Z"' est un groupe choisi parmi CONQ, $CONQCH_2CONQ$, $CONQCH_2$, NQCONQ, $CONQ(CH_2)_2NQCO$ et Q est H ou $(C_1\text{-}C_4)$alkyle, éventuellement hydroxylé, les groupes alkyle pouvant être linéaires ou ramifiés
ou un groupement FLASH de formule :

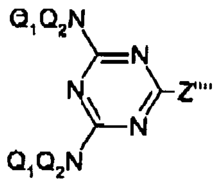

avec Z "" étant $NQCH_2(CH_2OCH_2)_i(CH_2)_jNH_2$, avec i = 2 à 6 et j = 1 à 6 ;
ainsi que les sels des composés de formule I1 avec des acides ou des bases minérales ou organiques pharmaceutiquement acceptables.

**5.** Composés selon la revendication 1, de formule :

W2 - $(A2)_{m2}$ (I2) PolyD RR

dans laquelle :

- m2 est 2, 3 ou 4 ;
- W2 est le radical d'une molécule organique portant m2 groupes carbonyle qui forment des groupes carboxamido avec A2 ;

ou W2 est un groupe :

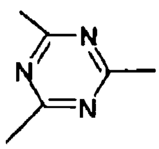

- A2 représente 1) le groupe :

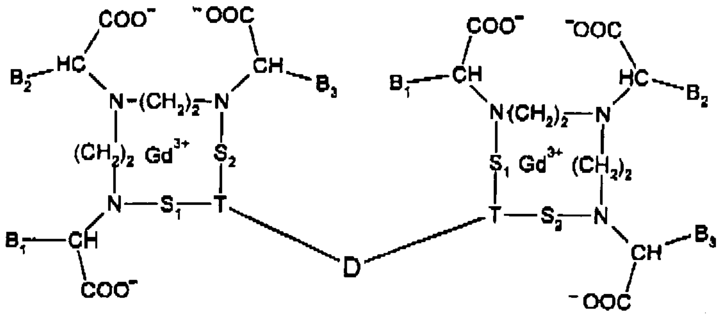

II2

dans lequel

- $S_1$-T-$S_2$- est

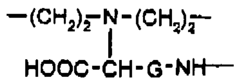

où $S_1 = S_2 = (CH_2)_2$
$B_1$, $B_2$, $B_3$ représentant tous les trois $(CH_2)_xCONHR$ avec x = 1, 2 ou 3
- ou A2 représente 2)

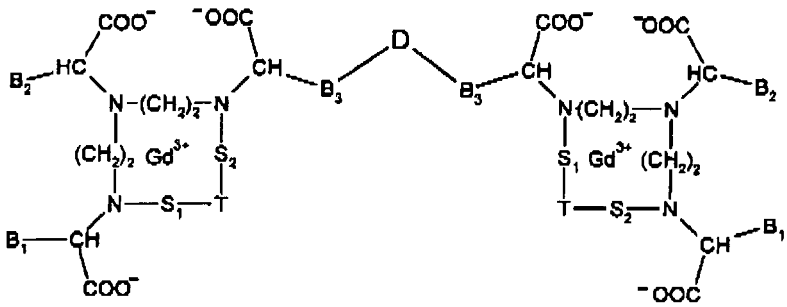

IIa2 (composé dit PCTA N fonctionnalisé)
Ou IIb2 (composé dit PCTA N fonctionnalisé et isomère de position du IIb2)

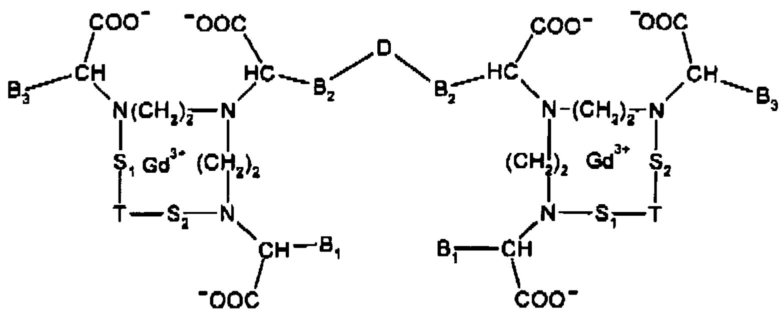

II b2

Dans lesquels $S_1$-T-$S_2$- est :

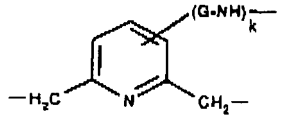

III2

avec k = 0 et $S_1 = S_2 = CH_2$;
$B_3$ représentant G-NH, et B1 et B2 représentant $(CH_2)_xCONHR$ pour IIa2
$B_2$ représentant G-NH, et B1 et B3 représentant $(CH_2)_xCONHR$ pour II b2
- ou A2 représente 3)

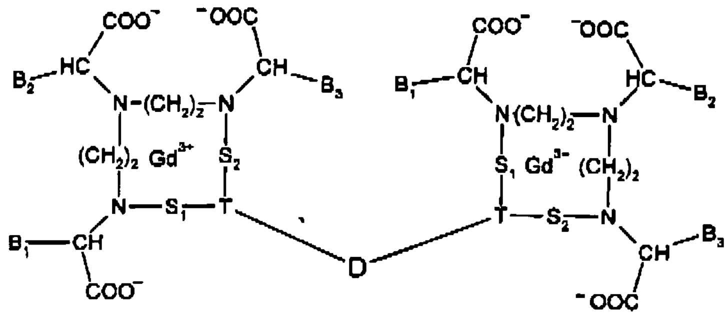

IIc2 (composé dit PCTA C fonctionnalisé)
lorsque $S_1$-T-$S_2$- est :

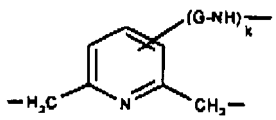

III2

avec k = 1 et $S_1 = S_2 = CH_2$;
$B_1$, $B_2$, $B_3$ représentant tous les trois $(CH_2)_xCONHR$ avec x = 1, 2 ou 3 pour II c 2
Sachant que, pour II2, IIa2, IIb2 et IIc2,
GNH est choisi parmi les groupes $-(CH_2)_n$-NH- avec n = 1 à 4,

ou

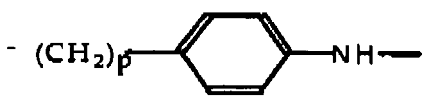 avec p = 0 à 3 ;

- D étant (Div - lien 2) et étant un diviseur, Div étant de préférence de type 1,3,5 triazine, et D étant de préférence :

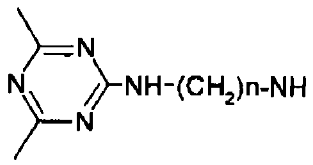

ou

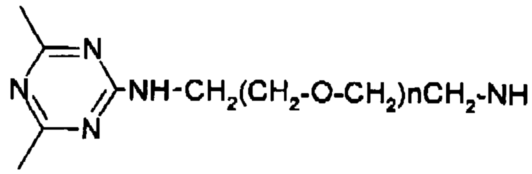

- R représente un groupe hydrophile de poids moléculaire supérieur à 200, de préférence R représente un groupement :

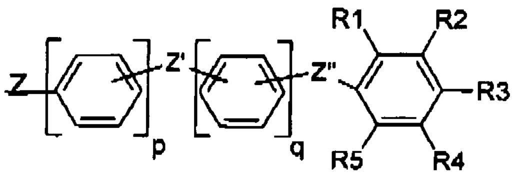

IV2

et Z est une liaison, $CH_2$, $CH_2CONH$ ou $(CH_2)_2NHCO$
Z' est une liaison, O, S, NQ, $CH_2$, CO, CONQ, NQCO, NQ-CONQ ou $CONQCH_2CONQ$,
Z" est une liaison, CONQ, NQCO ou $CONQCH_2CONQ$
p et q sont des nombres entiers dont la somme vaut 0 à 3 ;
$R_1$, $R_2$, $R_3$, $R_4$ ou $R_5$ représentent ;

- soit indépendamment l'un de l'autre H, Br, Cl, I, $CONQ_1Q_2$ ou $NQ_1COQ_2$ avec $Q_1$ et $Q_2$ identiques ou différents sont H ou un groupe $(C_1-C_8)$alkyle mono- ou polyhydroxylé ou éventuellement interrompu par un ou des atomes d'oxygène, et au moins l'un et au plus deux des $R_1$ à $R_5$ sont $CONQ_1Q_2$ ou $NQ_1COQ_2$;
- soit $R_2$ et $R_4$ représentent

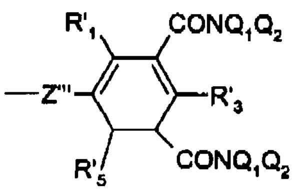

et $R_1$, $R'_1$, $R_3$, $R'_3$, $R_5$ et $R'_5$, identiques ou différents, représentent H, Br, Cl ou I, $Q_1$ et $Q_2$ ont la même signification que précédemment et Z"' est un groupe choisi parmi CONQ, $CONQCH_2CONQ$, $CONQCH_2$, NQCONQ, $CONQ(CH_2)_2NQCO$ et Q est H ou $(C_1-C_4)$alkyle, éventuellement hydroxylé, les groupes alkyle pouvant être linéaires ou ramifiés
ou un groupement FLASH de formule:

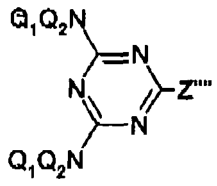

avec Z "" étant $NQCH_2(CH_2OCH_2)_i(CN_2)_jNH_2$, avec i = 2 à 6 et j = 1 à 6 ; ainsi que les sels des composés de formule I1 avec des acides ou des bases minérales ou organiques pharmaceutiquement acceptables.

**6.** Composés selon l'une quelconque des revendications précédentes dans lesquels respectivement, W pour les composés de formule (E), W1 pour les composés de formule I1, W2 pour les composés de formule 12, est choisi parmi les groupes :

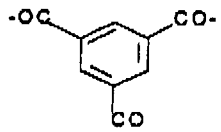 ; 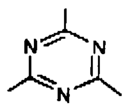 ; 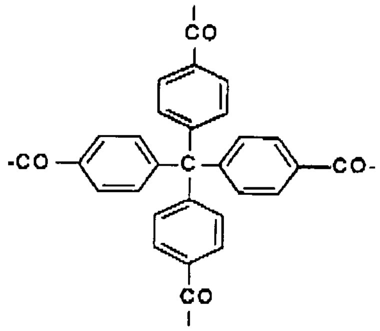 ;

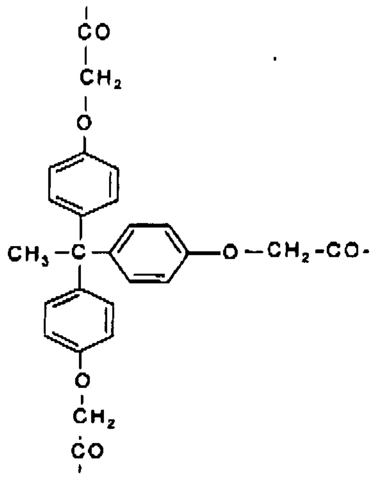 ; 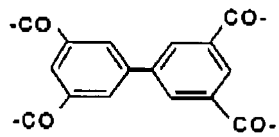 ;

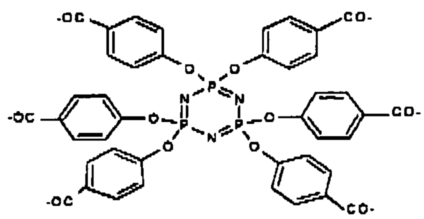 ; 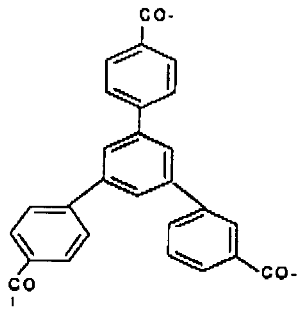

et m est égal au nombre de liaisons libres de W.

**7.** Composés selon l'une quelconque des revendications 1 à 4 dans lesquels, respectivement, W pour les composés de formule (E), W1 pour les composés de formule 11, W2 pour les composés de formule 12, est :

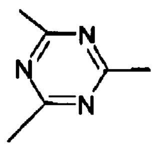

8. Composés selon l'une quelconque des revendications 1 à 4 dans lesquels, respectivement, W pour les composés de formule (E), W1 pour les composés de formule I1, W2 pour les composés de formule I2, est :

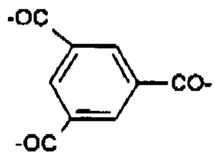

ou

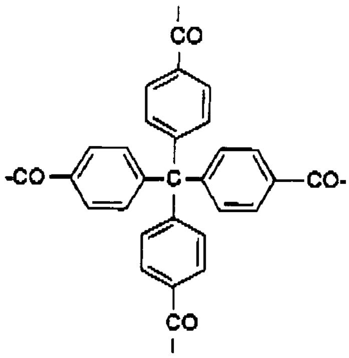

9. Composés selon l'une quelconque des revendications 1 à 4 dans lesquels, respectivement, W pour les composés de formule (E), W1 pour les composés de formule I1, W2 pour les composés de formule 12, est :

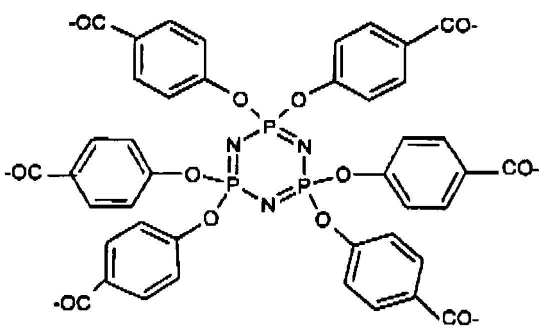

10. Composés selon l'une quelconque des revendications précédentes dans lesquels x est 2.

11. Composés selon l'une quelconque des revendications 4, ou 6 à 10, de formule I1, dans laquelle $-S_1 - T - S_2-$ représente :

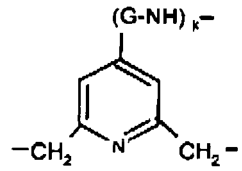

avec $S_1 = S_2 = CH_2$.

**12.** Composés selon la revendication 11 de formule I1 dans laquelle k est 1 et G est $-(CH_2)_3-$.

**13.** Composés selon la revendication 11 de formule I1 dans laquelle k est 0 et $B_2$ ou $B_3$ représente $-(-CH_2)_3NH-$ ou

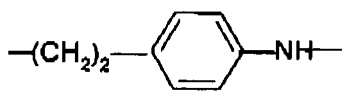

**14.** Composés selon l'une quelconque des revendications 4 à 10, de formule I1 ou 12, dans laquelle $-S_1 - T - S_2-$ représente:

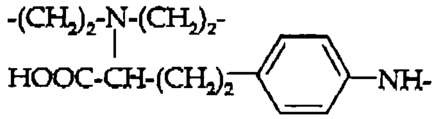

ou

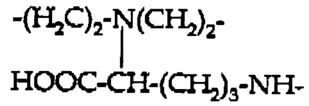

avec $S_1 = S_2 = (CH_2)_2$.

**15.** Composés selon l'une quelconque des revendications précédentes pour lesquels $B_1$, $B_2$ et $B_3$, lorsqu'ils ne représentent pas -G-NH, représentent $-(CH_2)_2CONHR$, avec dans R, p = q = 0 et Z étant $-CH_2CONH$.

**16.** Composés selon la revendication 15 pour lesquels R représente :

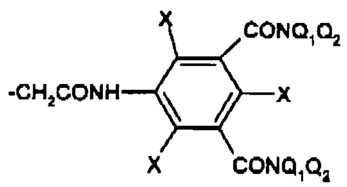

et les X sont identiques et représentent Br ou I tandis que $Q_1$ et $Q_2$, identiques ou différents, sont des groupes $(C_1-C_8)$alkyle, mono- ou polyhydroxylés, de telle sorte que chaque $CONQ_1Q_2$ comporte de 4 à 10 hydroxyles au total.

**17.** Composés selon la revendication 15 pour lesquels R représente :

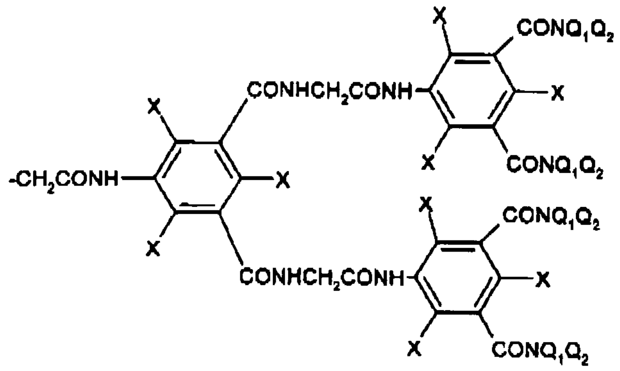

et les X identiques sont Br ou I et $Q_1$ et $Q_2$, identiques ou différents, sont des groupes $(C_1\text{-}C_8)$alkyle, mono ou polyhydroxylés de telle sorte que chaque groupe $CONQ_1Q_2$ comporte de 4 à 10 hydroxyles au total.

**18.** Composés selon l'une quelconque des revendications 1 à 14 pour lesquels R représente :

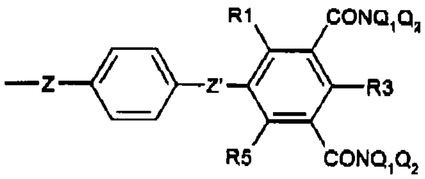

Z est $CH_2$ ou $CH_2CONH$, Z' est CONH ou $CONHCH_2CONH$, $R_1$, $R_3$, $R_5$, identiques, sont Br ou I, $Q_1$ et $Q_2$, identiques ou différents, étant des groupes $(C_1\text{-}C_6)$alkyle, mono ou polyhydroxylés, de telle sorte que chaque groupe $CONQ_1Q_2$ comporte de 4 à 10 hydroxyles au total.

**19.** Composés selon l'une quelconque des revendications 1 à 14 pour lesquels R représente :

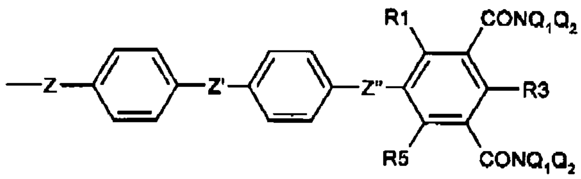

Z est $CH_2CONH$, Z' est CONH, Z" est $CONHCH_2CONH$ et $R_1$, $R_3$, $R_5$ identiques sont Br ou I et $Q_1$ et $Q_2$, identiques ou différents, sont des groupes $(C_1\text{-}C_8)$alkyle, monohydroxylés ou polyhydroxylés, de telle sorte que chaque groupe $CONQ_1Q_2$ comporte de 4 à 10 hydroxyles au total.

**20.** Composés selon l'une quelconque des revendications 1 à 14 pour lesquels R représente

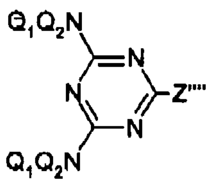

avec Z"" étant $NQ(CH_2)_j\ (CH_2OCH_2)_i\ (CH_2)_jNH_2$, avec i = 2 à 6 et j = 1 à 6, de préférence

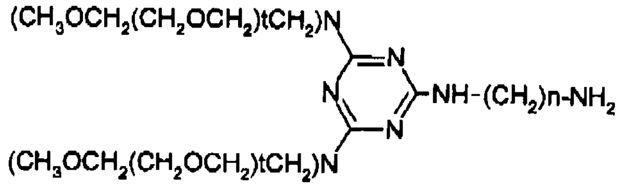

ou

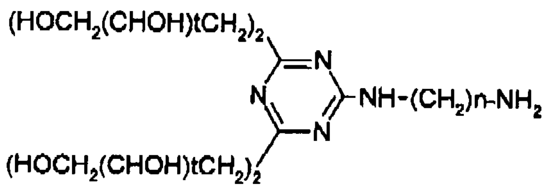

avec t =1,2,3 ou 4 et n=2 à 6.

**21.** Composés selon l'une quelconque des revendications précédentes, dans lesquels $Q_1$ représente $CH_2CHOHCH_2OH$ ou $CH_2(CHOH)_4CH_2OH$ et $Q_2$ représente $CH_2(CHOH)_4CH_2OH$.

**22.** Composés de formule

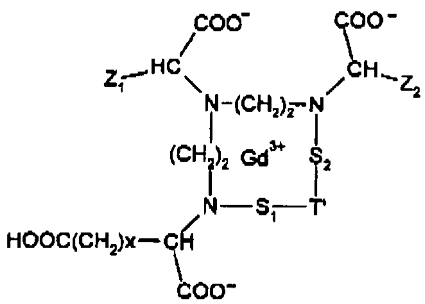

V1

dans laquelle x = 1, 2 ou 3 et $-S_1-T'-S_2-$ est : 1)

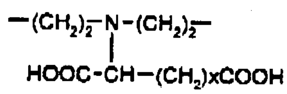

avec $S_1 = S_2 = (CH_2)_2$
ou

2)

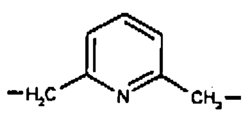

avec $S_1 = S_2 = CH_2$
et l'un des groupes $Z_1$ ou $Z_2$ est choisi parmi les groupes $-(-CH_2)_3NH_2$ ou

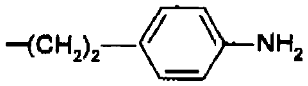

et l'autre des $Z_1$ ou $Z_2$ est $(CH_2)_xCOOH$ ;
sous forme de sels avec une base alcaline.

**23.** Composés selon la revendication 22 de formule V1 dans laquelle x = 2.

**24.** Composé de formule:

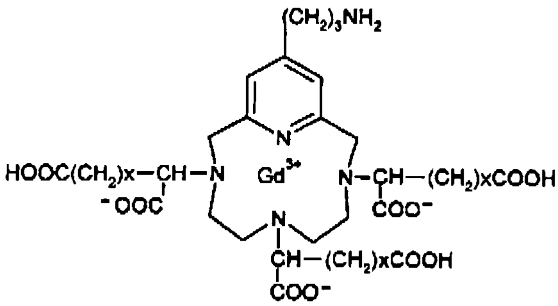

VI 1

dans laquelle x = 1, 2 ou 3
sous forme de sels avec une base alcaline.

**25.** Composés selon la revendication 24 de formule VI dans laquelle x = 2.

**26.** Composé de formule :

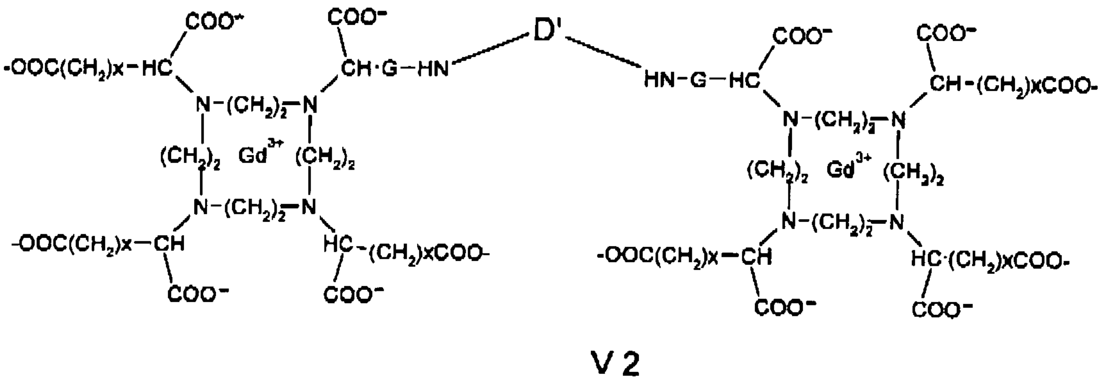

V 2

ou

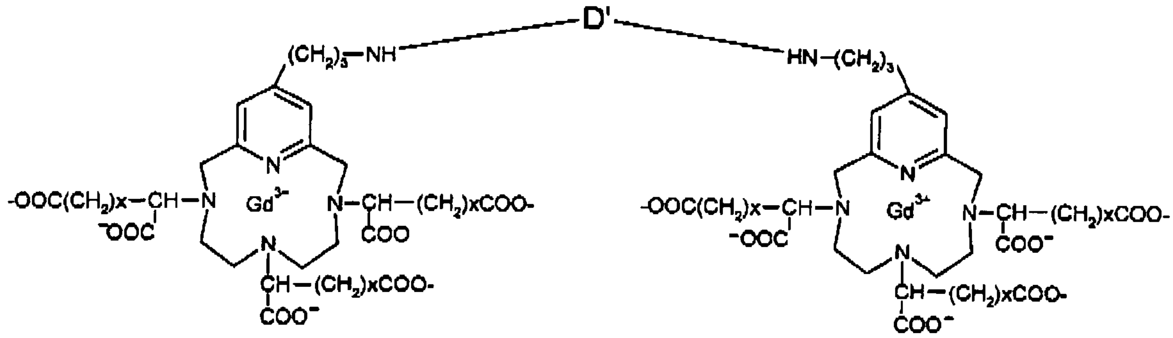

VI 2

dans laquelle x = 1, 2 ou 3
D'=D-H
D étant-:

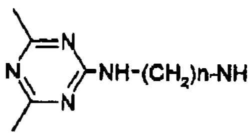

ou

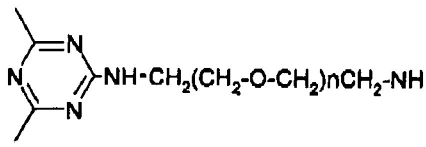

avec n= 2 à 6
G-NH étant $-(CH_2)_3$-NH- ou

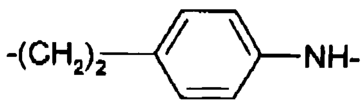

sous forme de sels avec une base alcaline.

**27.** Produit de contraste pour l'imagerie par résonance magnétique, **caractérisé en ce qu'**il comprend un composé selon l'une des revendications 1 à 21, éventuellement associé à un véhicule pharmaceutiquement acceptable.

**28.** Produit de contraste selon la revendication 27, présenté sous forme d'une solution stérile injectable.

**29.** Produit de contraste selon la revendication 27 pour son utilisation dans le diagnostique d'une pathologie cardiovasculaire, cancéreuse, inflammatoire.

**30.** Utilisation d'un produit de contraste selon la revendication 27 pour la préparation d'une composition destinée au diagnostique d'une pathologie cardiovasculaire, cancéreuse, inflammatoire.

**31.** Procédé de criblage consistant à sélectionner les composés de formule (E) selon la revendication 1, efficaces sur le plan diagnostic, ledit procédé comprenant :

- la préparation d'un composé candidat polymétallique de formule (E)
- l'utilisation du composé candidat dans un protocole de test approprié d'une indication diagnostique
- la sélection des candidats présentant une efficacité massique d'au moins 30%, de préférence d'au moins 50% supérieure à celle du chélate non polymétallique correspondant.

## Claims

**1.** Compounds with the following formula:

$$W\text{-}(A)_m \ (E)$$

in which:

- W is a central NUCLEUS
- A represents $[(D)q - ( I_{a,b,c,d,e,f})_r ]$;
Where:
- q = 0 or q = 1
- r = 1 when q = 0, or r is 2 or 3 when q = 1
- m is between 3 and 6 when q = 0 and m is between 2 and 4 when q = 1
- D is a polyfunctional molecule which can bond to the central NUCLEUS W by a linker 2 and to at least two metal chelates by links 1
- $I_{a,b,c,d,e,f}$ signifies $I_a$ or $I_b$ or $I_c$, or $I_d$ or $I_e$ or $I_f$ , which represent derivatives with Restricted Rotation

Where $I_a$, $I_b$ , $I_c$ have significances:

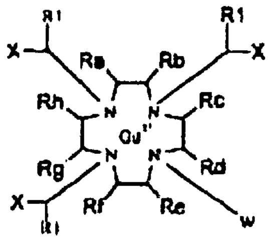

Ia

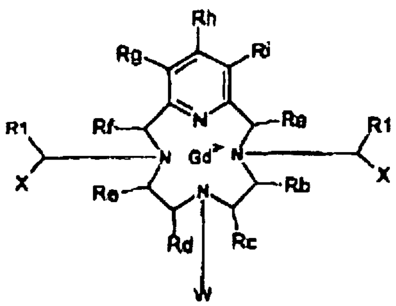

Ib

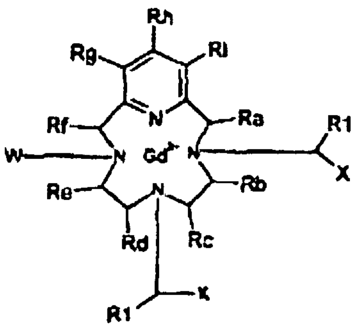

Ic

where:

- X represents $CO_2R'a$, CONR'bR'c or $P(R'd)O_2H$, where:
where R'a, R'b, R'c represent H or (C1-C8) alkyl, optionally hydroxylated;
R'd represents OH, (C1-C8) alkyl or (C1-C8) alkoxy, (C1-C8) arylalkyl or (C1-C8) alkoxyalkyl;

  - R1 represents a hydrophilic group, whose molecular mass is greater than 300 and less than 3000, which includes at least three oxygen atoms, chosen from amongst the following groups:
  - polyoxy (C2-C3) alkylene, in particular polyethylene glycol and its C1 to C3 monoethers and monoesters
  - Polyhydroxyalkyl
  - Polyol
  - $(R_2\ g)_e\ [(R_2\ g)_i\ R_3]_h$ where:
  - h = 1 or 2; i = 0, 1 or 2; e = 1 to 5
  - R2 represents (the $R_2$s being the same or different):
  - nothing, an alkylene, an alkoxyalkylene, a polyalkoxyalkylene;

- a phenylene, or a heterocyclic remainder, saturated or unsaturated, optionally substituted by OH, Cl, Br, I (C1-C8) alkyl, (C1-C8) alkyloxy, N02, $NR_xR_Y$, $NR_xCOR_y$, $CONR_xR_y$, $COOR_x$, where $R_x$ and $R_y$ are H or (C1-C8)alkyl, and the linear, cyclic or cross-linked C1 to C14 alkyl, alkylene, alkoxy may be hydroxylated;
- g represents (the g's being the same or different): nothing or an O, CO, OCO, COO, S03, OS02, CONR', NR'CO, NR'COO, OCONR',NR', NR'CS, CSNR', S02NR', NR'S02, NR'CSO, OCSNR',NR'CSNR', P(O)(OH)NR', NR'P(0)-(OH) function, in which R' is H (C1-C8)alkyl or $R_3$;
- $R_3$ represents alkyl, phenyl, alkyl substituted or interrupted by one or more phenyl, alkyleneoxy groups; amino or amido, substituted or not by alkyl optionally substituted or interrupted by one of the preceding groups; phenyl, phenylene and heterocyclic groups which may be substituted by OH, Cl, Br, I, (C1-C8)alkyl, (C1-C8)alkyloxy, N02, $NR_xR_y$, $NR_xCOR_Y$, $CONR_xR_y$, $COOR_x$, where $R_x$ and $R_y$ are H or (C1-C8)alkyl and the C1 to C14 linear, cross-linked or cyclic alkyl, alkylene, alkoxy groups may be hydroxylated
- $R_a$ to $R_i$ independently represent H, alkyl, hydroxyalkyl, alkylphenyl, cycloalkyl.
- U is a -CX R4-linker 1, CHR4CON-linker 1, CHR4-CHR5OH-linker 1 group
- R4, R5 independently represent H, alkyl, hydroxyalkyl.
- Where X has the above significance.
- where $I_d$, $I_e$, $I_f$ have the following significance:

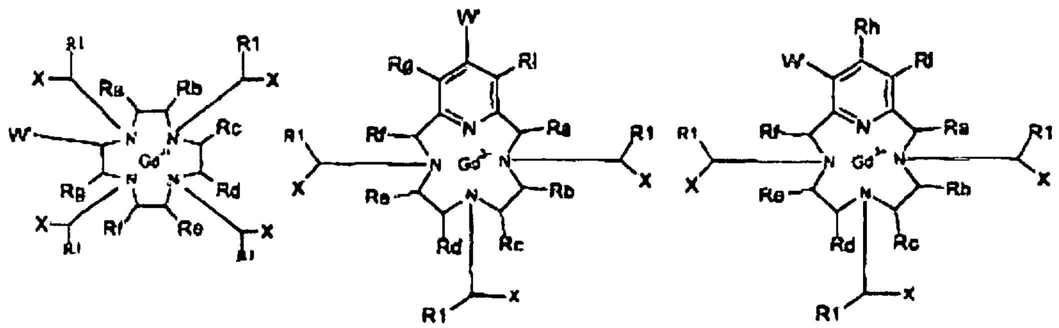

Id

If

$I_e$

- where X, R1, $R_a$ to $R_i$ have the same significance as above.
- U' is a linker 1;

as well as salts of compounds of formula (E) with pharmaceutically acceptable mineral or organic acids or bases.

**2.** Compounds according to claim 1, in which:

- D is an aromatic skeleton made polyfunctional by carboxylate and/or amine groups, preferably of the 1, 3, 5 triazine type with the formula:

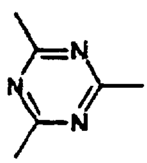

- linker 1 and linker 2 are chosen from a) and b) and preferably a):

a) (CH2)2-ϕ-NH2, (CH2)3 - NH2, NH-(CH2)2-NH, NH-(CH2)3-NH,
a) P1-I-P2, which are the same or different, with P1 and P2 being selected from OH, SH, NH2, nothing, CO2H, NCS, NCO, SO3H,
Where I= Alkylene, alkoxyalkylene, polyalkoxyalkylene, alkylene interrupted by phenylene, alkylidene, acylidene

- W is an organic molecule radical containing m carbonyl groups which form carboxamido groups with A; or W is a group as shown below:

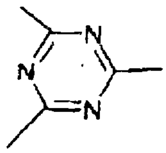

3. Compounds according to claims 1 or 2 in which R1 represents (CH2)xCONHR with x = 1, 2 or 3, and R is a hydrophilic group whose molecular weight is greater than 300, selected from amongst:

1) a group:

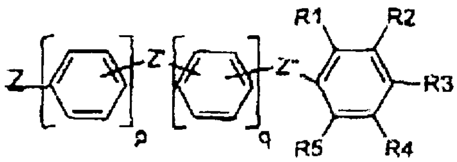

IV1

and Z is a bond, $CH_2$, $CH_2CONH$ or $(CH_2)_2NHCO$
Z' is a bond, 0, S, NQ, $CH_2$, CO, CONQ, NQCO, NQ-CONQ or $CONQCH_2CONQ$,
Z" is a bond, CONQ, NQCO or $CONQCH_2CONQ$
p and q are whole numbers the sum of which is equal to from 0 to 3;
$R_1$, $R_2$, $R_3$, $R_4$ or $R_5$ represent:

- either independently of each other H, Br, Cl, I, $CONQ_1Q_2$ or $NQ_1COQ_2$ where $Q_1$ and $Q_2$, which are the same or different are H or a ($C_1$-$C_8$)alkyl group which is mono- or poly- hydroxylated or optionally interrupted by one or more atoms of oxygen, and at least one, and at most two, of $R_1$ to $R_5$ are $CONQ_1Q_2$ or $NQ_1COQ_2$;
- or $R_2$ and $R_4$ represent

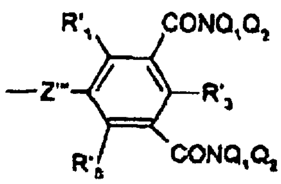

and $R_1$, $R'_1$, $R_3$, $R'_3$, $R_5$ and $R'_5$, which are the same or different, represent H, Br, Cl or I, $Q_1$ and $Q_2$ have the same significance as previously and Z"' is a group chosen from amongst CONQ, $CONQCH_2CONQ$, $CONQCH_2$, NQCONQ, $CONQ(CH_2)_2NQCO$ and Q is H or ($C_1$-$C_4$)alkyl, optionally hydroxylated, where the alkyl groups may be linear or cross-linked;

2) a so-called « flash » branch

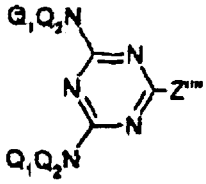

with Z''' being $NQCH_2(CH_2OCH_2)_i(CH_2)_jNH_2$, with i = 2 to 6 and j = 1 à 6

**4.** Compounds according to claim 1, with the formula:

W1 - $(A1)_{m1}$ (I1) PolyM RR

in which:

- ml is 3, 4, 5 or 6;
- W1 is an organic molecule radical containing m1 carbonyl groups which form carboxamido groups with A1; or W1 is a group:

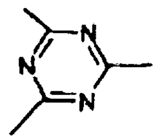

- A 1 represents the group

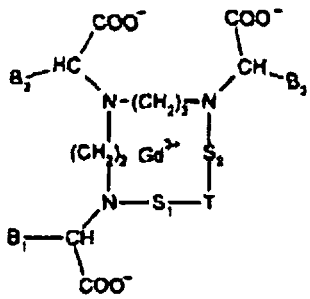

II 1

in which:

- $S_1$-T-$S_2$- is - either

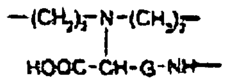

where $S_1 = S_2 = (CH_2)_2$
with $B_1$, $B_2$, $B_3$ representing all three $(CH_2)_xCONHR$ with x = 1, 2 or 3
- or

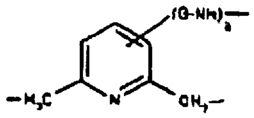

III1

with k = 0 and $S_1 = S_2 = CH_2$
one of B1,B2,B3 representing G-NH, and the others representing $(CH_2)_xCONHR$
- or

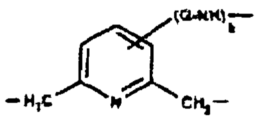

III1

where k =1
$B_1$, $B_2$, $B_3$ representing all three $(CH_2)_xCONHR$ with x = 1, 2 or 3 and GNH chosen from amongst:
- $(CH_2)_n$-NH- groups with n = 1 to 4,
or

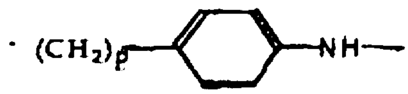

with p = 0 to 3;
it being specified that:
- R represents a hydrophilic group with molecular weight greater than 200, and preferably R represents a group :

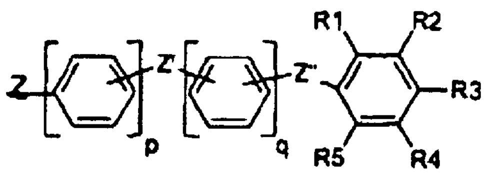

IV1

and Z is a bond, $CH_2$, $CH_2CONH$ or $(CH_2)_2NHCO$
Z' is a bond, 0, S, NQ, $CH_2$, CO, CONQ, NQCO, NQ-CONQ or $CONQCH_2CONQ$,
Z" is a bond CONQ, NQCO or $CONQCH_2CONQ$
p and q are whole numbers, the sum of which is equal to from 0 to 3;
$R_1$, $R_2$, $R_3$, $R_4$ or $R_5$ represent:
- either independently of each other H, Br, Cl, I, $CONQ_1Q_2$ or $NQ_1COQ_2$ where $Q_1$ and $Q_2$, which are the same or different, are H or a $(C_1$-$C_8)$alkyl group which is mono- or poly-hydroxylated or optionally interrupted by one or more atoms of oxygen, and at least one, and at most two, of $R_1$ to $R_5$ are $CONQ_1Q_2$ or $NQ_1COQ_2$;
- or $R_2$ and $R_4$ represent

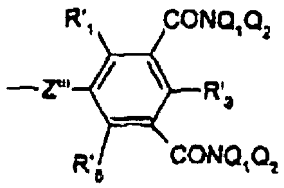

and $R_1$, $R'_1$, $R_3$, $R'_3$, $R_5$ and $R'_5$, which are the same or different, represent H, Br, Cl or I, $Q_1$ and $Q_2$ have the same significance as previously and Z''' is a group chosen from amongst CONQ, $CONQCH_2CONQ$, CONQCH2, NQCONQ, $CONQ(CH_2)_2NQCO$ and Q is H or $(C_1-C_4)$alkyl, optionally hydroxylated, where the alkyl groups may be linear or cross-linked;
or a FLASH group with the formula:

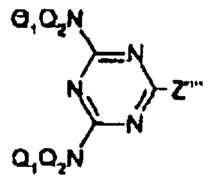

with Z'''' being $NQCH_2(CH_2OCH_2)_i(CH_2)_jNH2$, with i = 2 to 6 and j = 1 to 6;
as well as salts of compounds of formula I1 with pharmaceutically acceptable mineral or organic acids or bases.

**5.** Compounds according to claim 1, with the formula:

W2 - $(A2)_{m2}$ (12) PolyD RR

in which:

- m2 is 2, 3 or 4;
- W2 is an organic molecule radical containing m2 carbonyl groups which form carboxamido groups with A2; or W2 is a group:

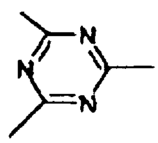

- A2 represents 1) the group:

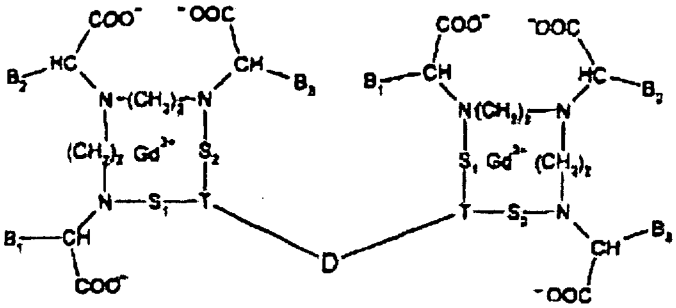

II2

in which
- $S_1$-T-$S_2$- is

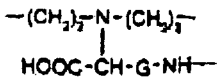

where $S_1 = S_2 = (CH_2)_2$
$B_1$, $B_2$, $B_3$ representing all three $(CH_2)_xCONHR$ with x = 1, 2 or 3
- or A2 represents 2)

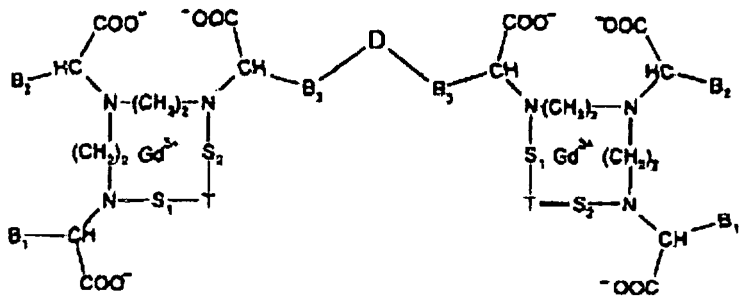

IIa2 (compound called N functionalized PCTA)
Or IIb2 (compound called N- functionalized PCTA and positional isomer of IIb2)

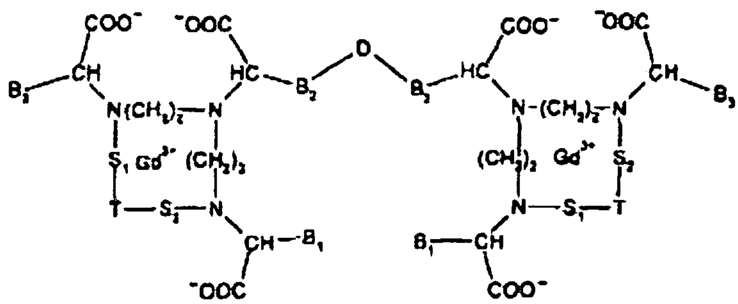

II b2

In which $S_1$-T-$S_2$- is:

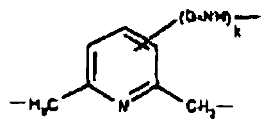

$III_2$

where k = 0 and $S_1 = S_2 = CH_2$;
with $B_3$ representing G-NH, and B1 and B2 representing $(CH_2)_x$CONHR for IIa2
$B_2$ representing G-NH, and B1 and B3 representing $(CH_2)_x$CONHR for II b2
- or A2 represents 3)

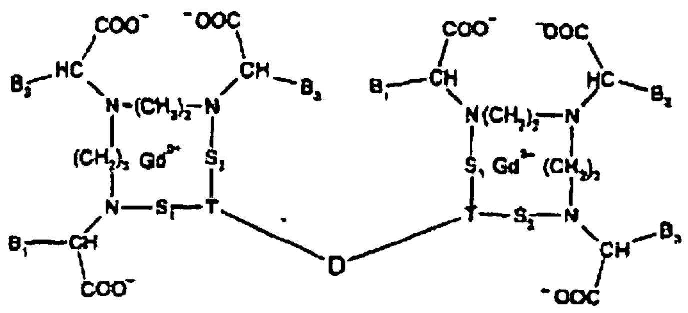

IIc2 (compound called N functionalized PCTA)
when -$S_1$-T-$S_2$- is:

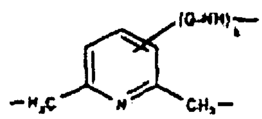

$III_2$

where k = 1 and $S_1 = S_2 = CH_2$;
$B_1$, $B_2$, $B_3$ representing all three $(CH_2)_x$CONHR with x = 1, 2 or 3 for II c2
Bearing in mind that for II2, IIa2, IIb2 and IIc2,
GNH is chosen from amongst -$(CH_2)_n$-NH- groups with n = 1 to 4,
or

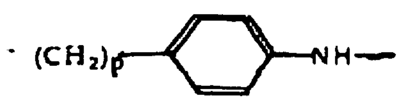

with p = 0 to 3
- D being (Div - linker 2) and being a divider, Div being preferably of the type 1,3,5 triazine, and D being preferably:

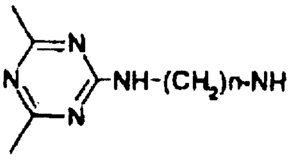

or

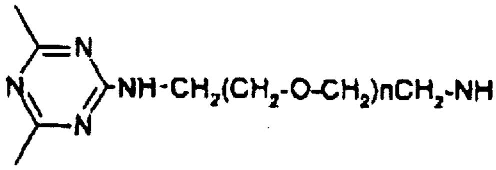

- R represents a hydrophilic group with molecular weight greater than 200, and preferably R represents a group:

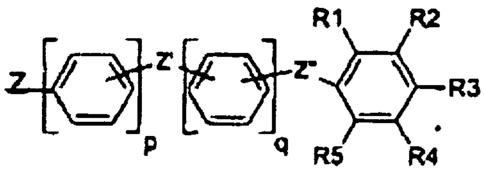

IV2

and Z is a bond, $CH_2$, $CH_2CONH$ or $(CH_2)_2NHCO$
Z' is a bond, 0, S, NQ, $CH_2$, CO, CONQ, NQCO, NQ-CONQ or $CONQCH_2CONQ$,
Z" is a bond, CONQ, NQCO or $CONQCH_2CONQ$
p and q are whole numbers, the sum of which is equal to from 0 to 3;
$R_1$, $R_2$, $R_3$, $R_4$ or $R_5$ represent:
- either independently of each other H, Br, Cl, I, $CONQ_1Q_2$ or $NQ_1COQ_2$ where $Q_1$ and $Q_2$, which are the same or different, are H or a $(C_1$-$C_8)$alkyl group which is mono- or poly-hydroxylated or optionally interrupted by one or more atoms of oxygen, and at least one, and at most two, of $R_1$ to $R_5$ are $CONQ_1Q_2$ or $NQ_1COQ_2$;
- or $R_2$ and $R_4$ represent

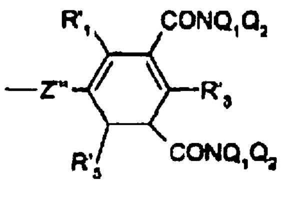

and $R_1$, $R'_1$, $R_3$, $R'_3$, $R_5$ and $R'_5$, which are identical or different, represent H, Br, Cl or I, $Q_1$ and $Q_2$ have the same significance as previously and Z"' is a group chosen from amongst CONQ, $CONQCH_2CONQ$, $CONQCH_2$, NQCONQ, $CONQ(CH_2)_2NQCO$, and Q is H or $(C_1$-$C_4)$alkyl, optionally hydroxylated, where the alkyl groups may be linear or cross-linked or a FLASH group with the formula:

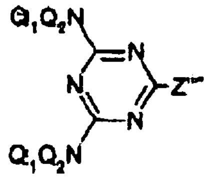

with Z'''' being $NQCH_2(CH_2OCH_2)_i(CH2)_jNH2$, with i = 2 to 6 and j = 1 à 6 ; as well as salts of compounds of formula I1 with pharmaceutically acceptable mineral or organic acids or bases.

6. Compounds according to any of the preceding claims whatsoever in which, respectively, W for compounds of formula (E), W1 for compounds of formula I1, W2 for compounds of formula 12, is selected from amongst the groups:

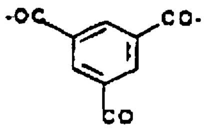 : 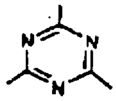 : 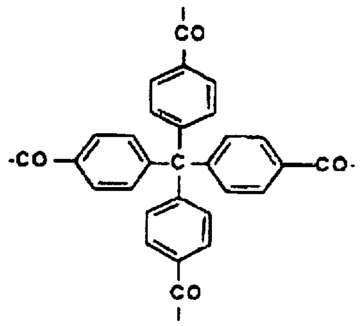 :

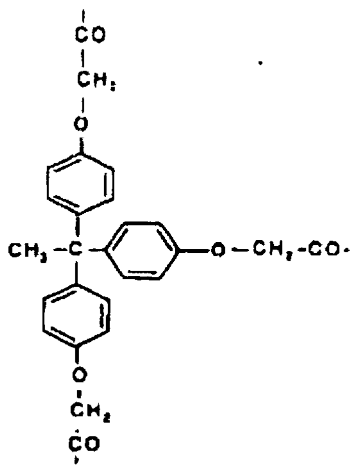 : 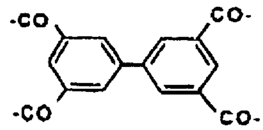 :

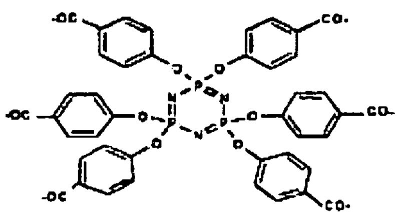 : 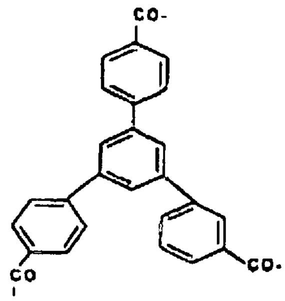

and m is equal to the number of free bonds in W.

7. Compounds according to any of claims 1 to 4 whatsoever in which, respectively, W for compounds of formula (E), W1 for compounds of formula I1, W2 for compounds of formula 12 is:

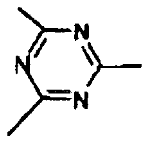

8. Compounds according to any of claims 1 to 4 whatsoever in which, respectively, W for compounds of formula (E), W1 for compounds of formula I1, W2 for compounds of formula 12 is:

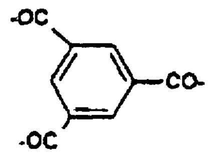

or

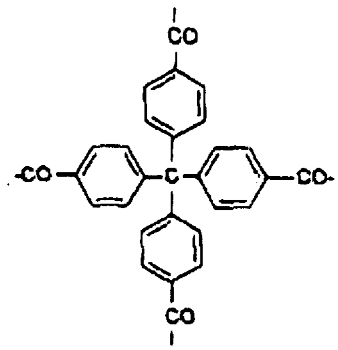

9. Compounds according to any of claims 1 to 4 whatsoever in which, respectively, W for compounds of formula (E), W1 for compounds of formula I1, W2 for compounds of formula 12 is:

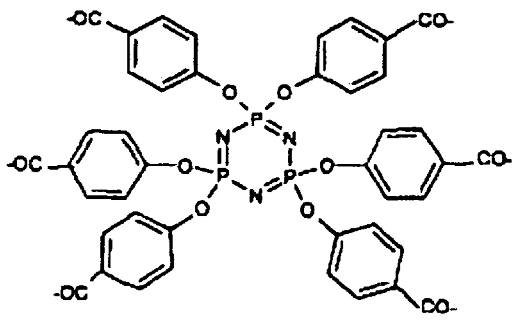

10. Compounds according to any of the preceding claims in which x is 2.

11. Compounds according to any of claims 4, or 6 to 10, with formula I1, in which $-S_1- T - S_2-$ represents:

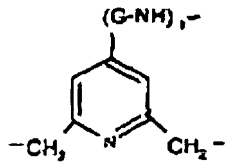

with $S_1 = S_2 = CH_2$.

**12.** Compounds according to claim 11 with formula I1 in which k is 1 and in which G is $-(CH_2)_3-$

**13.** Compounds according to claim 11 with formula I1 in which k is 0 and $B_2$ or $B_3$ represents $-(-CH_2)_3NH-$ or

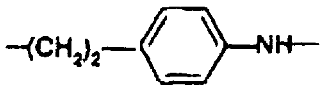

**14.** Compounds according to any of claims 4 to 10, with formula I1 or 12, in which $-S_1-T-S_2-$ represents:

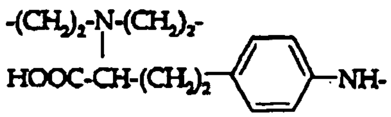

or

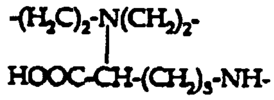

where $S_1 = S_2 = (CH_2)_2$.

**15.** Compounds according to any of the preceding claims for which $B_1$, $B_2$ and $B_3$, when they do not represents -G-NH, represent - $(CH_2)_2CONHR$, with, in R, p = q = 0 and Z being $-CH_2CONH$.

**16.** Compounds according to claim 15, in which R represents:

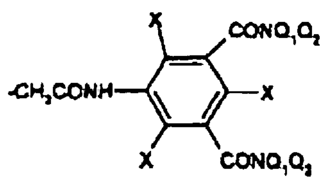

and the Xs are the same and represent Br or I whilst Q1 and $Q_2$, which are the same or different, are mono- or polyhydroxylated $(C_1-C_8)$alkyl groups, so that each $CONQ_1Q_2$ includes from 4 to 10 hydroxyls in total.

**17.** Compounds according to claim 15, in which R represents:

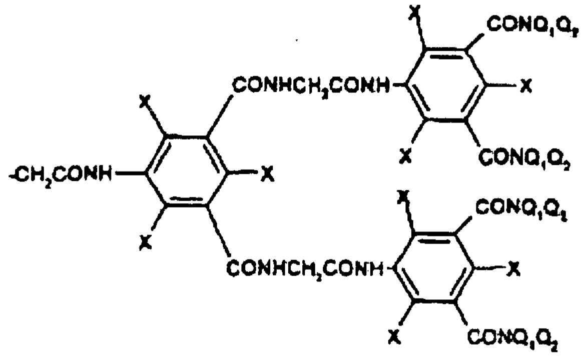

and the identical Xs are Br or I, and $Q_1$ and $Q_2$, which are the same or different, are mono- or poly-hydroxylated $(C_1-C_8)$alkyl groups, so that each $CONQ_1Q_2$ group includes from 4 to 10 hydroxyls in total.

**18.** Compounds according to any of claims 1 to 14 whatsoever in which R represents:

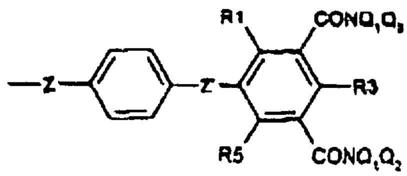

Z is $CH_2$ or $CH_2CONH$, Z' is CONH or $CONHCH_2CONH$, $R_1$, $R_3$, $R_5$, which are the same, are Br or I, with $Q_1$ and $Q_2$, which are the same or different being mono- or poly-hydroxylated $(C_1-C_8)$alkyl groups, so that each $CONQ_1Q_2$ group includes from 4 to 10 hydroxyls in total.

**19.** Compounds according to any of claims 1 to 14 whatsoever in which R represents:

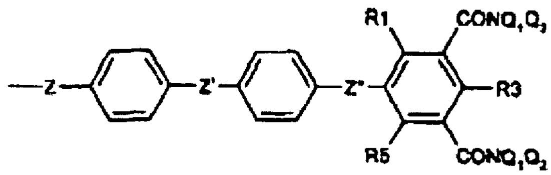

Z is $CH_2CONH$, Z' is CONH, Z" is $CONHCH_2CONH$ and $R_1$, $R_3$, $R_5$, which are the same, are Br or I, and $Q_1$ and $Q_2$, which are the same or different, are mono-hydroxylated or poly-hydroxylated $(C_1-C_8)$alkyl groups, so that each $CONQ_1Q_2$ group includes from 4 to 10 hydroxyls in total.

**20.** Compounds according to any of claims 1 to 14 in which R represents

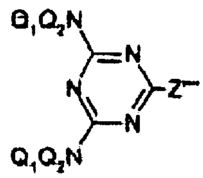

with Z"" being $NQ(CH_2)_j(CH_2OCH_2)_i(CH_2)_jNH_2$, with i = 2 to 6 and j = 1 to 6, preferably

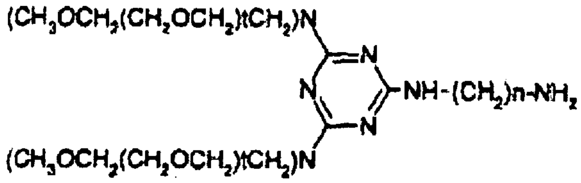

or

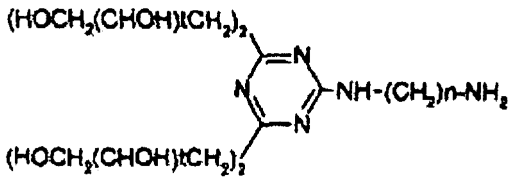

with t =1,2,3 or 4 and n = 2 to 6.

**21.** Compounds according to any of the preceding claims whatsoever, in which $Q_1$ represents $CH_2CHOHCH_2OH$ or $CH_2(CHOH)_4CH_2OH$ and $Q_2$ represents $CH_2(CHOH)_4CH_2OH$.

**22.** Compounds of formula

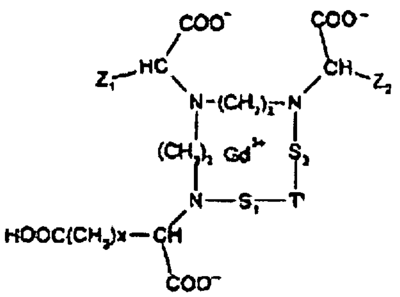

V1

in which x = 1, 2 or 3 and $-S_1-T'-S_2-$ is:

1)

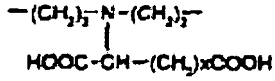

where $S_1 = S_2 = (CH_2)_2$. or

2)

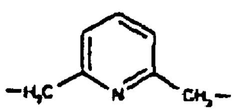

with $S_1 = S_2 = CH_2$
and one of groups $Z_1$ or $Z_2$ is chosen from amongst the groups $-(-CH_2)_3NH_2$ or

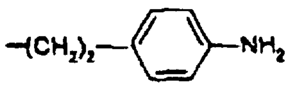

and the other of the $Z_1$ or $Z_2$ is $(CH_2)_xCOOH$;
in the form of salts with an alkaline base.

**23.** Compounds according to claim 22, with the formula VI in which x = 2.

**24.** Compound of formula:

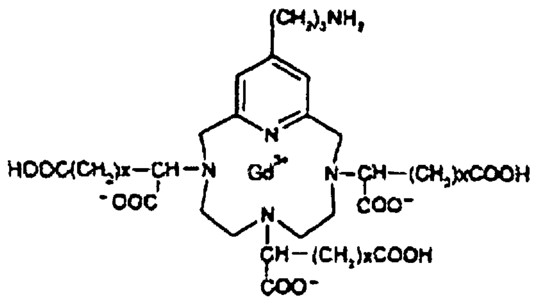

VI 1

in which x = 1, 2 or 3
in the form of salts with an alkaline base.

**25.** Compounds according to claim 24, with the formula VI in which x = 2.

**26.** Compound of formula:

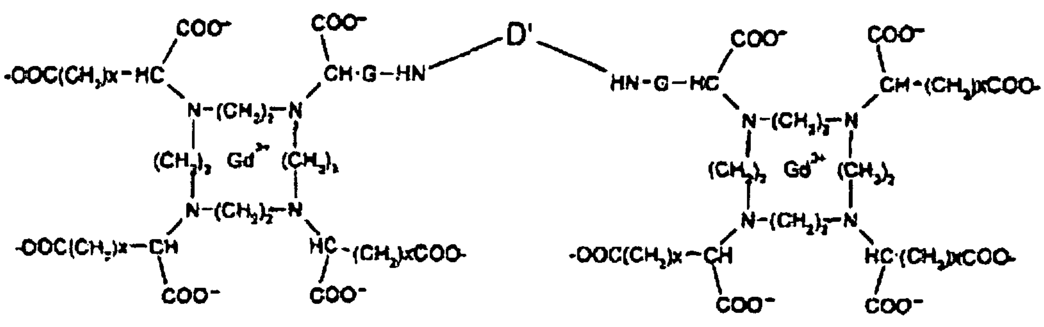

V 2

or

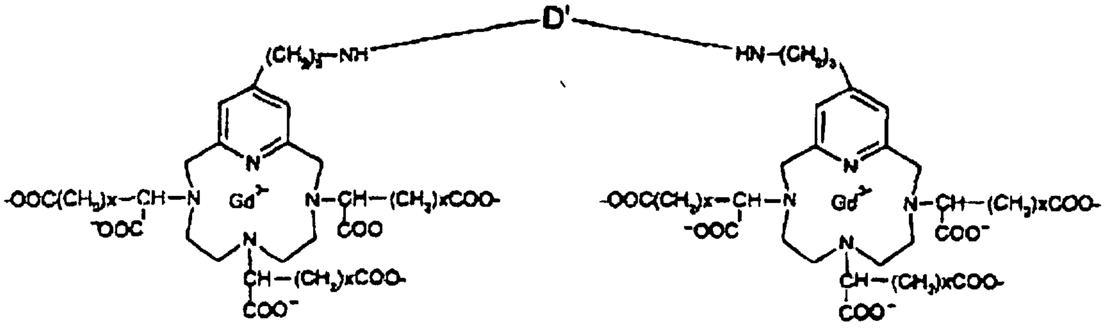

VI 2

in which x = 1, 2 or 3
D'=D-H
D being:

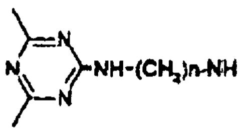

or

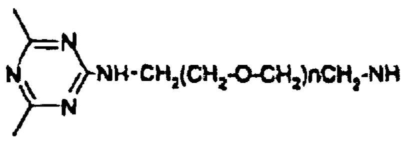

with n = 2 to 6
G-NH being $-(CH_2)_3$-NH- or

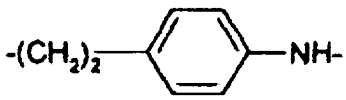

in the form of salts with an alkaline base.

**27.** Contrast product for magnetic resonance imaging, **characterised by** the fact that it includes a compound according to one of the claims 1 to 21, optionally associated with a pharmaceutically acceptable vehicle.

**28.** Contrast product in accordance with claim 27, presented in the form of an injectable sterile solution.

**29.** Contrast product in accordance with claim 27, for use in the diagnosis of cardiovascular, cancerous and inflammatory pathologies.

**30.** The use of a contrast product according to claim 27, for the preparation of a composition intended for the diagnosis of cardiovascular, cancerous or inflammatory pathologies.

**31.** Screening procedure involving selecting compounds of formula (E) according to claim 1, which are effective in diagnostic terms, with the said procedure involving:

- the preparation of a poly-metallic candidate compound of formula (E)
- the use of the candidate compound in an appropriate test for a diagnostic indication
- the selection of candidates which offer a mass efficiency which is at least 30%, preferably at least 50%, greater than that of the corresponding non-polymetal chelate.

## Patentansprüche

**1.** Verbindungen der folgenden allgemeinen Formel:

$$W\text{-}(A)_m \qquad (E)$$

worin:

- W ein zentraler KERN ist,
- A $[(D)_q\text{-}(I_{a,b,c,d,e,f})_r]$ darstellt

und

- q = 0 oder q = 1,
- r = 1, wenn q = 0 oder r 2 oder 3 ist, wenn q = 1,
- m zwischen 3 und 6 ist, wenn q = 0 und m zwischen 2 und 4 ist, wenn q = 1,
- D ein polyfunktionelles Molekül ist, das über eine Verbindungsgruppe 2 an den zentralen KERN W und über Verbindungsgruppen 1 an wenigstens zwei Metallchelate binden kann,
- $I_{a,b,c,d,e,f}$ $I_a$ oder $I_b$ oder $I_c$ oder $I_d$ oder $I_e$ oder $I_f$ bedeutet und Derivate mit eingeschränkter Rotation darstellt,

wobei $I_a$, $I_b$, $I_c$ die Bedeutungen:

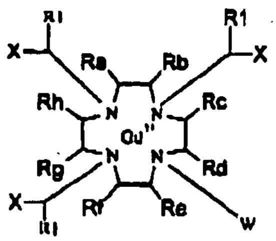

Ia

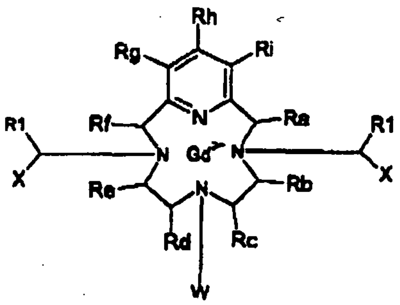

Ib

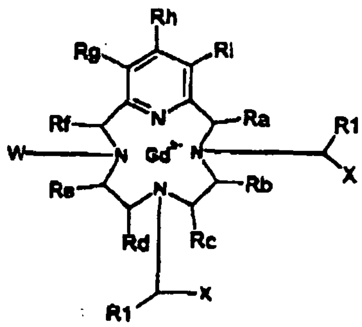

Ic

aufweisen, worin:

- X $CO_2R'a$, CONR'bR'c oder $P(R'd)O_2H$ darstellt und
R'a, R'b, R'c H oder gegebenenfalls hydroxyliertes (C1-C8)Alkyl darstellen;
R'd OH, (C1-C8)Alkyl oder (C1-C8)Alkoxy, (C1-C8)Arylalkyl oder (C1-C8)-Alkoxyalkyl darstellt,
- R1 eine hydrophile Gruppe mit einer Molmasse über 300 und unter 3000 darstellt, die mindestens drei Sauerstoffatome umfaßt und aus den folgenden Gruppen ausgewählt ist:
- Polyoxy(C2-C3)alkylen, insbesondere Polyethylenglykol und seine C1- bis C3-Monoether und Monoester
- Polyhydroxyalkyl
- Polyol
- $(R_2g)_e[(R_2g)_iR_3]_h$, worin:
    - h = 1 oder 2, i = 0, 1 oder 2, e = 1 bis 5
    - $R_2$ (wobei $R_2$ gleich oder verschieden ist)

- fehlt oder ein Alkylen, ein Alkoxyalkylen, ein Polyalkoxyalkylen;
- ein Phenylen oder einen gesättigten oder ungesättigten heterocyclischen Rest darstellt, der gegebenenfalls durch OH, Cl, Br, I, (C1-C8)Alkyl, (C1-C8)Alkyloxy, N02, $NR_xR_y$, $NR_xCOR_y$, $CONR_xR_y$ oder $COOR_x$ substituiert ist, wobei $R_x$ und $R_y$ H oder (C1-C8)Alkyl sind und die geraden, verzweigten oder cyclischen C1- bis C14-Alkyl-, Alkylen- und Alkoxygruppen hydroxyliert sein können,

- g (wobei g gleich oder verschieden ist): fehlt oder eine Funktion O, CO, OCO, COO, SO3, OSO2, CONR', NR'CO, NR'COO, OCONR',NR', NR'CS, CSNR', S02NR', NR'SO2, NR'CSO, OCSNR', NR'CSNR', P(O)(OH)NR', NR'P(O)-(OH) darstellt, worin R' H, (C1-C8)Alkyl oder $R_3$ ist,
- $R_3$ Alkyl, Phenyl, Alkyl, das durch eine oder mehrere Phenyl- oder Alkylenoxygruppen substituiert oder unterbrochen ist; Amino oder Amido darstellt, die durch Alkyl, das gegebenenfalls durch eine der vorangehenden Gruppen substituiert oder unterbrochen ist, substituiert oder nicht substituiert ist, wobei die Phenyl-, Phenylen- und heterocyclischen Gruppen durch OH, Cl, Br, I, (C1-C8)Alkyl, (C1-C8)Alkyloxy, NO2, $NR_xR_y$, $NR_xCOR_y$, $CONR_xR_y$ oder $COOR_x$ substituiert sein können und $R_x$ und $R_y$ H oder (C1-C8)Alkyl sind und die geraden, verzweigten oder cyclischen C1- bis C14-Alkyl-, Alkylen- und Alkoxygruppen hydroxyliert sein können,

- $R_a$ bis $R_i$ unabhängig H, Alkyl, Hydroxyalkyl, Alkylphenyl oder Cycloalkyl darstellen,
- U eine Gruppe -CXR4-Verbindungsgruppe 1, CHR4CON-Verbindungsgruppe 1 oder CHR4-CHR5OH-Verbindungsgruppe 1 ist, wobei

  - R4 und R5 unabhängig H, Alkyl oder Hydroxyalkyl darstellen,
  - X die vorstehende Bedeutung aufweist,

- $I_d$, $I_e$ und $I_f$ die Bedeutungen:

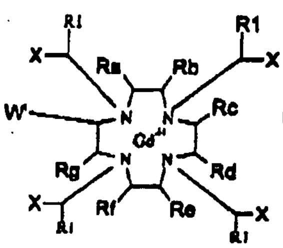

Id

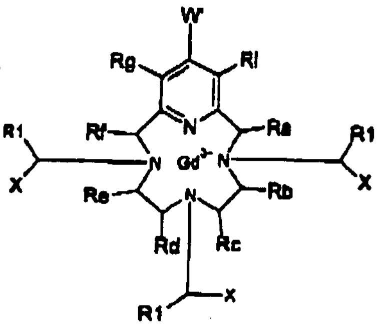

$I_e$

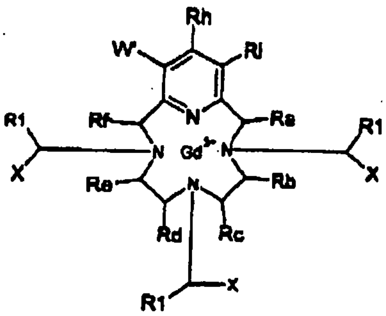

If

aufweisen,
- X, R1 und Ra bis Ri dieselbe Bedeutung wie vorstehend aufweisen und
- U' eine Verbindungsgruppe 1 ist,

sowie die Salze der Verbindungen der Formel (E) mit pharmazeutisch annehmbaren Mineralsäuren oder -basen oder organischen Säuren oder Basen.

**2.** Verbindungen gemäß Anspruch 1, wobei:

- D ein aromatisches Gerüst, vorzugsweise des 1,3,5-Triazintyps der Formel:

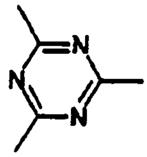

ist, das durch Carboxylat- und/oder Amingruppen polyfunktionalisiert ist,
- die Verbindungsgruppe 1 und die Verbindungsgruppe 2 aus a) und b), bevorzugt a) ausgewählt sind:

a) (CH2)2-φ-NH2, (CH2)3-NH2, NH-(CH2)2-NH, NH-(CH2)3-NH
a) P1-I-P2, wobei P1 und P2 gleich oder verschieden sind und aus OH, SH, NH2, fehlt, CO2H, NCS, NCO und SO3H ausgewählt sind,
und I = Alkylen, Alkoxyalkylen, Polyoxyalkylen, durch Phenylen, Alkyliden oder Acyliden unterbrochenes Alkylen,

- W der Rest eines organischen Moleküls ist, das m Carbonylgruppen trägt, die mit A Carbonsäureamidgruppen bilden oder W eine Gruppe

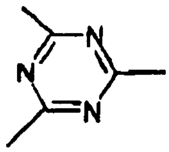

ist.

**3.** Verbindungen gemäß Anspruch 1 oder 2, wobei R1 (CH2)xCONHR mit x = 1, 2 oder 3 darstellt und R eine hydrophile Gruppe mit einem Molekulargewicht über 300 ist, ausgewählt aus:

1) einer Gruppe

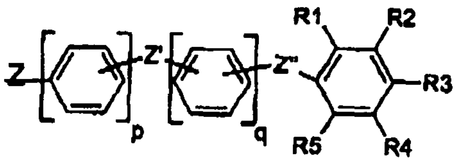

IV1

und Z ist eine Bindung, $CH_2$, $CH_2CONH$ oder $(CH_2)_2NHCO$,
Z' ist eine Bindung, O, S, NQ, $CH_2$, CO, CONQ, NQCO, NQ-CONQ oder $CONQCH_2CONQ$,
Z" ist eine Bindung, CONQ, NQCO oder $CONQCH_2CONQ$,
p und q sind ganze Zahlen, deren Summe 0 bis 3 beträgt,
$R_1$, $R_2$, $R_3$, $R_4$ oder $R_5$ stellen

- entweder unabhängig voneinander H, Br, Cl, I, $CONQ_1Q_2$ oder $NQ_1COQ_2$ dar, wobei $Q_1$ und $Q_2$, die gleich oder verschieden sind, H oder eine mono- oder polyhydroxylierte oder gegebenenfalls durch ein oder mehrere Sauerstoffatome unterbrochene $(C_1\text{-}C_8)$Alkylgruppe sind und mindestens eines und höchstens zwei von $R_1$ bis $R_5$ $CONQ_1Q_2$ oder $NQ_1COQ_2$ sind;
- oder $R_2$ und $R_4$ stellen

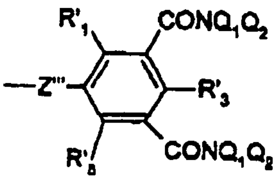

dar und $R_1$, $R'_1$, $R_3$, $R'_3$, $R_5$ und $R'_5$, die gleich oder verschieden sind, stellen H, Br, Cl oder I dar, $Q_1$ und $Q_2$ weisen dieselbe Bedeutung wie vorangehend auf und Z"' ist eine aus CONQ, $CONQCH_2CONQ$, $CONQCH_2$, NQCONQ, $CONQ(CH_2)_2NQCO$ ausgewählte Gruppe und Q ist H oder gegebenenfalls hydroxyliertes $(C_1\text{-}C_4)$Alkyl,

wobei die Alkylgruppen gerade oder verzweigt sein können;
2) einer "Flash" genannten Verzweigung

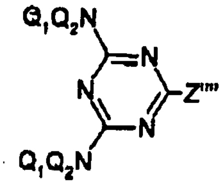

wobei Z"" $NQCH_2(CH_2OCH_2)_i(CH_2)_jNH_2$ mit i = 2 bis 6 und j = 1 bis 6 ist.

**4.** Verbindungen gemäß Anspruch 1 der Formel:

$$W1\text{-}(A1)_{m1} \quad (I1) \; PolyM \; RR$$

worin:

- m1 3, 4, 5 oder 6 ist,
- W1 der Rest eines organischen Moleküls ist, das m1 Carbonylgruppen trägt, die mit A1 Carbonsäureamidgruppen bilden
oder W1 eine Gruppe

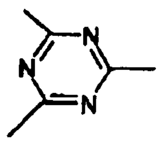

ist,
- A1 die Gruppe

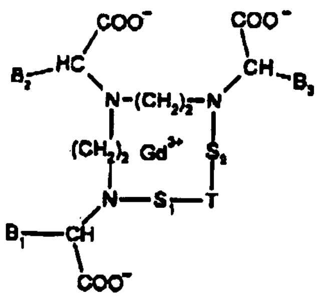

II 1

darstellt, worin
- $S_1$-T-$S_2$ entweder

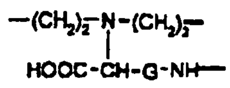

worin $S_1 = S_2 = (CH_2)_2$
und $B_1$, $B_2$ und $B_3$ alle drei $(CH_2)_xCONHR$ mit x = 1, 2 oder 3 darstellen,
oder

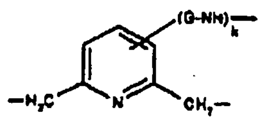

III1

mit k = 0 und $S_1 = S_2 = CH_2$, wobei eines von B1, B2 und B3 G-NH darstellt und die anderen $(CH_2)_xCONHR$ darstellen,
oder

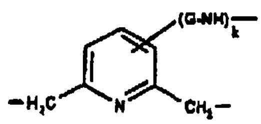

III1

mit k = 1 ist, wobei $B_1$, $B_2$ und $B_3$ alle drei $(CH_2)_xCONHR$ mit x = 1, 2 oder 3 darstellen und GNH aus:
den Gruppen $-(CH_2)_n$-NH- mit n = 1 bis 4
oder

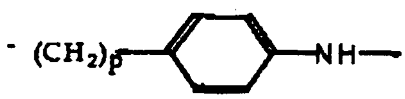

mit p = 0 bis 3 ausgewählt ist,
wobei
- R eine hydrophile Gruppe mit einem Molekulargewicht über 200 darstellt und R bevorzugt eine Gruppe

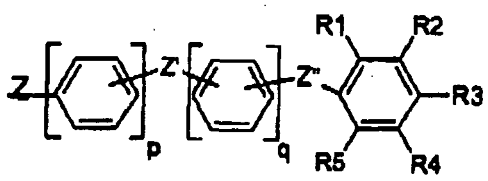

IV1

darstellt und Z eine Bindung, $CH_2$, $CH_2CONH$ oder $(CH_2)_2NHCO$ ist
Z' eine Bindung, O, S, NQ, $CH_2$, CO, CONQ, NQCO, NQ-CONQ oder $CONQCH_2CONQ$ ist,
Z" eine Bindung, CONQ, NQCO oder $CONQCH_2CONQ$ ist,
p und q ganze Zahlen sind, deren Summe 0 bis 3 beträgt,
$R_1$, $R_2$, $R_3$, $R_4$ oder $R_5$
- entweder unabhängig voneinander H, Br, Cl, I, $CONQ_1Q_2$ oder $NQ_1COQ_2$ darstellen und $Q_1$ und $Q_2$, die gleich

oder verschieden sind, H oder eine mono- oder polyhydroxylierte oder gegebenenfalls durch ein oder mehr Sauerstoffatome unterbrochene (C1-C8)Alkylgruppe sind und mindestens eines und höchstens zwei $R^1$ bis $R^5$ $CONQ_1Q_2$ oder $NQ_1COQ_2$ sind,
oder $R_2$ und $R_4$

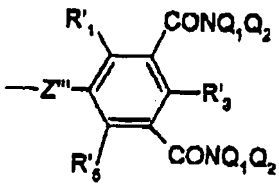

darstellen und $R_1$, $R'_1$, $R_3$, $R'_3$, $R_5$ und $R'_5$, die gleich oder verschieden sind, H, Br, Cl oder I darstellen, $Q_1$ und $Q_2$ dieselbe Bedeutung wie vorangehend aufweisen und Z'" eine aus CONQ, $CONQCH_2CONQ$, $CONQCH_2$, NQCONQ und
$CONQ(CH_2)_2NQCO$ ausgewählte Gruppe ist und Q H oder gegebenenfalls hydroxyliertes $(C_1$-$C_4)$Alkyl ist, wobei die Alkylgruppen gerade oder verzweigt sein können,
oder eine FLASH-Gruppe der Formel

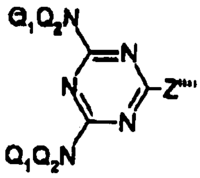

darstellen und Z"" $NQCH_2(CH_2OCH_2)_i(CH_2)_jNH_2$ ist und i = 2 bis 6 und j = 1 bis 6, sowie die Salze der Verbindungen der Formel I1 mit pharmazeutisch annehmbaren Mineralsäuren oder -basen oder organischen Säuren oder Basen.

**5.** Verbindungen gemäß Anspruch 1 der Formel

$W2$-$(A2)_{m2}$ (12) PolyD RR,

worin

- m 2, 3 oder 4 ist,
- W2 der Rest eines organischen Moleküls ist, das m2 Carbonylgruppen trägt, die mit A2 Carbonsäureamidgruppen bilden,
oder W2 eine Gruppe

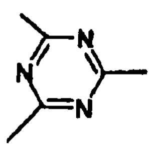

ist,
- A2 1) die Gruppe

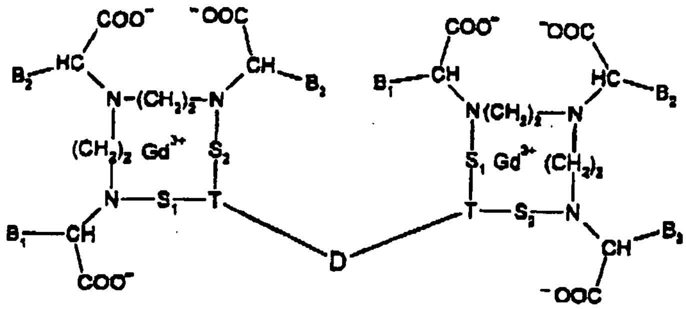

II2

darstellt, worin
- $S_1$-T-$S_2$

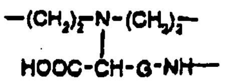

ist, worin $S_1 = S_2 = (CH_2)_2$ ist und
$B_1$, $B_2$ und $B_3$ alle drei $(CH_2)_xCONHR$ mit x = 1, 2 oder 3 darstellen,
- oder A2 2)

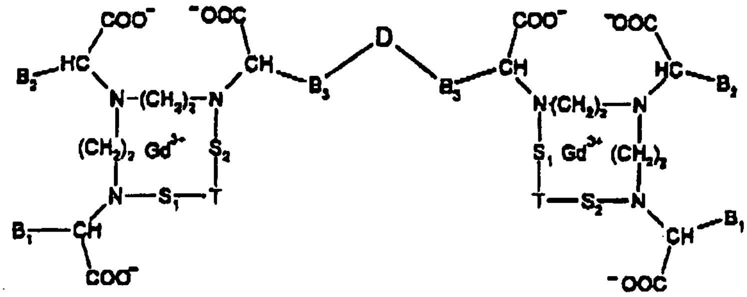

IIa2 (N-funktionalisiertes PCTA genannte Verbindung) oder
IIb2 (N-funktionalisiertes PCTA genannte Verbindung und Stellungsisomer von IIb2) darstellt

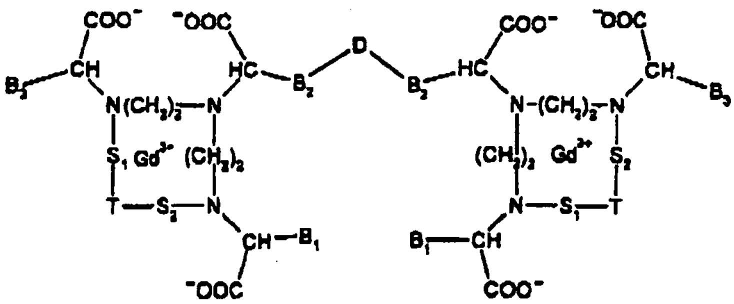

IIb2

worin $S_1$-T-$S_2$-

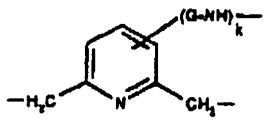

III2

mit k = 0 und $S_1 = S_2 = CH_2$, wobei
bei IIa2 $B_3$ G-NH bedeutet und B1 und B2 $(CH_2)$xCONHR bedeuten,
bei IIb2 $B_2$ G-NH bedeutet und B1 und B3 $(CH_2)$xCONHR bedeuten,

- oder A2 3)

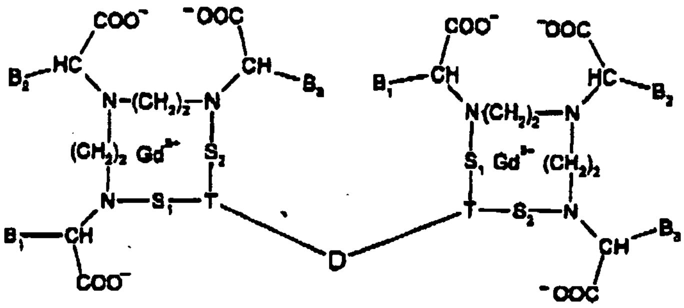

IIc2 (C-funktionalisiertes PCTA genannte Verbindung)
darstellt, wenn $S_1$-T-$S_2$-

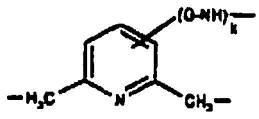

III2

mit k = 1 und $S_1 = S_2 = CH_2$ ist,

wobei IIc2 $B_1$, $B_2$ und $B_3$ alle drei $(CH_2)_xCONHR$ mit x = 1, 2 oder 3 bedeuten,
wobei bei II2, IIa2, IIb2 und IIc2
GNH aus den Gruppen $-(CH_2)_n-NH-$ mit n = 1 bis 4
oder

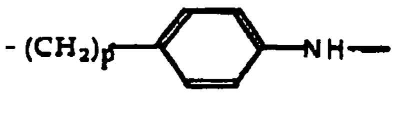

mit p = 0 bis 3 ausgewählt ist,

- und D (Div - Verbindungsgruppe 2) ist und eine Trenngruppe ist, wobei Div bevorzugt vom 1,3,5-Triazintyp ist und D bevorzugt

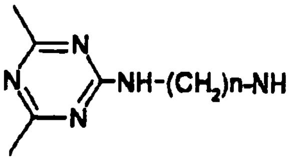

oder

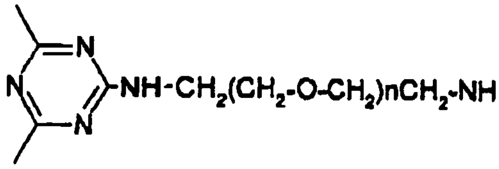

ist,

- R eine hydrophile Gruppe mit einem Molekulargewicht über 200 ist und R bevorzugt eine Gruppe

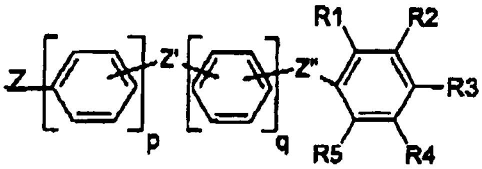

IV2

darstellt und Z eine Bindung, $CH_2$, $CH_2CONH$ oder $(CH_2)_2NHCO$ ist,
Z' eine Bindung, O, S, NQ, $CH_2$, CO, CONQ, NQCO, NQ-CONQ oder $CONQCH_2CONQ$ ist,
Z" eine Bindung, CONQ, NQCO oder $CONQCH_2CONQ$ ist,
p und q ganze Zahlen sind, deren Summe 0 bis 3 beträgt,
$R_1$, $R_2$, $R_3$, $R_4$ oder $R_5$

- entweder unabhängig voneinander H, Br, Cl, I, $CONQ_1Q_2$ oder $NQ_1COQ_2$ darstellen und $Q_1$ und $Q_2$, die gleich oder verschieden sind, H oder eine mono- oder polyhydroxylierte oder gegebenenfalls durch ein oder mehr Sauerstoffatome unterbrochene $(C_1-C_8)$Alkylgruppe sind und mindestens eines und höchstens zwei $R_1$ bis $R_5$ $CONQ_1Q_2$ oder $NQ_1COQ_2$ sind,
- oder $R_2$ und $R_4$

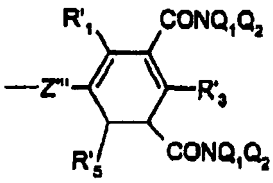

darstellen und $R_1$, $R'_1$, $R_3$, $R'_3$, $R_5$ und $R'_5$, die gleich oder verschieden sind, H, Br, Cl oder I darstellen, $Q_1$ und $Q_2$ dieselbe Bedeutung wie vorangehend aufweisen und Z"' eine aus CONQ, $CONQCH_2CONQ$, $CONQCH_2$, NQCONQ und
$CONQ(CH_2)_2NQCO$ ausgewählte Gruppe ist und Q H oder gegebenenfalls hydroxyliertes $(C_1-C_4)$Alkyl ist, wobei die Alkylgruppen gerade oder verzweigt sein können,
oder eine FLASH-Gruppe der Formel

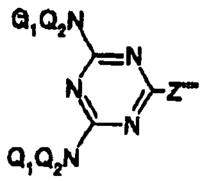

darstellen und Z'''' $NQCH_2(CH_2OCH_2)_i(CH_2)_jNH_2$ mit i = 2 bis 6 und j = 1 bis 6 ist, sowie die Salze der Verbindungen der Formel I1 mit pharmazeutisch annehmbaren Mineralsäuren oder -basen oder organischen Säuren oder Basen.

6. Verbindungen gemäß einem der vorangehenden Ansprüche, bei denen W bei den Verbindungen der Formel (E), W1 bei den Verbindungen der Formel I1 beziehungsweise W2 bei den Verbindungen der Formel 12 aus den Gruppen

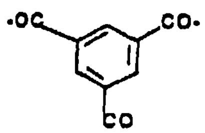 ; 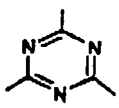 ; 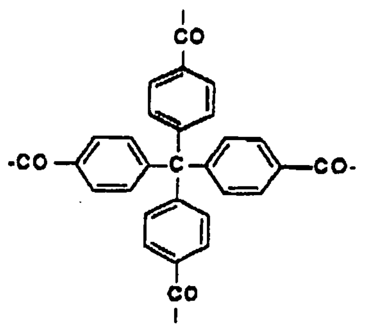 ;

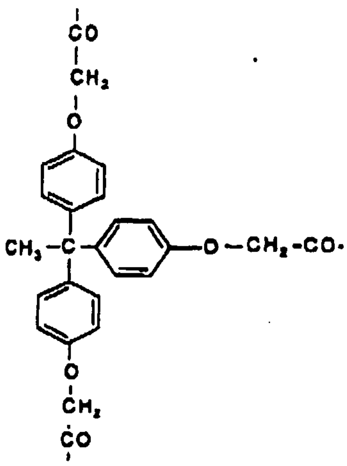 ; 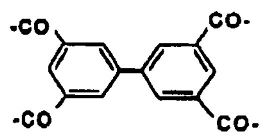 ;

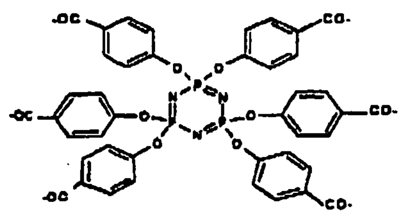 ; 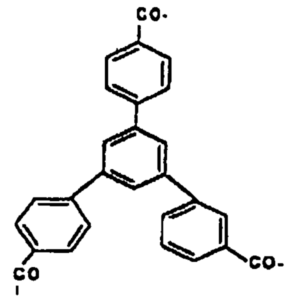

ausgewählt ist und m gleich der Zahl der freien Bindungen von W ist.

7. Verbindungen gemäß einem der Ansprüche 1 bis 4, bei denen W bei den Verbindungen der Formel (E), W1 bei den Verbindungen der Formel I1 beziehungsweise W2 bei den Verbindungen der Formel 12

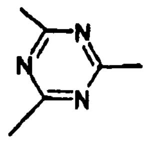

ist.

**8.** Verbindungen gemäß einem der Ansprüche 1 bis 4, bei denen W bei den Verbindungen der Formel (E), W1 bei den Verbindungen der Formel I1 beziehungsweise W2 bei den Verbindungen der Formel 12

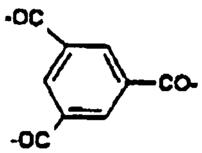

oder

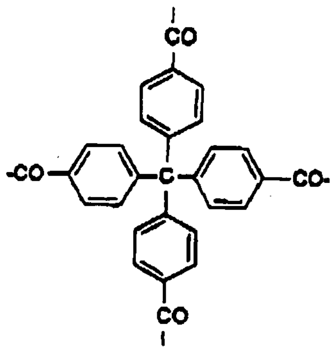

ist.

**9.** Verbindungen gemäß einem der Ansprüche 1 bis 4, bei denen W bei den Verbindungen der Formel (E), W1 bei den Verbindungen der Formel I1 beziehungsweise W2 bei den Verbindungen der Formel 12

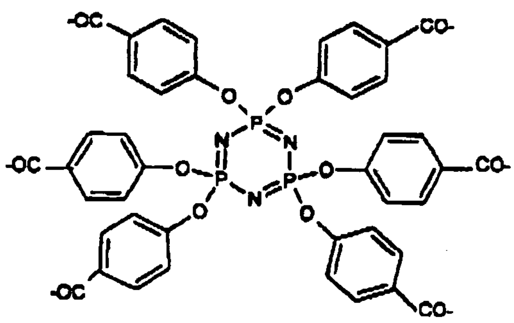

ist.

**10.** Verbindungen gemäß einem der vorangehenden Ansprüche, bei denen x 2 ist.

**11.** Verbindungen gemäß einem der Ansprüche 4 oder 6 bis 10 der Formel I1, worin $-S_1-T-S_2-$

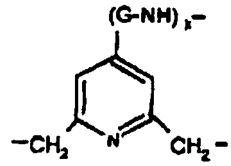

mit $S_1 = S_2 = CH_2$ darstellt.

**12.** Verbindungen gemäß Anspruch 11 der Formel I1, worin k 1 ist und G $-(CH_2)_3-$ ist.

**13.** Verbindungen gemäß Anspruch 11 der Formel I1, worin k 0 ist und $B_2$ oder $B_3$ $-(CH_2)_3NH-$ oder

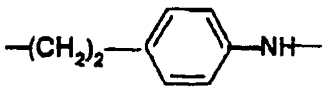

ist.

**14.** Verbindungen gemäß einem der Ansprüche 4 bis 10 der Formel I1 oder 12, worin $-S_1-T-S_2-$

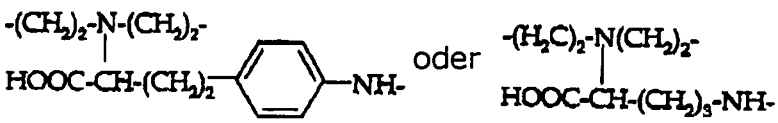

mit $S_1 = S_2 = (CH_2)_2$ darstellt.

**15.** Verbindungen gemäß einem der vorangehenden Ansprüche, bei denen $B_1$, $B_2$ und $B_3$, wenn sie nicht -G-NH darstellen, $-(CH_2)_2CONHR$ darstellen und bei R p = q = 0 und Z -CHCONH ist.

**16.** Verbindungen gemäß Anspruch 15, wobei R

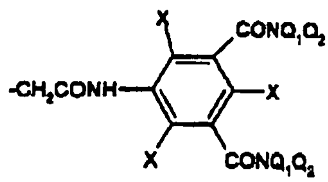

darstellt und X gleich ist und Br oder I darstellt, während $Q_1$ und $Q_2$, die gleich oder verschiedenen sind, mono- oder polyhydroxylierte $(C_1-C_8)$Alkylgruppen der Art sind, daß jede Gruppe $CONQ_1Q_2$ insgesamt 4 bis 10 Hydroxy umfaßt.

**17.** Verbindungen gemäß Anspruch 15, wobei R

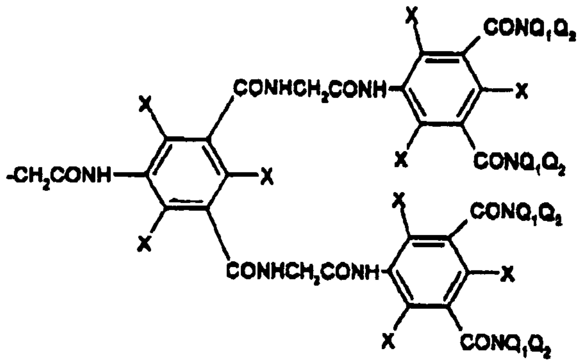

darstellt und X gleich ist und Br oder I sind, während $Q_1$ und $Q_2$, die gleich oder verschiedenen sind, mono- oder polyhydroxylierte ($C_1$-$C_8$)Alkylgruppen der Art sind, daß jede Gruppe $CONQ_1Q_2$ insgesamt 4 bis 10 Hydroxy umfaßt.

**18.** Verbindungen gemäß einem der Ansprüche 1 bis 14, wobei R

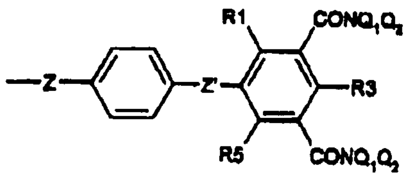

darstellt, Z $CH_2$ oder $CH_2CONH$ ist, Z' CONH oder $CONHCH_2CONH$ ist, $R_1$, $R_3$ und $R_5$ gleich sind und Br oder I sind und $Q_1$ und $Q_2$, die gleich oder verschiedenen sind, mono- oder polyhydroxylierte ($C_1$-$C_8$)Alkylgruppen der Art sind, daß jede Gruppe $CONQ_1Q_2$ insgesamt 4 bis 10 Hydroxy umfaßt.

**19.** Verbindungen gemäß einem der Ansprüche 1 bis 14, wobei R

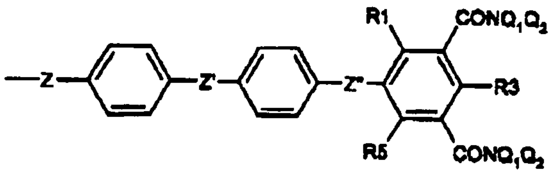

darstellt, Z $CH_2CONH$ ist, Z' CONH, Z" $CONHCH_2CONH$ ist und $R_1$, $R_3$ und $R_5$ gleich sind und Br oder I sind und $Q_1$ und $Q_2$, die gleich oder verschiedenen sind, mono- oder polyhydroxylierte ($C_1$-$C_8$)Alkylgruppen der Art sind, daß jede Gruppe $CONQ_1Q_2$ insgesamt 4 bis 10 Hydroxy umfaßt.

**20.** Verbindungen gemäß einem der Ansprüche 1 bis 14, wobei R

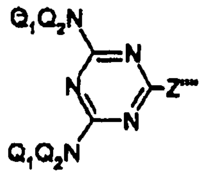

darstellt und Z"" $NQ(CH_2)_j(CH_2OCH_2)_i(CH_2)_jNH_2$ mit i = 2 bis 6 und j = 1 bis 6, bevorzugt

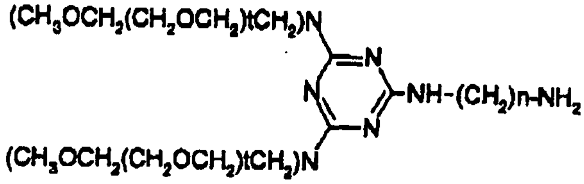

oder

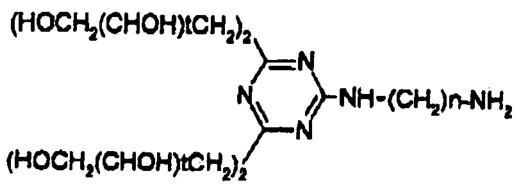

mit t = 1, 2, 3 oder 4 und n = 2 bis 6 ist.

**21.** Verbindungen gemäß einem der vorangehenden Ansprüche, wobei $Q_1$ $CH_2CHOHCH_2OH$ oder $CH_2(CHOH)_4CH_2OH$ darstellt und $Q_2$ $CH_2(CHOH)_4CH_2OH$ darstellt.

**22.** Verbindungen der Formel

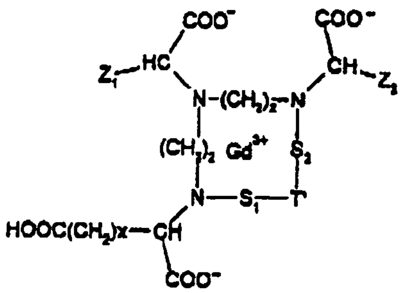

V1

worin x = 1, 2 oder 3 und $-S_1$-T'-$S_2$-

1)

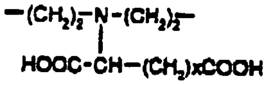

mit $S_1 = S_2 = (CH_2)_2$
oder
2)

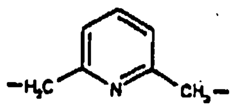

mit $S_1 = S_2 = (CH_2)_2$ ist und eine der Gruppen $Z_1$ oder $Z_2$ aus den Gruppen $-(-CH_2)_3NH_2$ oder

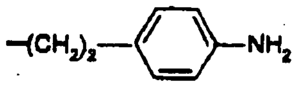

ausgewählt ist und das andere $Z_1$ oder $Z_2$ $(CH_2)_xCOOH$ ist, in Form von Salzen mit einer alkalischen Base.

**23.** Verbindungen gemäß Anspruch 22 der Formel V1, worin x = 2.

**24.** Verbindung der Formel

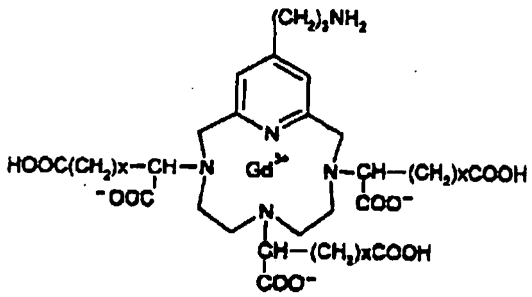

VI 1

worin x = 1, 2 oder 3, in Form von Salzen mit einer alkalischen Base.

**25.** Verbindungen gemäß Anspruch 24 der Formel VI, worin x = 2.

**26.** Verbindung der Formel

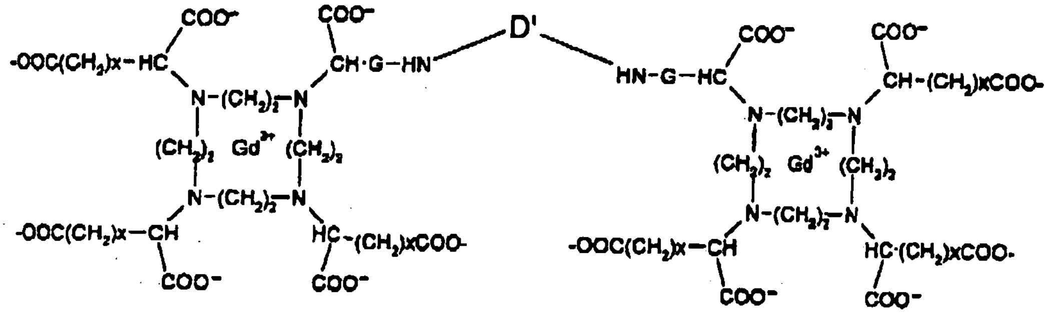

V 2

oder

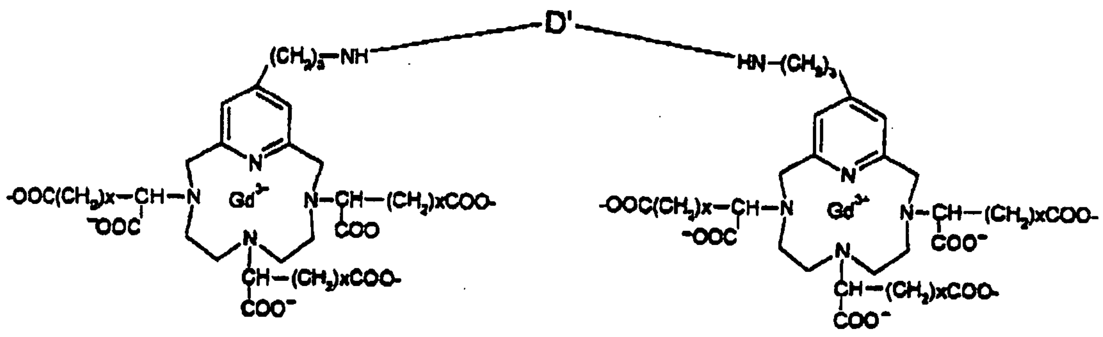

VI 2

worin x = 1, 2 oder 3,
D' = D-H,
wobei D

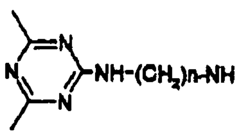

oder

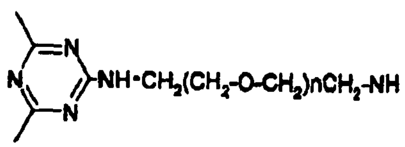

mit n = 2 bis 6 ist,
wobei G-NH $-(CH_2)_3$-NH- oder

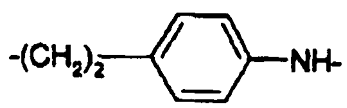

ist, in Form von Salzen mit einer alkalischen Base.

**27.** Kontrastmittel zur Bildgebung durch Magnetresonanz, **dadurch gekennzeichnet, daß** es eine Verbindung gemäß einem der Ansprüche 1 bis 21 gegebenenfalls in Verbindung mit einem pharmazeutisch annehmbaren Träger umfaßt.

**28.** Kontrastmittel gemäß Anspruch 27 in Form einer injizierbaren sterilen Lösung.

**29.** Kontrastmittel gemäß Anspruch 27 zur Verwendung bei der Diagnose einer Herzkreislauf-, Krebs- und Entzündungskrankheit.

**30.** Verwendung eines Kontrastmittels gemäß Anspruch 27 zur Herstellung einer Zusammensetzung, die zur Diagnose einer Herzkreislauf-, Krebs- und Entzündungskrankheit bestimmt ist.

**31.** Durchmusterungsverfahren bestehend aus dem Selektionieren der bei der Diagnose wirkungsvollen Verbindungen

der Formel (E) gemäß Anspruch 1, wobei das Verfahren

- die Herstellung eines polymetallischen Verbindungskandidaten der Formel (E),
- die Verwendung des Verbindungskandidaten bei einem für eine diagnostische Indikation geeigneten Testprotokoll und
- die Selektion von Kandidaten umfaßt, die eine spezifische Wirksamkeit von mindestens 30%, bevorzugt mindestens 50% über der des entsprechenden, nicht polymetallischen Chelats zeigen.